(19) Europäisches Patentamt
European Patent Office
Office européen des brevets

(11) Veröffentlichungsnummer: **0 294 666 B1**

(12) # EUROPÄISCHE PATENTSCHRIFT

(45) Veröffentlichungstag der Patentschrift: **16.02.94**

(51) Int. Cl.5: **C07D 249/12**, C07D 405/12, A01N 47/38

(21) Anmeldenummer: **88108489.1**

(22) Anmeldetag: **27.05.88**

Die Akte enthält technische Angaben, die nach dem Eingang der Anmeldung eingereicht wurden und die nicht in dieser Patentschrift enthalten sind.

(54) **Substituierte Triazolinone.**

(30) Priorität: **12.06.87 DE 3719575**
**05.02.88 DE 3803523**

(43) Veröffentlichungstag der Anmeldung:
**14.12.88 Patentblatt 88/50**

(45) Bekanntmachung des Hinweises auf die Patenterteilung:
**16.02.94 Patentblatt 94/07**

(84) Benannte Vertragsstaaten:
**AT BE CH DE ES FR GB GR IT LI NL SE**

(56) Entgegenhaltungen:
**EP-A- 0 140 194**
**DE-A- 1 620 161**
**DE-A- 3 131 982**

**CHEMICAL ABSTRACTS, vol. 95, no. 23, 07 Dezember 81 Columbus, Ohio, USA W. RUDNICKA et al.: "Some derivatives of 4-amino-1,2,4-triazole-3-thione" & Acta Pol. Pharm. 1981, Bd.38, Nr.2, Seiten 153-162 Kat. D**

(73) Patentinhaber: **BAYER AG**

**D-51368 Leverkusen(DE)**

(72) Erfinder: **Lindig, Markus, Dr.**
**Dahlienweg 16**
**D-4010 Hilden(DE)**
Erfinder: **Findeisen, Kurt, Dr.**
**In der Follmühle 10**
**D-5068 Odenthal 2(DE)**
Erfinder: **Müller, Klaus-Helmut, Dr.**
**Bockhackstrasse 55**
**D-4000 Düsseldorf 13(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Berg.-Gladbach 2(DE)**
Erfinder: **Strang, Harry, Dr.**
**Unterdorfstrasse 6A**
**D-4000 Düsseldorf 31(DE)**
Erfinder: **Feucht, Dieter, Dr.**
**Rüttersweg 108**
**D-5090 Leverkusen 1(DE)**

EP 0 294 666 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, vol. 90, no. 19, 07 Mai 1979 Columbus, Ohio, USA S. IWAI et al.: "Triazoline derivatives" Seite 617; ref. no. 152195P & JP-A-53 135981

PATENT ABSTRACTS OF JAPAN vol. 2, no. 24 (C-77)(4198) 16 Februar 1978, & JP-A-52 125168 (NIPPON SODA K.K.) 20 Oktober 1977,

CHIMICA ACTA TURCICA, vol. 7, no. 3, 1979, Istanbul; Seiten 269 - 290; A. IKIZLER et al.: "Reactions of ester ethoxycarbonylhydrazones with some amine type compounds"

JOURNAL OF HETEROCYCLIC CHEMISTRY, vol. 14, no. 8, 1980, Seiten 1691 - 1696; R. MILCENT et al.: "Recherche en série du triazole-1,2,4. II Réactivité des amino-4 aryl-3 triazol-1,2,4 ones-5" * Seite 1692, Tabelle, Verbindungen 3c, 3d, 3e

ORGANIC MASS SPECTROMETRY, vol. 14, no. 7, 1979, London; Seiten 369 - 378; A. BERNARDINI et al.: "Fragmentation sous l'Impact Electronique d'Amino-4 triazole-1,2,4 ones-5" * Seite 369, rechte Spalte, Tabelle, Verbindung 22

JOURNAL OF HETEROCYCLIC CHEMISTRY, vol. 20, 1983, Seiten 77 - 80; R. MILCENT et al.: "Oxydations d'hydrazones par le bioxyde de plomb: nouvelles synthèses d'oxadiazoles-1,3,4 et de dérivés ..." * Seite 79, Schema 3; Seite 80, linke Spalte, letzter Absatz

## Beschreibung

Die Erfindung betrifft neue substituierte Triazolinone, mehrere Verfahren zu ihrer Herstellung und ihre Verwendung als Herbizide.

Es ist bekannt, daß bestimmte Stickstoffheterocyclen wie beispielsweise das 4-Amino-3-methyl-6-phenyl-1,2,4-triazin-5-on oder das N-isobutyl-2-oximidazolidin-1-carboxamid (vgl.z.B. DE-OS 23 64 474 und R. Wegler "Chemie der Pflanzenschutz- und Schädlingsbekämpfungsmittel Band 5, 219 (1977)) herbizide Eigenschaften besitzen.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Die JP-A-52 125 168 beschreibt 4-Amino-3-alk(en/in)yl-1,2,4-triazolin-5-on-Derivate, deren Synthese und auch deren Verwendung als Herbizide.

Weiterhin sind bestimmte substituierte Triazolinone, wie beispielsweise das 1-(N,N-Dimethylcarbamoyl)-3-phenyl-4-amino-1,2,4-triazolin-5-on bekannt (vergl. J.Heterocycl. Chem. 17, 1691-1696 [1980]; Org. Mass. Spectrom. 14, 369-378 [1979]).
Über eine Wirksamkeit dieser vorbekannten Triazolinone als Herbizide ist bisher nichts bekannt.

Es wurden neue substituierte Triazolinone der allgemeinen Formel (I),

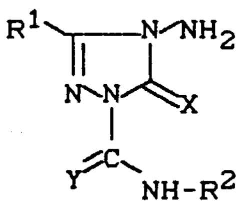

(I)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit 1 bis 6 Kohlenstoffatomen in den einzelnen Alkylteilen, für Cycloalkyl oder Cycloalkylalkyl mit jeweils 3 bis 7 Kohlenstoffatomen im Cycloalkylteil und gegebenenfalls 1 bis 6 Kohlenstoffatomen im geradkettigen oder verzweigten Alkylteil, für einfach oder mehrfach, gleich oder verschieden substituiertes Aryl mit jeweils 6 bis 10 Kohlenstoffatomen im Arylteil steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für Wasserstoff, für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für Cycloalkyl mit 11 Kohlenstoffatomen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenene Halogenatomen oder geradkettiges oder verzweigtes Halogenalkenyl mit bis zu 4 Kohlenstoffatomen und 1 bis 5 gleichen oder verschiedenen Halogenatomen oder jeweils zweifach verknüpftes Alkandiyl, bzw. Alkendiyl mit jeweils bis zu 4 Kohlenstoffatomen; $R^2$ außerdem für im Heterocyclylteil gegebenenfalls einfach oder mehrfach, gleich oder verchieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; $R^2$ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aroyl, Aryl, Aralkyloxy oder Aryloxy, mit jeweils 6 bis 10 Kohlenstoffatomen im Arylteil und gegebenenfalls 1 bis 8 Kohlenstoffatomen im geradkettigen oder verzweigten Alkylteil steht, wobei als Alkylsubstituenten gegebenenfalls Halogen und Cyano infrage kommen und wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy; oder $R^2$ für Benzyl mit im Phenylteil ankondensierter $-O-CH_2-O-$ Gruppe steht,

X für Sauerstoff oder Schwefel steht und

Y für Sauerstoff oder Schwefel steht

gefunden.

Die Verbindungen der Formel (I) können gegebenenfalls in Abhängigkeit von der Art der Substituenten $R^1$ und $R^2$ als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Triazolinone der allgemeinen Formel (I),

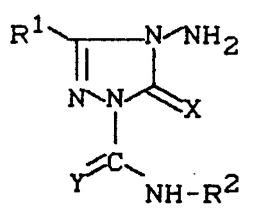

(I)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit 1 bis 6 Kohlenstoffatomen in den einzelnen Alkylteilen, für Cycloalkyl oder Cycloalkylalkyl mit jeweils 3 bis 7 Kohlenstoffatomen im Cycloalkylteil und gegebenenfalls 1 bis 6 Kohlenstoffatomen im geradkettigen oder verzweigten Alkylteil, für einfach oder mehrfach, gleich oder verschieden substituiertes Aryl mit jeweils 6 bis 10 Kohlenstoffatomen im Arylteil steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für Wasserstoff, für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8

Kohlenstoffatomen und 1 bis 6 Hydroxygruppen, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für Cycloalkyl mit 11 Kohlenstoffatomen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenene Halogenatomen oder geradkettiges oder verzweigtes Halogenalkenyl mit bis zu 4 Kohlenstoffatomen und 1 bis 5 gleichen oder verschiedenen Halogenatomen oder jeweils zweifach verknüpftes Alkandiyl, bzw. Alkendiyl mit jeweils bis zu 4 Kohlenstoffatomen; $R^2$ außerdem für im Heterocyclylteil gegebenenfalls einfach oder mehrfach, gleich oder verchieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; $R^2$ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aroyl, Aryl, Aralkyloxy oder Aryloxy, mit jeweils 6 bis 10 Kohlenstoffatomen im Arylteil und gegebenenfalls 1 bis 8 Kohlenstoffatomen im geradkettigen oder verzweigten Alkylteil steht, wobei als Alkylsubstituenten gegebenenfalls Halogen und Cyano infrage kommen und wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy; oder $R^2$ für Benzyl mit im Phenylteil ankondensierter $-O-CH_2-O-$ Gruppe steht,

X für Sauerstoff oder Schwefel steht und

Y für Sauerstoff oder Schwefel steht,

erhält, wenn man

(a) Hydrazone der Formel (II),

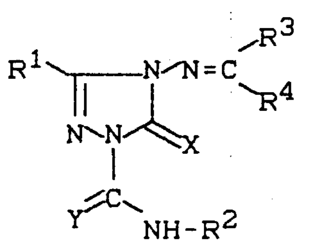

(II)

in welcher

$R^1$, $R^2$, X und Y die oben angegebene Bedeutung haben und

$R^3$ und $R^4$ unabhängig voneinander jeweils für Wasserstoff, für geradkettiges oder verzweigtes Alkyl mit 1 bis 4 Kohlenstoffatomen oder für Phenyl oder Benzyl stehen

mit einer Säure gegebenenfalls in Gegenwart eines Verdünnungsmittels umsetzt,

oder wenn man

(b) 1H-Triazolinone der Formel (III),

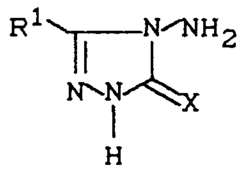

(III)

in welcher

$R^1$ und X die oben angegebene Bedeutung haben,

mit Iso(thio)cyanaten der Formel (IV),

$R^2$-N = C = Y (IV)

in welcher

$R^2$ und Y die oben angegebene Bedeutung haben,

gegebenenfalls in Gegenwart eines Verdünnungsmittels und gegebenenfalls in Gegenwart eines Reaktionshilfsmittels umsetzt,

oder wenn man

(c) Triazolinone der Formel (V),

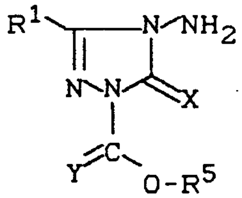

(V)

in welcher

$R^1$, X und Y die oben angegebene Bedeutung haben,

und

$R^5$ steht vorzugsweise für geradkettiges oder verzweigtes Alkyl mit 1 bis 4 Kohlenstoffatomen oder für gegebenenfalls ein- bis dreifach, gleich oder verschieden substituiertes Phenyl oder Benzyl, wobei als Substituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

mit Aminen der Formel (VI),

$R^2$-$NH_2$ (VI)

in welcher

$R^2$ die oben angegebene Bedeutung hat,

gegebenenfalls in Gegenwart eines Verdünnungsmittels und gegebenenfalls in Gegenwart eines Reaktionshilfsmittels umsetzt.

Schließlich wurde gefunden, daß die neuen substituierten Triazolinone der allgemeinen Formel (I) herbizide Eigenschaften besitzen.

Überraschenderweise zeigen die erfindungsgemäßen substituierten Triazolinone der allgemeinen Formel (I) eine erheblich höhere herbizide Potenz gegenüber Problemunkräutern als die aus dem Stand der Technik bekannten Stickstoffheterocyclen, wie beispielsweise das 4-Amino-3-methyl-6-phenyl-1,2,4-triazin-5-on, welche chemisch und wirkungsmäßig naheliegende Verbindungen sind.

Die erfindungsgemäßen substituierten Triazolinone sind durch die Formel (I) allgemein definiert.

Bevorzugt sind Verbindungen der Formel (I) bei welchen

$R^1$ für Wasserstoff, Methyl, Ethyl, n- oder i-Propyl, n-, i-, s- oder t-Butyl, n- oder i-Pentyl, n- oder i-Hexyl, für Allyl, Propargyl, geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Methoxymethyl, Ethoxymethyl, Propoxymethyl, Cyclopentyl, Cyclohexyl, Cyclopropyl, Cyclopropylmethyl, Cyclohexylmethyl, Cyclohexylethyl, für für gegebenenfalls ein- bis dreifach gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^2$ Wasserstoff, Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl, Dodecyl, für Allyl, jeweils geradkettiges oder verzweigtes Butenyl, Pentenyl oder Hexenyl, Propargyl, jeweils geradkettiges oder verzweigtes Butinyl, Pentinyl oder Hexinyl, für geradkettiges oder verzweigtes Halogenalkyl mit 1 bis 8 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 8 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl steht, wobei als Substituenten jeweils infrage kommen: Fluor, Chlor, Brom, Methyl, Ethyl, n- oder i-Propyl, n-, i-, s- oder t-Butyl, Cyano, Methandiyl, Ethandiyl, Butandiyl oder Butadiendiyl oder Dichlorallyl;

$R^2$ weiterhin für im Heterocyclylteil gegebenenfalls ein- bis dreifach, gleich oder verschieden substituiertes Heterocyclylmethyl, Heterocyclylpropyl oder Heterocyclylethyl steht, wobei als Heterocyclen jeweils infrage kommen:

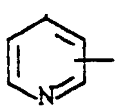 ; 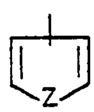 ; 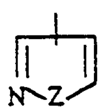 ; 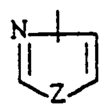 ;

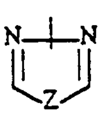 ; 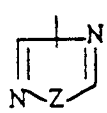 ; 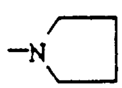 ; 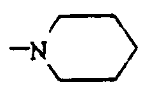 ;

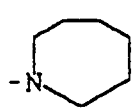 ; 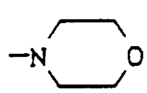 oder 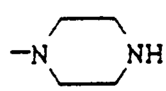

wobei Z jeweils für Sauerstoff oder Schwefel steht und wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;

$R^2$ außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl steht, wobei als Phenylsubstituenten

jeweils infrage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy,

X für Sauerstoff oder Schwefel steht und

Y für Sauerstoff oder Schwefel steht.

Besonders bevorzugt sind Verbindungen der Formel (I), bei welchen

$R^1$ für Wasserstoff, Methyl, Ethyl, n- oder i-Propyl, Methoxymethyl, Ethoxymethyl oder Propoxymethyl steht,

$R^2$ für Wasserstoff, für jeweils gegebenenfalls ein-bis dreifach durch Fluor und/oder Chlor substituiertes Methyl, Ethyl, n- oder i-Propyl, n-, i-, s- oder t-Butyl, Allyl, Propargyl, jeweils geradkettiges oder verzweigtes Pentyl, Hexyl, Heptyl, Octyl, Butenyl, Pentenyl, Hexenyl, Butinyl, Pentinyl oder Hexinyl steht; außerdem für jeweils gegebenenfalls ein- bis dreifach gleich oder verschieden durch Fluor, Chlor, Methyl, Ethyl und/oder Cyano substituiertes Cyclopropyl, Cyclopentyl, Cyclohexyl, Cyclohexenyl, Cyclopropylmethyl, Cyclopropylethyl, Cyclohexylmethyl, Cyclohexylethyl oder Cycloheptyl steht und schließlich für Benzyl, Phenylethyl oder Phenyl steht,

X für Sauerstoff oder Schwefel steht und

Y für Sauerstoff oder Schwefel steht.

Im einzelnen seien außer den bei den Herstellungsbeispielen genannten Verbindungen die folgenden substituierten Triazolinone der allgemeinen Formel (I) genannt:

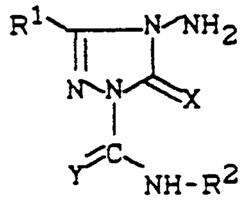

(I)

| $R^1$ | $R^2$ | X | Y |
|---|---|---|---|
| $CH_3$ | 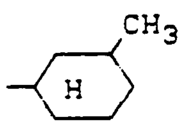 | O | O |
| $CH_3$ | 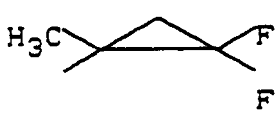 | O | O |
| $CH_3$ | 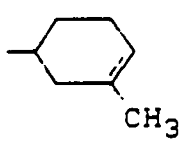 | O | O |
| $CH_3$ | $-C(CH_3)_3$ | O | S |

| $R^1$ | $R^2$ | X | Y |
|---|---|---|---|
| $CH_3$ | $-C(CH_3)_3$ | S | S |
| $CH_3$ | 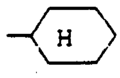 | S | S |
| $CH_3$ | 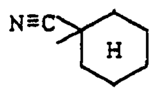 | O | O |
| $C_2H_5$ | $-C(CH_3)_3$ | O | O |
| $C_2H_5$ | $-C(CH_3)_3$ | O | S |
| $C_2H_5$ | $-C(CH_3)_3$ | S | O |
| $C_2H_5$ | 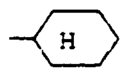 | O | S |
| $C_2H_5$ | 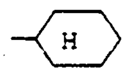 | S | O |
| $C_2H_5$ | 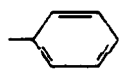 | O | O |
| $C_2H_5$ | 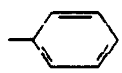 | O | S |
| $C_2H_5$ | 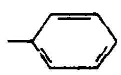 | S | O |
| $C_2H_5$ | 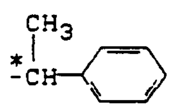 S-Konfiguration | O | O |
| H | $-C(CH_3)_3$ | O | O |
| H | 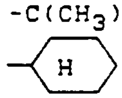 | O | O |

| $R^1$ | $R^2$ | X | Y |
|---|---|---|---|
| H | 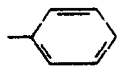 | O | O |
| H | $-C(CH_3)_3$ | O | S |
| H | $-C(CH_3)_3$ | S | O |
| H | 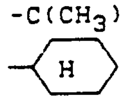 | O | S |
| H | 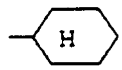 | S | O |
| $CH_3$ | 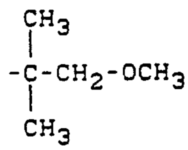 | O | O |
| $CH_3$ | 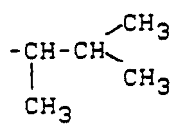 | O | O |
| $CH_3$ | 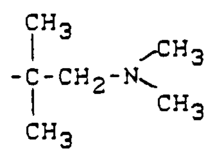 | O | O |
| $CH_3$ | 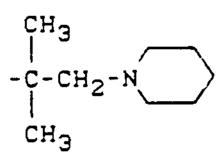 | O | O |
| $CH_3$ | 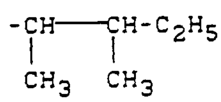 | O | O |

| $R^1$ | $R^2$ | X | Y |
|---|---|---|---|
| $CH_3$ | 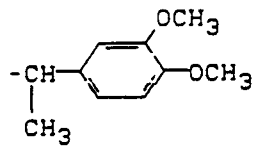 | O | O |
| $CH_3$ | 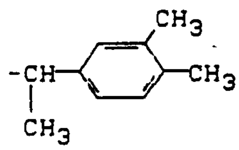 | O | O |
| $CH_3$ | 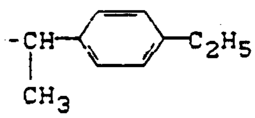 | O | O |
| $CH_3$ | 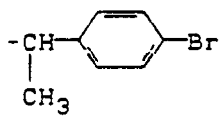 | O | O |
| $CH_3$ | 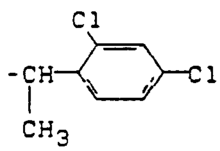 | O | O |
| $CH_3$ | 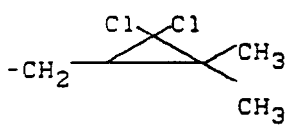 | O | O |
| $CH_3$ | 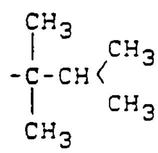 | O | O |
| $CH_3$ | 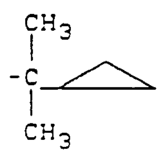 | O | O |

| $R^1$ | $R^2$ | X | Y |
|---|---|---|---|
| $CH_3$ | 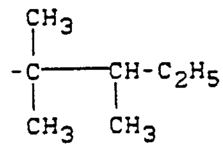 | O | O |
| $CH_3$ | 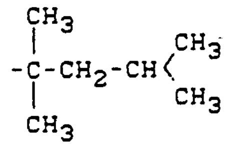 | O | O |
| $CH_3$ | 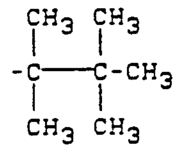 | O | O |
| $CH_3$ | 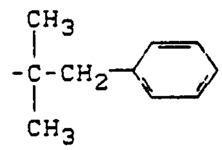 | O | O |
| $CH_3$ | 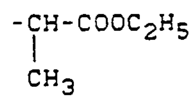 | O | O |
| $CH_3$ | 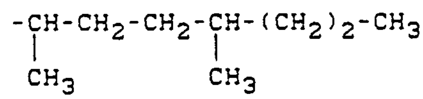 | O | O |
| $CH_3$ | 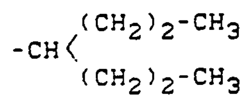 | O | O |
| $CH_3$ | 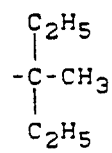 | O | O |

| $R^1$ | $R^2$ | X | Y |
|---|---|---|---|
| $CH_3$ | 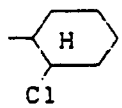 | O | O |
| $CH_3$ | 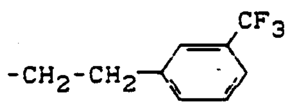 | O | O |
| $CH_3$ | 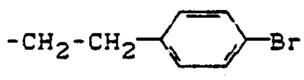 | O | O |
| $CH_3$ | 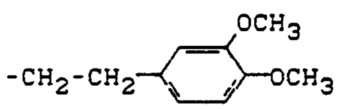 | O | O |
| $CH_3$ | 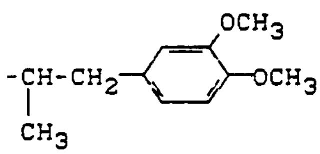 | O | O |
| $CH_3$ | 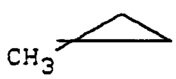 | O | O |
| $CH_3$ | 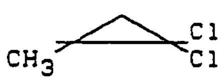 | O | O |
| $CH_3$ | 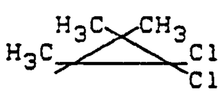 | O | O |
| $CH_3$ | 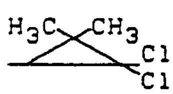 | O | O |

| $R^1$ | $R^2$ | X | Y |
|---|---|---|---|
| $CH_3$ | 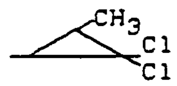 | O | O |
| $CH_3$ | 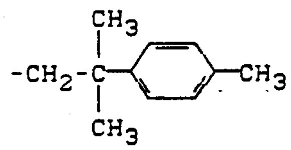 | O | O |
| $CH_3$ | 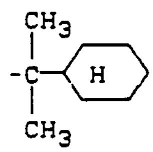 | O | O |
| $CH_3$ | 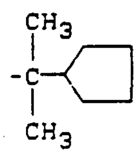 | O | O |
| $CH_3$ | 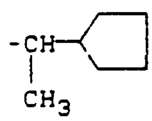 | O | O |
| $CH_3$ | 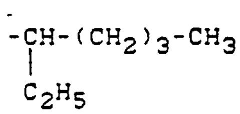 | O | O |
| $CH_3$ | 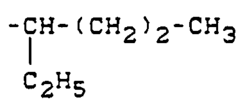 | O | O |
| $CH_3$ | 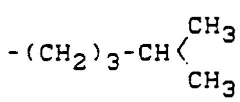 | O | O |

| $R^1$ | $R^2$ | X | Y |
|---|---|---|---|
| $CH_3$ | 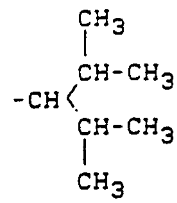 | O | O |
| $CH_3$ | 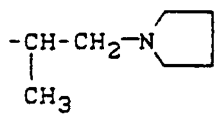 | O | O |
| $CH_3$ | 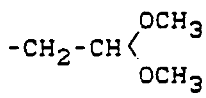 | O | O |
| $CH_3$ | 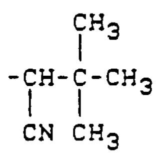 | O | O |
| $CH_3$ | 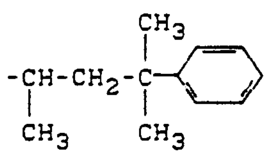 | O | O |
| $CH_3$ | 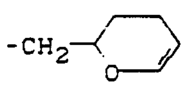 | O | O |
| $CH_3$ | 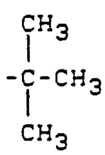 | O | S |
| $CH_3$ | 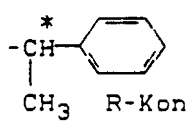 R-Konfiguration | O | S |

| $R^1$ | $R^2$ | X | Y |
|---|---|---|---|
| $CH_3$ | 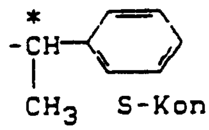 S-Konfiguration | O | S |
| $CH_3$ | 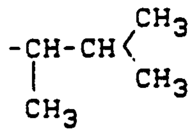 | O | S |
| $CH_3$ | 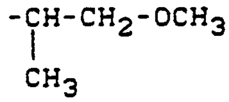 | O | S |
| $CH_3$ | $-(CH_2)_2-CH_3$ | O | S |
| $CH_3$ | 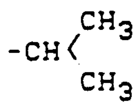 | O | S |
| $CH_3$ | 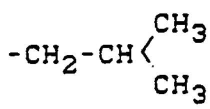 | O | S |
| $CH_3$ | 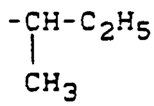 | O | S |
| $CH_3$ | 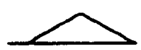 | O | S |
| $CH_3$ | 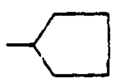 | O | S |
| $CH_3$ | $-(CH_2)_2-CH_3$ | S | O |
| $CH_3$ | $-(CH_2)_3-CH_3$ | S | O |
| $CH_3$ | 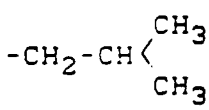 | S | O |
| $CH_3$ | 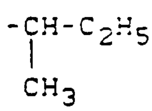 | S | O |

Verwendet man beispielsweise 1-(N-Isobutylcarbamoyl)-4-isopropylidenimino-3-methyl-1,2,4-triazolin-5-on als Ausgangsverbindung, so läßt sich der Reaktionsablauf des erfindungsgemäßen Verfahrens (a) durch das folgende Formelschema darstellen:

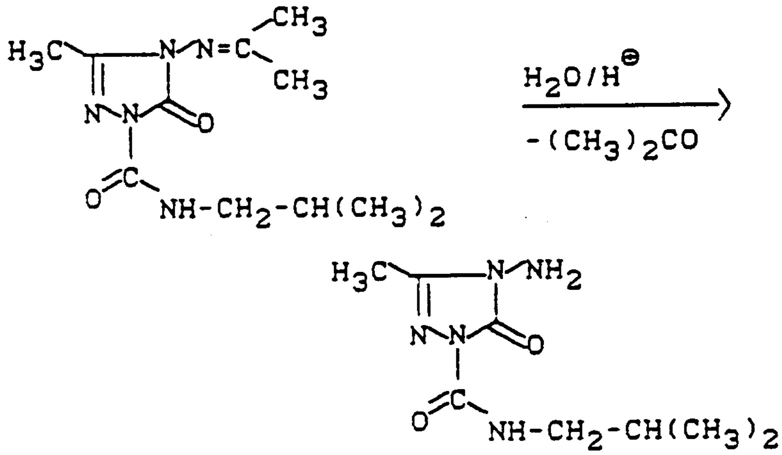


Verwendet man beispielsweise 4-Amino-3-methyl-1,2,4-(1H)-triazolin-5-on und t-Butylisocyanat als Ausgangsstoffe, so läßt sich der Reationsablauf des erfindungsgemäßen Verfahrens (b) durch das folgende Formelschema darstellen:

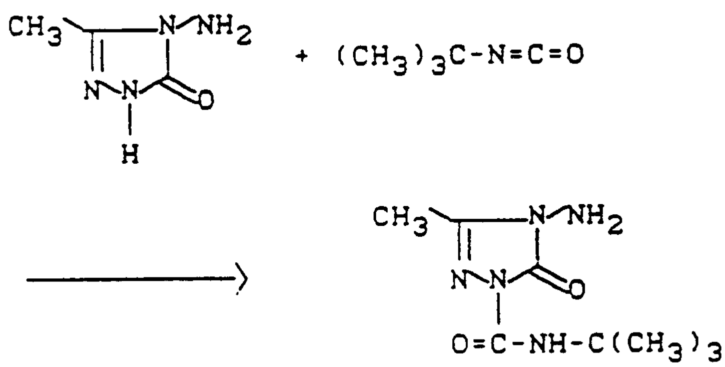

Verwendet man beispielsweise 1-Ethoxycarbonyl-4-amino-3-methyl-1,2,4-triazolin-5-on und N,N-Diethylpropan-1,3-diamin als Ausgangsstoffe, so läßt sich der Reaktionsablauf des erfindungsgemäßen Verfahrens (c) durch das folgende Formelschema darstellen:

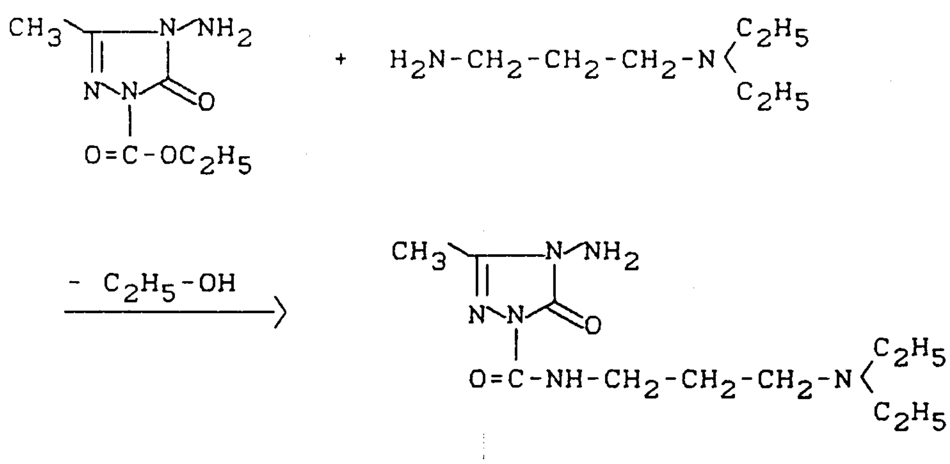


Die zur Durchführung des erfindungsgemäßen Verfahrens (a) als Ausgangsstoffe benötigten Hydrazone sind durch die Formel (II) allgemein definiert. In dieser Formel (II) stehen $R^1$, $R^2$, X und Y vorzugsweise für

diejenigen Reste, die bereits im Zusammenhang mit der Beschreibung der erfindungsgemäßen Stoffe der Formel (I) als bevorzugt für diese Substituenten genannt wurden.

Die Hydrazone der Formel (II) sind noch nicht bekannt, ausgenommen die Verbindung 1-(N-4-Bromphenylthiocarbamoyl)-4-phenylidenamino-1,2,4-triazolin-5-thion.

Sie sind ebenfalls Gegenstand der vorliegenden Erfindung. Man erhält sie jedoch in Analogie zu bekannten Verfahren (vergl. z.B. Acta Pol. Pharm. 38, 153-162 [1981] bzw. C.A. 95: 203841j), beispielsweise wenn man 1-unsubstituierte 4-Amino-triazolinone der Formel (III),

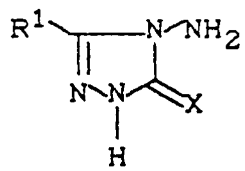

(III)

in welcher

$R^1$ und X die oben angegebene Bedeutung haben,

mit Aldehyden oder Ketonen der Formel (VII),

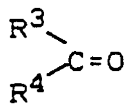

(VII)

in welcher

$R^3$ und $R^4$ die oben angegebene Bedeutung haben,

gegebenenfalls in Gegenwart eines Verdünnungsmittels wie beispielsweise Dichlormethan oder Toluol und gegebenenfalls in Gegenwart eines Katalysators wie beispielsweise p-Toluolsulfonsäure bei Temperaturen zwischen 40 °C und 120 °C umsetzt und die so erhältlichen 1-unsubstituierten Triazolinon-Hydrazone der Formel (VIII),

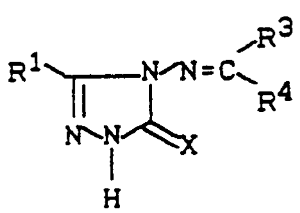

(VIII)

in welcher

$R^1$, $R^3$, $R^4$ und X die oben angegebene Bedeutung haben,

entweder in einer anschließenden 2.Stufe mit Iso(thio)cyanaten der Formel (IV),

$R^2$-N = C = Y (IV)

in welcher

$R^2$ und Y die oben angegebene Bedeutung haben,

gegebenenfalls in Gegenwart eines Verdünnungsmittels wie beispielsweise Dichlormethan oder Dioxan und gegebenenfalls in Gegenwart eines Reaktionshilfsmittels wie beispielsweise Triethylamin bei Temperaturen zwischen 50 °C und 150 °C umsetzt;

oder alternativ in einer anschließenden 2. Stufe mit (Thio)Chlorameisensäureestern der Formel (IX),

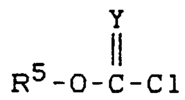 (IX)

in welcher

$R^5$ und Y die oben angegebenen Bedeutungen haben,

gegebenenfalls in Gegenwart eines Verdünnungsmittels wie beispielsweise Tetrahydrofuran und gegebenenfalls in Gegenwart eines Reaktionshilfsmittels wie beispielsweise Natriumhydrid oder Kalium-t-butylat bei Temperaturen zwischen - 20 °C und + 40 °C umsetzt und die so erhältlichen Triazolinone der Formel (X),

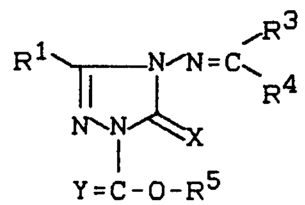 (X)

in welcher

$R^1$, $R^3$, $R^4$, $R^5$, X und Y die oben angegebene Bedeutung haben,

in einer anschließenden 3. Stufe mit Aminen der Formel (VI),

$R^2$-$NH_2$ (VI)

in welcher

$R^2$ die oben angegebene Bedeutung hat,

gegebenenfalls in Gegenwart eines Verdünnungsmittels wie beispielsweise Tetrahydrofuran sowie gegebenenfalls in Gegenwart einer Base wie beispielsweise Natriumhydroxid oder Kaliumhydroxid bei Temperaturen zwischen 20 °C und 50 °C umsetzt.

Dabei ist es auch möglich und gegebenenfalls von Vorteil die Umsetzung der 1-unsubstituierten Triazolinon-Hydrazone der Formel (VIII) mit (Thio)Chlorameisensäureestern der Formel (IX) und die anschließende Umsetzung der so erhältlichen Triazolinone (X) mit Aminen der Formel (VI) in einem Reaktionsschritt in einem sogenannten Eintopfverfahren durchzuführen (vgl. Beispiel 3).

Die 1-unsubstituierten 4-Amino-triazolinone der Formel (III) sind bekannt oder erhältlich in Analogie zu bekannten Verfahren (vergl. z.B. J. Heterocycl. Chem. 16, 403 [1979]; J.Heterocycl. Chem. 17, 1691 [1980]; Europ, J. Med. Chem. 18, 215 [1983]; Chem. Ber. 98, 3025 [1965] oder Liebigs Ann. Chem. 637, 135 [1960]).

Die Aldehyde oder Ketone der Formel (VII) sind allgemein bekannte Verbindungen der organischen Chemie.

Die 1-unsubstituierten Triazolinon-Hydrazone der Formel (VIII) sind zum großen Teil bekannt (vgl. z.B. J. Heterocycl. Chem. 20, 77-80 [1983]; J. Heterocycl. Chem. 16, 403-407 [1979]; Chim. Acta. Turc. 7, 269-290 [1979]; J. chem. Soc.; Perkin Trans. II, 1973, 9-11; J. org. Chem. 36, 2190-2192 [1971]).

Die (Thio)Chlorameisensäureester der Formel (IX) sind allgemein bekannte Verbindungen der organischen Chemie.

Die als Zwischenprodukte genannten Triazolinone der Formel (X) sind teilweise bekannt (vgl. z.B. Acta. Pol. Pharm. 38, 153-162 [1981] bzw. C.A. 95: 203841j).

Noch nicht bekannt und ebenfalls Gegenstand der vorliegenden Anmeldung sind Triazolinone der Formel (Xa),

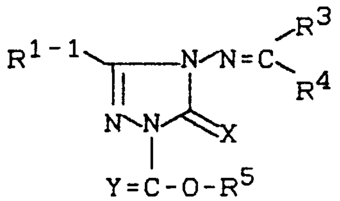

(Xa)

in welcher

$R^{1-1}$ für geradkettiges oder verzweigtes Alkyl mit 1 bis 4, insbesondere mit 1 bis 3 Kohlenstoffatomen steht; ganz besonders bevorzugt steht $R^{1-1}$ für Methyl, und

$R^3$, $R^4$, $R^5$, X und Y die oben angegebenen Bedeutungen haben,

$R^5$ steht vorzugsweise für Methyl, Ethyl, n- oder i-Propyl, n-, i-, s- oder t-Butyl oder für gegebenenfalls ein- bis zweifach, gleich oder verschieden substituiertes Phenyl oder Benzyl, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy oder Trifluormethylthio;

X und Y stehen jeweils unabhängig voneinander für Sauerstoff und Schwefel vorzugsweise für Sauerstoff.

Die zur Durchführung des erfindungsgemäßen Verfahrens (b) als Ausgangsstoffe benötigten 1H-Triazolinone sind durch die Formel (III) allgemein definiert. In dieser Formel (III) stehen $R^1$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die 1H-Triazolinone der Formel (III) sind bekannt oder erhältlich in Analogie zu bekannten Verfahren (vgl. z.B. J.Heterocycl. Chem. 16, 403 [1979]; J. Heterocycl. Chem. 17, 1691 [1980]; Europ. J. med. Chem. 18, 215 [1983]; Chem. Ber. 98, 3025 [1965]; Liebigs Ann. Chem. 637, 135 [1960]).

Die zur Durchführung des erfindungsgemäßen Verfahrens (b) weiterhin als Ausgangsstoffe benötigten Iso(thio)cyanate sind durch die Formel (IV) allgemein definiert. In dieser Formel (IV) stehen $R^2$ und Y vorzugsweise für diejenigen Reste, die bereits im Zusammenhang mit der Beschreibung der erfindungsgemäßen Stoffe der Formel (I) als bevorzugt für diese Substituenten genannt wurden.

Die Iso(thio)cyanate der Formel (IV) sind größtenteils bekannte Verbindungen der organischen Chemie. Die Verbindungen 2,2,2-Trifluorisopropylcyanat und 2,2,2-Trifluor-1,1-dimethylethylcyanat sind noch nicht bekannt, lassen sich jedoch nach bekannten Methoden herstellen. So erhält man Iso(thio)cyanate beispielsweise, wenn man die entsprechenden Amine mit Phosgen gegebenenfalls in Gegenwart einer Base wie beispielsweise Triethylamin umsetzt (vgl. z.B. DE-OS 28 04 082, DE-OS 25 12 514, US-P 35 84 028, US-P 33 11 654, JP 50/29599, Synthesis 1985, page 682 oder J. Am. Chem. Soc. 77 1901-1902 (1955)).

Die zur Durchführung des erfindungsgemäßen Verfahrens (c) als Ausgangsstoffe benötigten Triazolinone sind durch die Formel (V) allgemein definiert. In dieser Formel (V) stehen $R^1$, X und Y vorzugsweise für diejenigen Reste, die bereits im Zusammenhang mit der Beschreibung der erfindungsgemäßen Stoffe der Formel (I) als bevorzugt für diese Substituenten genannt wurden.

Die Triazolinone der Formel (V) sind teilweise bekannt (vgl. z.B. J. Heterocycl. Chem. 17, 1691-1696 [1980]).

Noch nicht bekannt und ebenfalls Gegenstand der vorliegenden Anmeldung sind Triazolinone der Formel (Va),

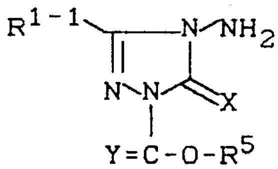

(Va)

in welcher

$R^{1-1}$ für geradkettiges oder verzweigtes Alkyl mit 1 bis 4, insbesondere mit 1 bis 3 Kohlenstoffatomen, besonders bevorzugt für Methyl steht und

$R^5$, X und Y die oben angegebene Bedeutung haben.

Man erhält sie in Analogie zur Herstellung der bekannten Verbindungen der Formel (V), wenn man 1H-Triazolinone der Formel (IIIa),

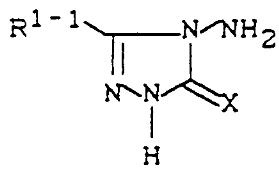

(IIIa)

in welcher

$R^{1-1}$ und X die oben angegebene Bedeutung haben,

mit (Thio)Chlorameisensäureestern der Formel (IX),

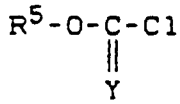

(IX)

in welcher

$R^5$ und Y die oben angegebene Bedeutung haben,

gegebenenfalls in Gegenwart eines Verdünnungsmittels wie beispielsweise Tetrahydrofuran und gegebenenfalls in Gegenwart eines Reaktionshilfsmittels wie beispielsweise Kalium-t-butylat oder Natriumhydrid bei Temperaturen zwischen - 20 °C und + 40 °C umsetzt (vgl. auch die Herstellungsbeispiele).

Die 1H-Triazolinone der Formel (IIIa) sind bekannt oder erhältlich in Analogie zu bekannten Verfahren (vgl. z.B. J. Heterocycl. Chem. 16, 403 [1979]; J. Heterocycl. Chem. 17, 1691 [1980]; Europ. J. med. Chem. 18, 215 [1983]; Chem. Ber. 98, 3025 [1965]; Liebigs Ann. Chem. 637, 135 [1960]).

Als Säuren zur Durchführung des erfindungsgemäßen Verfahrens (a) kommen als üblicherweise für Hydrazonspaltungen verwendbaren anorganischen und organischen Säuren infrage. Vorzugsweise verwendet man anorganische Mineralsäuren wie Chlorwasserstoffsäure, Schwefelsäure oder Phosphorsäure.

Als Verdünnungsmittel zur Durchführung des erfindungsgemäßen Verfahrens (a) kommen alle üblichen organischen oder anorganischen Lösungsmittel infrage. Vorzugsweise verwendet man polare mit Wasser mischbare organische Lösungsmittel, insbesondere Alkohole, wie Methanol, Ethanol, Propanol, oder Butanol, deren Gemische mit Wasser oder reines Waser als Verdünnungsmittel.

Die Reaktionstemperaturen können bei der Durchführung des erfindungsgemäßen Verfahrens (a) in einem größeren Bereich variiert werden. Im allgemeinen arbeitet man bei Temperaturen zwischen 20° C und 150° C, vorzugsweise bei Temperaturen zwischen 50° C und 120° C.

Das erfindungsgemäße Verfahren (a) wird üblicherweise bei Normaldruck oder unter vermindertem Druck durchgeführt. Arbeitet man unter Vermindertem Druck, so kommen Druckbereiche zwischen 20 und 400 mbar, vorzugsweise zwischen 100 und 200 mbar infrage.

Zur Durchführung des erfindungsgemäßen Verfahrens (a) setzt man pro Mol an Hydrazon der Formel (II) im allgemeinen 1 bis 50 Mol, vorzugsweise 1 bis 20 Mol an Säure ein. Dabei löst man das Hydrazon der Formel (II) in einer geeigneten Menge an Verdünnungsmittel, setzt dann die erforderliche Menge Säure zu und engt die Mischung unter vermindertem Druck über mehrere Stunden langsam ein.

In einer besonderen Durchführungsform ist es auch möglich, das erfindungsgemäße Verfahren (a) und die Herstellung der dafür erforderlichen Vorprodukte der Formel (II) in einem Reaktionsschritt in einem sogenannten Eintopfverfahren durchzuführen.

Dabei gibt es die Möglichkeit, als Ausgangsverbindungen die Triazolinone der Formel (X) zu wählen und diese nacheinander im Eintopfverfahren mit Aminen der Formel (VI) und anschließend mit Säure gemäß dem erfindungsgemäßen Verfahren (a) umzusetzen (vgl. hierzu auch die Herstellungsbeispiele) oder alternativ, als Ausgangsverbindungen die Triazolin-Hydrazone der Formel (VIII) zu wählen und diese nacheinander im Eintopfverfahren mit (Thio)Chlorameisensäureestern der Formel (IX), dann mit Aminen der Formel (VI) und anschließend mit Säure gemäß dem erfindungsgemäßen Verfahren (a) umzusetzen.

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder diethylether, Nitrile, wie Acetonitril oder Propionitril, Amid, wie Dimethylformamid, Dimethylacetamid, N-Methylformanilid, N-Methylpyrrolidon oder Hexamethylphosphorsäuretriamid oder Ester, wie Essigsäureethylester.

Das erfindungsgemäße Verfahren (b) wird gegebenenfalls in Gegenwart eines geeigneten Reaktionshilfsmittels durchgeführt. Als solche kommen alle üblichen anorganischen oder organischen Basen in Frage. Hierzu gehören beispielsweise tertiäre Amine, wie Triethylamin, N,N-Dimethylanilin, N,N-Diethylbenzylamin, N,N-Dimethylcyclohexylamin oder Dibutylzinndilaureat, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 150 °C, vorzugsweise bei Temperaturen zwischen 20 °C und 100 °C.

Zur Durchführung des erfindungsgemäßen Verfahrens (b) setzt man pro Mol an 1H-Triazolinon der Formel (III) im allgemeinen 1.0 bis 2.0 Mol, vorzugsweise 1.0 bis 1.5 Mol an Iso(thio)cyanat der Formel (IV) und gegebenenfalls 0.001 bis 2.0 Mol, vorzugsweise 0.001 bis 1.0 Mol an Reaktionshilfsmittel ein.
Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester, oder Sulfoxide, wie Dimethylsulfoxid.

Das erfindungsgemäße Verfahren (c) kann gegebenenfalls in Gegenwart eines geeigneten Reaktionshilfsmittels durchgeführt werden. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 120 °C, vorzugsweise bei Temperaturen zwischen 20 °C und 50 °C.

Zur Durchführung des erfindungsgemäßen Verfahrens (c) setzt man pro Mol an Triazolinon der Formel (V) im allgemeinen 1.0 bis 5.0 Mol, vorzugsweise 1.0 bis 2.5 Mol an Amin der Formel (VI) und gegebenenfalls 1.0 bis 2.0 Mol, vorzugsweise 1.0 bis 1.2 Mol an Reaktionshilfsmittel ein.
Die Reaktionsdurchführung, Aufarbeitung und Isolierung der Reaktionsprodukte erfolgt nach allgemein üblichen Methoden.

In einer besonderen Durchführungsform ist es auch möglich, das erfindungsgemäße Verfahren (c) und die Herstellung der dafür erforderlichen Vorprodukte der Formel (V) in einem Reaktionsschritt in einem sogenannten Eintopfverfahren durchzuführen.

Man geht dabei aus von 1H-Triazolinonen der Formel (III) und setzt diese nacheinander im Eintopverfahren zunächst mit (Thio)Chlorameisensäureestern der Formel (IX) und anschließend mit Aminen der Formel (IV) gemäß dem erfindungsgemäßen Verfahren (c) um.

Eine weitere Methode, erfindungsgemäße Verbindungen der Formel (I) zu erhalten, besteht darin, daß man Oxadiazolinone der Formel (XI),

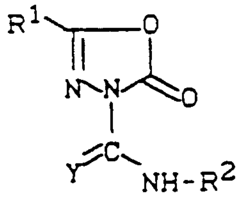

(XI)

in welcher

$R^1$, $R^2$ und Y die oben angegebene Bedeutung haben,

mit Hydrazinhydrat in Gegenwart eines geeigneten Verdünnungsmittels wie beispielsweise Methanol oder Ethanol bei Temperaturen zwischen 20 °C und 100 °C umsetzt und die so erhältlichen Carbazidsäure-Derivate der Formel (XII),

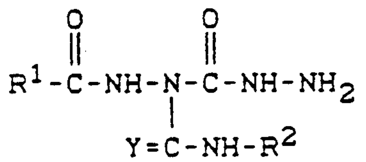

(XII)

in welcher

$R^1$, $R^2$ und Y die oben angegebene Bedeutung haben

in Gegenwart eines geeigneten Verdünnungsmittels wie beispielsweise Toluol, Chlorbenzol oder Dichlorbenzol bei Temperaturen zwischen 80 °C und 200 °C thermisch cyclisiert.

Oxadiazolinone der Formel (XI) sind bekannt (vgl. z.B. FR 14 15 605 bzw. C.A. 64: P5105g sowie NL 6 510 645 bzw. C.A. 65: P2274d-f) oder erhältlich nach allgemein bekannten Verfahren beispielsweise durch Umsetzung der entsprechenden 4H-Oxadiazolinone mit Iso(thio)cyanaten der Formel (IV) in Analogie zur Durchführung des erfindungsgemäßen Verfahrens (b) oder zur Synthese der Vorprodukte der Formel (II).

Die Reinigung der Endprodukte der Formel (I) erfolgt mit Hilfe üblicher Verfahren, beispielsweise säulenchromatographisch oder durch Umkristallisieren.
Die Charakterisierung erfolgt mit Hilfe des Schmelzpunktes der bei nicht kristallisierenden Verbindungen mit Hilfe des Protonen-Kernresonanzspektrums.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonom, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea und Mercurialis.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung mono- und dikotyler Unkräuter insbesondere in dikotylen Kulturen wie beispielsweise Zuckerrüben einsetzen.

Zu erwähnen ist besondere auch die hervorragende Wirksamkeit gegen das schwer bekämpfbare Problemunkraut Bingelkraut (Mercurialis).

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in

polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwaserstoffe, wie Cycvlohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. Ammoniumsalze und natürliche Gesteinsmehle, wie Kaoline, Tonerden, Talkum, Kreide, Quarz, Attapulgit,

Montmorillonit oder Diatomeenderde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichstprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N′-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)3-methylthio-1,2,4-triazin-5(4H)-on zur Umkrautbekämpfung in Sojabohnen, infrage. Auch Mischungen mit 5-(2-Chlor-4-trifluormethyl-phenoxy)-2-nitro-benzoesäure; Chloressigsäure-N-(methoxymethyl)-2,6-diethylanilid; Methyl-6,6-dimethyl-2,4-dioxo-3-[1-(2-propenyloxyamino)-butyliden]-cyclohexancarbonsäure; Methyl-5-(2,4-dichlorphenoxy)-2-nitrobenzoat; 5-Amino-4-chlor-2-phenyl-2,3-dihydro-3-oxy-pyridazin; Ethyl-2-{[(4-chlor-6-methoxy-2-pyrimidinyl)-aminocarbonyl]-aminosulfonyl}benzoat; exo-1-Methyl-4-(1-methylethyl)-2-(2-methylphenyl-methoxy)-7-oxabicyclo(2,2,1)-heptan; Ethyl-2-{[(4-chlor-6-methoxy-2-pyridinyl)-aminocarbonyl]-aminosulfonyl}-benzoat; 3,6-Dichlor-2-pyridincarbonsäure; N,S-Diethyl-N-cyclohexyl-thiolcarbamat; 3-(Methoxycarbonylaminophenyl)-N-phenyl-carbamat; 2-[4-(2,4-Dichlorphenoxy)-phenoxy]-propionsäure, deren Methyl- oder deren Ethylester, 4-Amino-6-t-butyl-3-ethylthio-1,2,4-triazin-5(4H)-on; 2-{4-[(6-Chlor-2-benzoxazolyl)-oxy]-phenoxy}propansäure, deren Methyl- oder deren Ethylester; 2-[4-(3,5-Dichlorpyrid-2-yloxy)-phenoxy]-propionsäure-(trimethylsilylmethylester); 5-(2-Chlor-4-trifluormethylphenoxy)-N-methylsulfonyl-2-nitrobenzamid;2-{-4-[(3-Chlor-5-(trifluormethyl)-2-pyridinyl)-oxy]-phenoxy}-propansäure bzw. -propansäureethylester; 2-[4-(2,4-Dichlorphenoxy)-phenoxy]-propionsäure-methylester; 2-[5-Methyl-5-(1-methylethyl)-4-oxo-2-imidazolin-2-yl]-3-chinolincarbonsäure; 1-Isobutylamoniocarbonyl-2-imidazolidinon; 3-Cyclohexyl-5,6-trimethylen-uracil; N-Methyl-2-(1,3-benzthiazol-2-yloxy)-acetanilid; 2-Chlor-N-(2,6-dimethylphenyl)-N-[(1H)-pyrazol-1-yl-methyl]-acetamid; 2-Ethyl-6-methyl-N-(1-methyl-2-methoxyethyl)-chloracetanilid; 3-(Ethoxycarbonylaminophenyl)-N-3′-methylphenyl)-carbamat; 2-[1-(Ethoxamino)-butyliden]-5-(2-ethylthiopropyl)-1,3-cyclohexadion;N,N-Diisoproyl-S-(2,3,3-trichlorallyl)-thiolcarbamat oder 2,6-Dinitro-4-trifluormethyl-N,N-dipropylanilin sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1

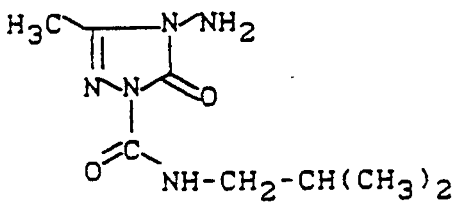

(Verfahren a)

Zu 11,1 g (0,04 Mol) 1-(N-Isobutylcarbamoyl)-4-isopropylidenimino-3-methyl-1,2,4-triazolin-5-on in 100 ml Ethanol gibt man 20 ml konzentrierte Salzsäure und engt die Lösung bei 60 °C und ca. 200 mbar im Laufe von 5 Stunden am Rotationsverdampfer ein. Der Rückstand wird durch Verreiben mit Ethanol/Diethylether (1:1) zur Kristallisation gebracht und an der Luft getrocknet.
Man erhält 4,3 g (50 % der Theorie) an 4-Amino-1-(N-isobutylcarbamoyl)-3-methyl-triazolin-5-on vom Schmelzpunkt 183 °C.

Beispiel 2

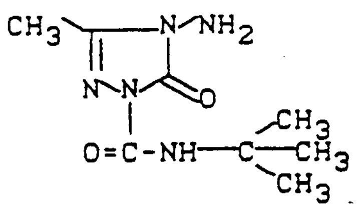

(Verfahren b)

Zu 3.42 g (0.03 Mol) 4-Amino-3-methyl-1,2,4-(1H)-triazolin-5-on in 80 ml absolutem Acetonitril gibt man 3,6 g (0.036 Mol) t-Butylisocyanat und 0,05 g bis 0,1 g Diazabicycloundecen (DBU), rührt 2 Stunden bei 20 °C, engt im Vakuum ein, nimmt den Rückstand in Dichlormethan auf, wäscht mit Wasser, trocknet über

Natriumsulfat, engt im Vakuum ein und kristallisiert den Rückstand durch Verreiben mit Diethylether.

Man erhält 5,0 g (78,3 % der Theorie) an 4-Amino-1-(N-t-butylcarbamoyl)-3-methyl-1,2,4-triazolin-5-on vom Schmelzpunkt 132 °C.

Beispiel 3

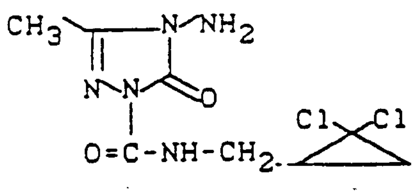

(Verfahren a -Eintopfvariante)

Zu 8,2 g (0,03 Mol) 4-Isopropylidenimino-3-methyl-1-phenoxycarbonyl-1,2,4-triazolin-5-on in 50 ml absolutem Tetrahydrofuran gibt man 4,2 g (0.03 Mol) (2,2-Dichlorcyclopropyl)methylamin, rührt dann 12 Stunden bei 20 °C, engt im Vakuum ein, nimmt den Rückstand in 100 ml Ethanol auf, gibt 3 ml konzentrierte Salzsäure zu und rührt 3 bis 4 Stunden bei 60 °C und 200 mbar. Zur Aufarbeitung wird im Vakuum eingeengt, der Rückstand in Dichlormethan aufgenommen, dreimal mit gesättigter wässriger Natriumhydrogencarbonatlösung gewaschen, über Natriumsulfat getrocknet und im Vakuum eingeengt. Der Rückstand wird durch Verreiben mit Diethylether zur Kristallisation gebracht.

Man erhält 4,4 g (52 % der Theorie) an 4-Amino-1-[N-(2,2-Dichlorcyclopropylmethyl)carbamoyl]-3-methyl-1,2,4-triazolin-5-on vom Schmelzpunkt 149 °C.

Beispiel 4

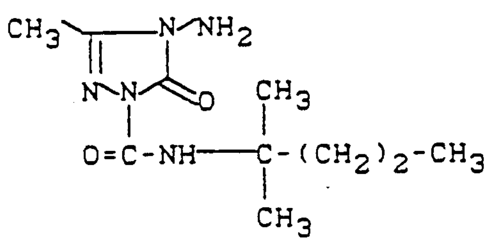

(Verfahren c)

Zu 3,2 g (0.0137 Mol) 4-Amino-3-methyl-1-phenoxycarbonyl-1,2,4-triazolin-5-on in einem Gemisch aus 25 ml Tetrahydrofuran und 10 ml Dioxan gibt man 8,9 g (0.088 Mol) 1,1-Dimethylbutylamin, erhitzt 24 Stunden auf Rückflußtemperatur, engt im Vakuum ein, nimmt den Rückstand in Dichlormethan auf, wäscht mit zweiprozentiger Natronlauge und Wasser, trocknet über Natriumsulfat, entfernt das Lösungsmittel im Vakuum und kristallisiert den Rückstand durch Verreiben mit Diethylether.

Man erhält 1,9 g (61 % der Theorie) an 4-Amino-1-[N-(1,1-dimethylbutyl)-carbamoyl]-3-methyl-1,2,4-triazolin-5-on vom Schmelzpunkt 110 °C.

In entsprechender Weise und gemäß den allgemeinen Angaben zur Herstellung erhält man die folgenden substituierten Triazolinone der allgemeinen Formel (I):

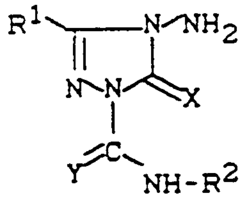

(I)

Tabelle 1

| Bsp. Nr. | $R^1$ | $R^2$ | X | Y | physikalische Eigenschaften |
|---|---|---|---|---|---|
| 5 | $CH_3$ | 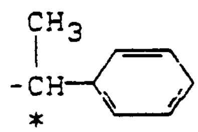 R-Konfiguration | O | O | $^1$H-NMR*) 1,5 (d) |
| 6 | $CH_3$ | 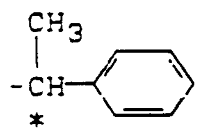 S-Konfiguration | O | O | $^1$H-NMR*) 1,5 (d) |
| 7 | $C_2H_5$ | 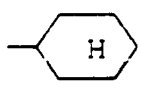 | O | O | Fp. 139° C |

Tabelle 1 - Fortsetzung

| Bsp. Nr. | $R^1$ | $R^2$ | X | Y | physikalische Eigenschaften |
|---|---|---|---|---|---|
| 8 | H | 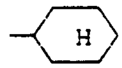 | O | O | Fp. 161° C |
| 9 | $CH_3$ | $-CH(CH_3)_2$ | O | O | Fp. 63° C |
| 10 | $CH_3$ | $-(CH_2)_5-CH_2Cl$ | O | O | $^1H$-NMR*) 1,45 (m,4H) |
| 11 | $CH_3$ | 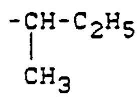 | O | O | $^1H$-NMR*) 0,95 (t,3H) |
| 12 | $CH_3$ | 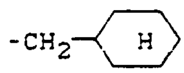 | O | O | Fp. 133° C |
| 13 | $CH_3$ | $-CH(C_2H_5)_2$ | O | O | Fp. 103° C |
| 14 | $CH_3$ | 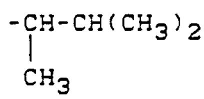 | O | O | Fp. 103° C |
| 15 | $CH_3$ | 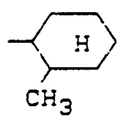 | O | O | Fp. 105° C |
| 16 | $CH_3$ | 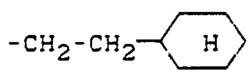 | O | O | Fp. 135° C |
| 17 | $CH_3$ | 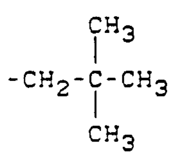 | O | O | Fp. 106° C (Zers.) |

Tabelle 1 - Fortsetzung

| Bsp. Nr. | $R^1$ | $R^2$ | X | Y | physikalische Eigenschaften |
|---|---|---|---|---|---|
| 18 | $CH_3$ | 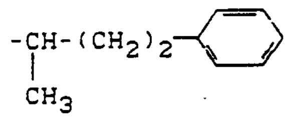 | O | O | $n_D^{20}$ 1.5496 |
| 19 | $CH_3$ | 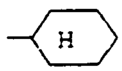 | S | O | Fp 128° C |
| 20 | $CH_3$ | 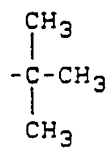 | S | O | Fp 100° C |
| 21 | H | $-(CH_2)_5-CH_2Cl$ | O | O | Fp 131° C |
| 22 | $CH_3$ | 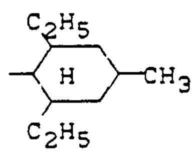 | O | O | Fp 153° C |
| 23 | $CH_3$ | 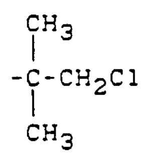 | O | O | Fp 118° C |
| 24 | $CH_3$ | $-CH_2-CH=CH_2$ | O | O | Fp 92° C |
| 25 | $CH_3$ | 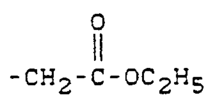 | O | O | Fp 127° C |

Tabelle 1 - Fortsetzung

| Bsp. Nr. | $R^1$ | $R^2$ | X | Y | physikalische Eigenschaften |
|---|---|---|---|---|---|
| 26 | $CH_3$ | 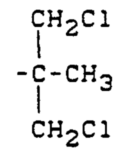 | O | O | $n_D^{22}$ 1.5055 |
| 27 | $CH_3$ | 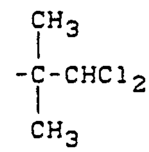 | O | O | Fp 176° C |
| 28 | $C_2H_5$ | 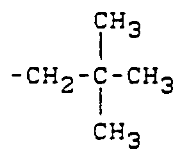 | O | O | $^1$H-NMR*): 4,4; 7,95 |
| 29 | $CH_3$ | 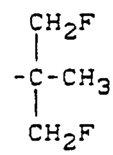 | O | O | Fp 133° C |
| 30 | $C_2H_5$ | 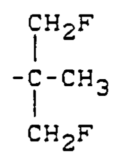 | O | O | Fp30-40° C |
| 31 | $CH_3$ | 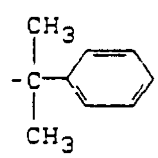 | O | O | $^1$H-NMR*): 4,36; 8,23 |

Tabelle 1 - Fortsetzung

| Bsp. Nr. | $R^1$ | $R^2$ | X | Y | physikalische Eigenschaften |
|---|---|---|---|---|---|
| 32 | $CH_3$ | 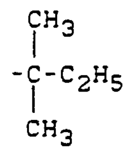 | O | O | Fp 99° C |
| 33 | $CH_3$ | 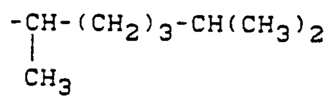 | O | O | $^1$H-NMR*): 4,40; 7,61 |
| 34 | $CH_3$ | 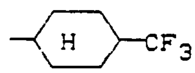 | O | O | Fp 162° C |
| 35 | $CH_3$ | 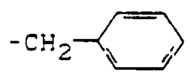 | O | O | Fp 198° C |
| 36 | $CH_3$ | $-(CH_2)_3-CH_3$ | O | O | Fp 108-109 °C |
| 37 | $CH_3$ | $CH_3$ | O | O | Fp 168-170 °C |
| 38 | $CH_3$ | $-(CH_2)_2-CH_3$ | O | O | Fp 134-136 °C |
| 39 | $CH_3$ | 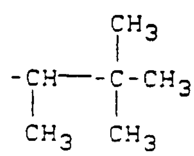 | O | O | Fp 149° C |

Tabelle 1 - Fortsetzung

| Bsp. Nr. | $R^1$ | $R^2$ | X | Y | physikalische Eigenschaften |
|---|---|---|---|---|---|
| 40 | $CH_3$ | 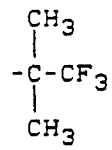 | O | O | Fp 149-151 °C |
| 41 | $CH_3$ | 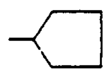 | O | O | Fp 93-94 °C |
| 42 | $CH_3$ | $-CH_2-CH_2-CN$ | O | O | Fp 175-178 °C |
| 43 | $CH_3$ | 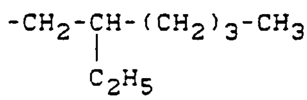 | O | O | Fp 91-92 °C |
| 44 | $C_2H_5$ | 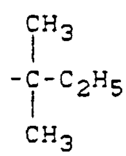 | O | O | Fp 102-103 °C |
| 45 | $CH_3$ | 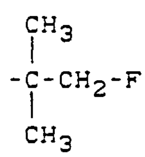 | O | O | Fp 178 °C |
| 46 | $CH_3$ | 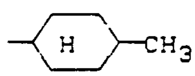 | O | O | Fp 113 °C |
| 47 | $CH_3$ | 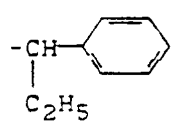 | O | O | Fp 109 °C |

Tabelle 1 - Fortsetzung

| Bsp. Nr. | $R^1$ | $R^2$ | X | Y | physikalische Eigenschaften |
|---|---|---|---|---|---|
| 48 | $CH_3$ | 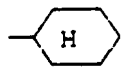 | O | O | Fp 148 °C |
| 49 | $CH_3$ | 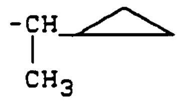 | O | O | $^1$H-NMR*): 0.35-0.6; 0.93 |
| 50 | $CH_3$ | 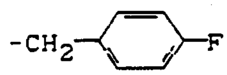 | O | O | Fp 175 °C |
| 51 | $CH_3$ | 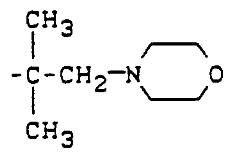 | O | O | Fp 211 °C (Hydrochlorid) |
| 52 | $CH_3$ | 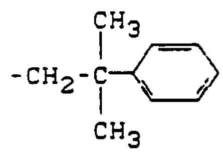 | O | O | Fp 152 °C |
| 53 | $CH_3$ | $-C_2H_5$ | O | O | Fp 185 °C |
| 54 | $CH_3$ | 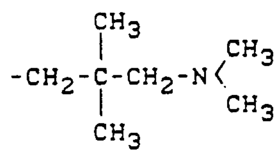 | O | O | Fp 198 °C (Hydrochlorid) |
| 55 | $CH_3$ | 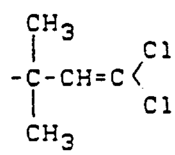 | O | O | Fp 135 °C |

Tabelle 1 - Fortsetzung

| Bsp. Nr. | $R^1$ | $R^2$ | X | Y | physikalische Eigenschaften |
|---|---|---|---|---|---|
| 56 | $CH_3$ | 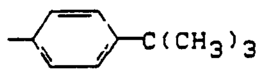 | O | O | Fp 200-203 °C |
| 57 | $CH_3$ | 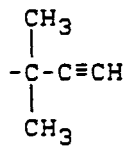 | O | O | Fp 119 °C |
| 58 | $CH_3$ | 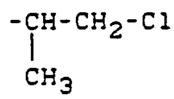 | O | O | Fp 136 °C |
| 59 | $CH_3$ | 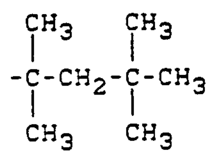 | O | O | Fp 122 °C |
| 60 | $CH_3$ | 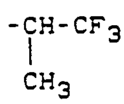 | O | O | Fp 141 °C |
| 61 | $CH_3$ | 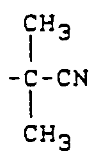 | O | O | Fp 176 °C |
| 62 | $CH_3$ | 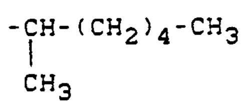 | O | O | Fp 67 °C |
| 63 | $CH_3$ | 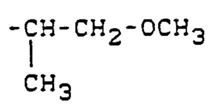 | O | O | Fp 120 °C |

Tabelle 1 - Fortsetzung

| Bsp. Nr. | $R^1$ | $R^2$ | X | Y | physikalische Eigenschaften |
|---|---|---|---|---|---|
| 64 | $CH_3$ | $-(CH_2)_2-OCH_3$ | O | O | Fp 114 °C |
| 65 | $CH_3$ | 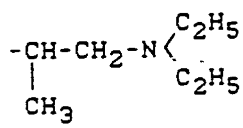 | O | O | Fp 84 °C (Hydro-chlorid) |
| 66 | $CH_3$ | $-(CH_2)_3-CH_3$ | O | S | Fp 147 °C |
| 67 | $CH_3$ | 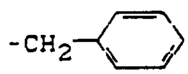 | O | S | Fp 195 °C |
| 68 | $CH_3$ | $-C_2H_5$ | O | S | Fp 205 °C |
| 69 | $CH_3$ | $-CH_3$ | O | S | Fp 212 °C |
| 70 | $CH_3$ | 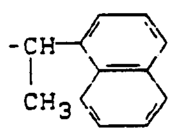 | O | O | Fp 139 °C |
| 71 | $CH_3$ | 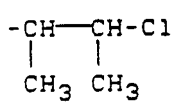 | O | O | Fp 114 °C |
| 72 | $CH_3$ | 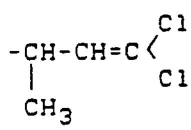 | O | O | $n_D^{20}$ 1.5328 |
| 73 | $CH_3$ | 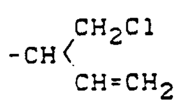 | O | O | Fp 120 °C |

Tabelle 1 - Fortsetzung

| Bsp. Nr. | $R^1$ | $R^2$ | X | Y | physikalische Eigenschaften |
|---|---|---|---|---|---|
| 74 | $CH_3$ | 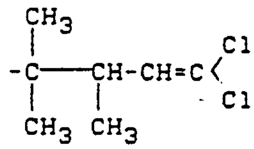 | O | O | $n_D^{20}$ 1.3840 |
| 75 | $CH_3$ | 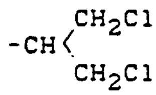 und 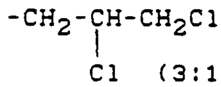 (3:1) | O | O | Fp 158 °C |
| 76 | $CH_3$ | 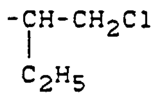 | O | O | Fp 147 °C |
| 77 | $CH_3$ | 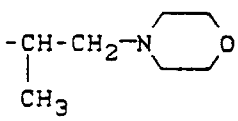 | O | O | $n_D^{22}$ 1.4995 (Hydro-chlorid) |
| 78 | $CH_3$ | 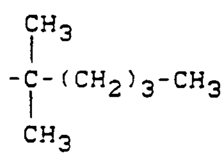 | O | O | $n_D^{22}$ 1.4891 |
| 79 | $-CH_3$ | 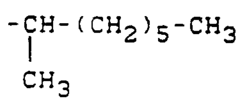 | O | O | $n_D^{20}$ 1.4920 |
| 80 | $CH_3$ | 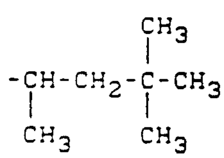 | O | O | Fp 140 °C |

Tabelle 1 - Fortsetzung

| Bsp. Nr. | $R^1$ | $R^2$ | X | Y | physikalische Eigenschaften |
|---|---|---|---|---|---|
| 81 | $CH_3$ | 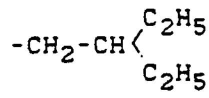 | O | O | Fp 115 C |
| 82 | $CH_3$ | 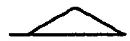 | O | O | Fp 134 °C |
| 83 | $CH_3$ | 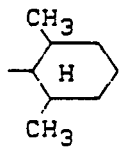 | O | O | Fp 164 °C |
| 84 | $CH_3$ | 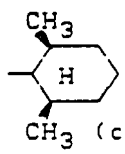 (cis) | O | O | Fp 144 °C |
| 85 | $CH_3$ | 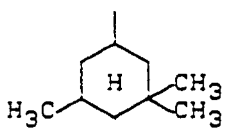 | O | O | Fp 121 °C |
| 86 | $CH_3$ | 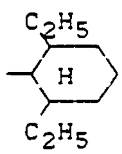 | O | O | $^1$H-NMR*): 2.33 |
| 87 | $CH_3$ | $-CH_2-CF_3$ | O | O | Fp 152 °C |
| 88 | $CH_3$ | 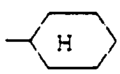 | O | S | Fp 178 °C |

Tabelle 1 - Fortsetzung

| Bsp. Nr. | $R^1$ | $R^2$ | X | Y | physikalische Eigenschaften |
|---|---|---|---|---|---|
| 89 | $CH_3$ | 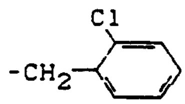 | O | O | Fp 156 °C |
| 90 | $CH_3$ | 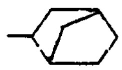 | O | O | Fp 167 °C |
| 91 | $CH_3$ | 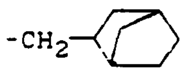 | O | O | Fp 113 °C |
| 92 | $CH_3$ | 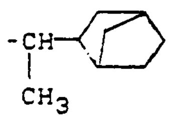 | O | O | Fp 133 °C |
| 93 | $CH_3$ | 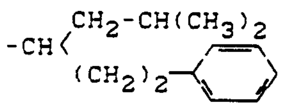 | O | O | Fp 98 °C |
| 94 | $CH_3$ | 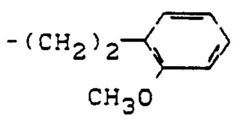 | O | O | Fp 156 °C |
| 95 | $CH_3$ | 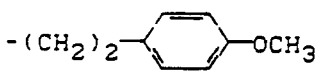 | O | O | Fp 210 °C |
| 96 | $CH_3$ | 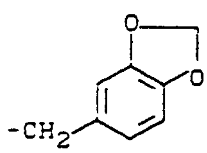 | O | O | Fp 175 °C |

Tabelle 1 - Fortsetzung

| Bsp. Nr. | $R^1$ | $R^2$ | X | Y | physikalische Eigenschaften |
|---|---|---|---|---|---|
| 97 | $CH_3$ | 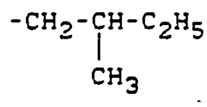 | O | O | Fp 106 °C |
| 98 | $CH_3$ | $-(CH_2)_2-CH(CH_3)_2$ | O | O | Fp 104 °C |
| 99 | $CH_3$ | 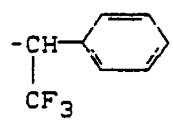 | O | O | Fp 120 °C (Zers.) |
| 100 | $CH_3$ | 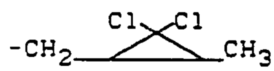 | O | O | Fp 108 °C |
| 101 | $CH_3$ | 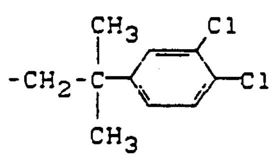 | O | O | Fp 151 °C |
| 102 | $CH_3$ | 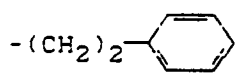 | O | O | Fp 151 °C |
| 103 | $CH_3$ | 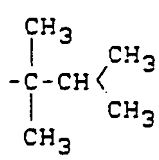 | O | O | Fp 134 °C |
| 104 | $CH_3$ | 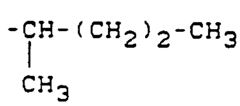 | O | O | $n_D^{20}$ 1.4972 |

Tabelle 1 - Fortsetzung

| Bsp. Nr. | $R^1$ | $R^2$ | X | Y | physikalische Eigenschaften |
|---|---|---|---|---|---|
| 105 | $CH_3$ | $C_2H_5$<br>\|<br>$-C-C_2H_5$<br>\|<br>$C_2H_5$ | O | O | Fp 128 °C |
| 106 | $CH_3$ | 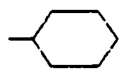 | O | O | Fp 101 °C |
| 107 | $CH_3$ | $-CH-(CH_2)_2-CH<^{CH_3}_{CH_3}$<br>\|<br>$CH_3$ | O | O | Fp 73 °C |
| 108 | $CH_3$ | $-(CH_2)_3-N<^{C_2H_5}_{C_2H_5}$ | O | O | $n_D^{20}$ 1.5815 (Hydro-chlorid) |
| 109 | $CH_3$ | $-(CH_2)_3-N<^{CH_3}_{CH_3}$ | O | O | Fp 210 °C (Hydro-chlorid) |
| 110 | $CH_3$ | $-CH-CH_2-CN$<br>\|<br>$C_2H_5$ | O | O | Fp 145 °C |
| 111 | $CH_3$ | $-CH-CH_2-CH-C_2H_5$<br>\| \|<br>$C_2H_5$ $CH_3$ | O | O | $n_D^{20}$ 1.4890 |
| 112 | $CH_3$ | $-CH-CH_2-CH<^{CH_3}_{CH_3}$<br>\|<br>$CH_3$ | O | O | $n_D^{20}$ 1.4858 |

Tabelle 1 - Fortsetzung

| Bsp. Nr. | $R^1$ | $R^2$ | X | Y | physikalische Eigenschaften |
|---|---|---|---|---|---|
| 113 | $CH_3$ | 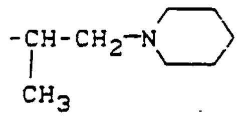 | O | O | Fp 108 °C (Hydrochlorid) |
| 114 | $CH_3$ | $-(CH_2)_4-CH_3$ | O | O | Fp 81 °C |
| 115 | $CH_3$ | 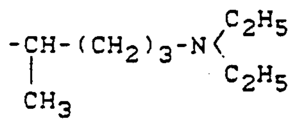 | O | O | $n_D^{20}$ 1.5100 (Hydrochlorid) |
| 116 | $CH_3$ | 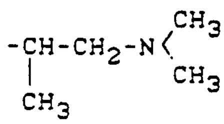 | O | O | $n_D^{20}$ 1.5150 (Hydrochlorid) |
| 117 | $CH_3$ | 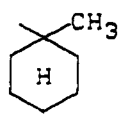 | O | O | Fp 157 °C |
| 118 | $CH_3$ | 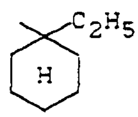 | O | O | Fp 116 °C |
| 119 | $CH_3$ | 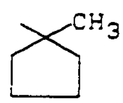 | O | O | Fp 145 °C |
| 120 | $CH_3$ | 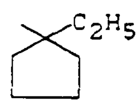 | O | O | Fp 118 °C |

Tabelle 1 - Fortsetzung

| Bsp. Nr. | $R^1$ | $R^2$ | X | Y | physikalische Eigenschaften |
|---|---|---|---|---|---|
| 121 | $CH_3$ | $-CH_2-CH_2-OC_2H_5$ | O | O | Fp 123 °C |
| 122 | $CH_3$ | 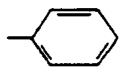 | O | O | Fp 213 °C |
| 123 | $CH_3$ | 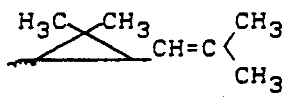 | O | O | Fp 93° C |
| 124 | $CH_3$ | 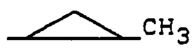 | O | O | Fp 93 °C |
| 125 | $CH_3$ | 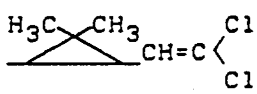 | O | O | 1H-NMR*): 1.58 (dd); 1.77 (t); 2.76 (t) |
| 126 | $CH_3$ | 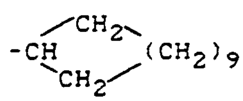 | O | O | Fp 142 °C |
| 127 | $CH_3$ | 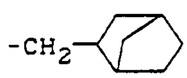 | O | O | Fp 123 °C (endo-Form) |
| 128 | $CH_3$ | 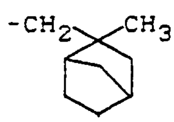 | O | O | Fp 131 °C (exo-Form) |
| 129 | $CH_3$ | 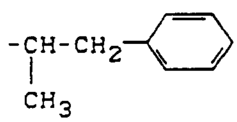 | O | O | Fp 133 °C |
| 130 | $CH_3$ | 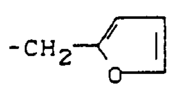 | O | O | Fp 125 °C |

Tabelle 1 - Fortsetzung

| Bsp. Nr. | $R^1$ | $R^2$ | X | Y | physikalische Eigenschaften |
|---|---|---|---|---|---|
| 131 | $CH_3$ | 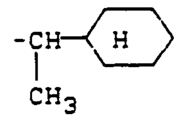 | O | O | Fp 117 °C |
| 132 | $CH_3$ | 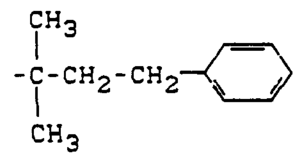 | O | O | $^1$H-NMR*): 1.45(s); 7.04-7.43 (m) |
| 133 | CH3 | 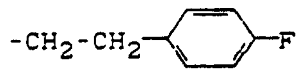 | O | O | Fp 168 °C |
| 134 | $CH_3$ | 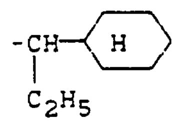 | O | O | Fp 118 °C |
| 135 | $CH_3$ | 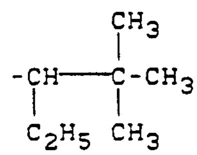 | O | O | Fp 157 °C |
| 136 | $CH_3$ | 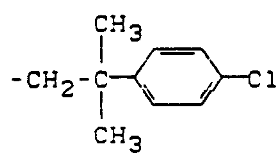 | O | O | Fp 180 °C |
| 137 | $CH_3$ | 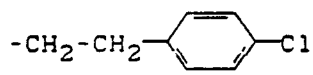 | O | O | Fp 188 °C |
| 138 | $CH_3$ | 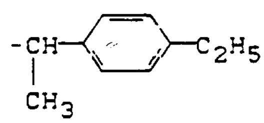 | O | O | Fp 95 °C |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Herstellung der Ausgangsverbindungen

Beispiel II-1

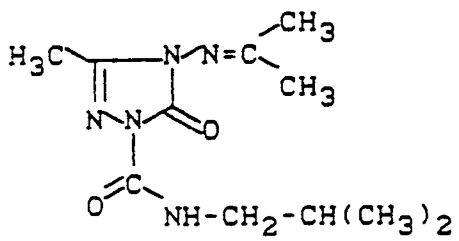

Zu 6 g (0,04 Mol) 4-Isopropylidenimino-3-methyl-1H-triazolin-5-on und 4 g (0,04 Mol) Triethylamin in 20 ml Dioxan gibt man bei 20 °C tropfenweise unter Rühren 12 g (0,12 Mol) Isobutylisocyanat und rührt nach beendeter Zugabe 3 Stunden bei 100 °C. Zur Aufarbeitung wird die Reaktionsmischung im Vakuum eingeengt, der Rückstand in 100 ml Dichlormethan aufgenommen, mehrfach mit jeweils 100 ml Wassser gewaschen, über Natriumsulfat getrocknet und im Vakuum vom Lösungsmittel befreit.
Man erhält 11,2 g (100 % der Theorie) an 1-(N-Isobutylcarbamoyl)-4-isopropylidenimino-3-methyl-1,2,4-triazolin-5-on als Öl.
$^1$H-NMR ($CDCl_3$): $\delta$ = 0,85 (d, 6H)ppm.

In entsprechender Weise und gemäß den allgemeinen Angaben zur Herstellung erhält man die folgenden Vorprodukte der allgemeinen Formel (II):

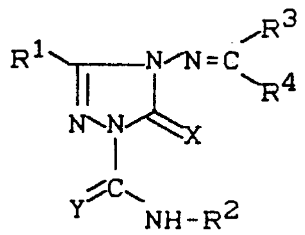

(II)

Tabelle 2

| Bsp. NR: | $R^1$ | $R^2$ | 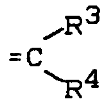 | X | Y | physikalische Eigenschaften |
|---|---|---|---|---|---|---|
| II-2 | $CH_3$ | 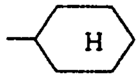 | $=C(CH_3)_2$ | O | O | Fp 125 °C |
| II-3 | $CH_3$ | 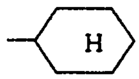 | $=CH-CH(CH_3)_2$ | O | O | $^1$H-NMR*): 3,9 |
| II-4 | $CH_3$ | $-CH(CH_3)-C_2H_5$ | $=C(CH_3)_2$ | O | O | Fp 87 °C |
| II-5 | $CH_3$ | $-CH(CH_3)_2$ | $=C(CH_3)_2$ | O | O | Fp 107 °C |
| II-6 | $CH_3$ | $-CH(C_2H_5)_2$ | $=C(CH_3)_2$ | O | O | Fp 68 °C |
| II-7 | $CH_3$ | $-CH-CH(CH_3)_2$ | $=C(CH_3)_2$ | O | O | $^1$H-NMR*): 0,54(d,$CH_3$) |

Tabelle 2 - Fortsetzung

| Bsp. Nr. | $R^1$ | $R^2$ | 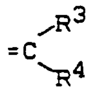 | X | Y | physikalische Eigenschaften |
|---|---|---|---|---|---|---|
| II-8 | $CH_3$ | 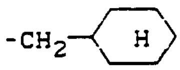 | $=C(CH_3)_2$ | O | O | Fp 115 °C |
| II-9 | $CH_3$ | 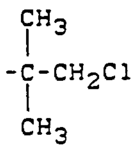 | $=C(CH_3)_2$ | O | O | Fp 88 °C |
| II-10 | $CH_3$ | 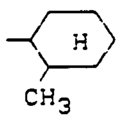 | $=C(CH_3)_2$ | O | O | Fp 139 °C |
| II-11 | $CH_3$ | 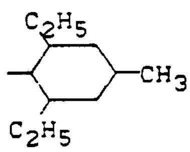 | $=C(CH_3)_2$ | O | O | Öl |
| II-12 | $CH_3$ | $-CH_2-COOC_2H_5$ | $=C(CH_3)_2$ | O | O | Fp 108 °C |
| II-13 | $CH_3$ | $-CH_2-CH=CH_2$ | $=C(CH_3)_2$ | O | O | Fp 86 °C |
| II-14 | $CH_3$ | 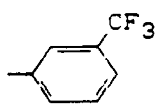 | $=C(CH_3)_2$ | O | O | Fp 158 °C |
| II-15 | $CH_3$ | 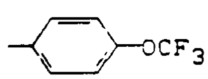 | $=C(CH_3)_2$ | O | O | Fp 218 °C |
| II-16 | $CH_3$ | 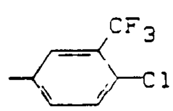 | $=C(CH_3)_2$ | O | O | Fp 185 °C |

Tabelle 2 - Fortsetzung

| Bsp. Nr. | $R^1$ | $R^2$ | 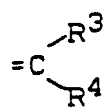 | X | Y | physikalische Eigenschaften |
|---|---|---|---|---|---|---|
| II-17 | $CH_3$ | 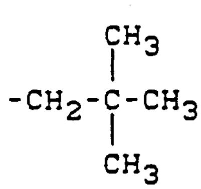 | $=C(CH_3)_2$ | O | O | Fp 121-122° C |

Beispiel III-1

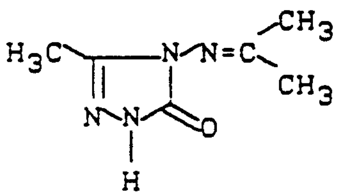

11,4 g (0,1 Mol) 4-Amino-3-methyl-1,2,4-(1H)-triazolin-5-on (vergl. Europ. J.Med. Chem.; Chim. Ther. 18, 215-220 [1983]) und 0,1 g p-Toluolsulfonsäure in 100 ml (79,06 g; 1,36 Mol) Aceton werden 40 Stunden bei 70 °C gerührt und anschließend im Vakuum eingeengt.
Man erhält 15,4 g (100 % der Theorie) an 4-Isopropylidenimino-3-methyl-1,2,4-(1H)-triazolin-5-on vom Schmelzpunkt 140-144 °C.

Beispiel V-1

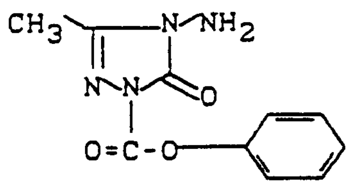

Zu 2,3 g (0,02 Mol) 4-Amino-3-methyl-1,2,4-(1H)-triazolin-5-on in 25 ml absolutem Tetrahydrofuran gibt man 2,7 g (0.024 Mol) Kalium-t-butylat, rührt 1 Stunde bei 20 °C, gibt dann tropfenweise unter Rühren 3,1 g (0.02 Mol) Chlorameisensäurephenylester zu, rührt 12 Stunden bei Raumtemperatur, stellt dann mit Eisessig auf pH 5 ein, engt im Vakuum ein, nimmt den Rückstand in Chloroform auf, wäscht mit Wasser, trocknet über Natriumsulfat, engt im Vakuum ein und kristallisiert den Rückstand durch Verreiben mit Ether.

Man erhält 1,1 g (23,5 % der Theorie) an 4-Amino-3-methyl-1-phenoxycarbonyl-1,2,4-triazolin-5-on vom Schmelzpunkt 175 °C.

Beispiel X-1

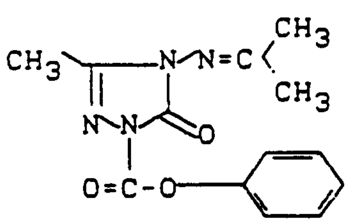

Zu 15,4 g (0.1 Mol) 4-Isopropylidenimino-3-methyl-1,2,4-(1H)-triazolin-5-on in 100 ml absolutem Tetrahydrofuran gibt man bei Raumtemperatur zunächst 13,4 g (0.12 Mol) Kalium-t-butylat, rührt eine Stunde bei Raumtemperatur, gibt dann 15,5 g (0.1 Mol) Chlorameisensäurephenylester zu und rührt weitere 12 Stunden bei 20 °C.
Zur Aufarbeitung wird mit Eisessig aufgesäuert, im Vakuum eingeengt, der Rückstand in Chloroform aufgenommen, mit Wasser gewaschen, über Natriumsulfat getrocknet, abermals im Vakuum eingeengt und aus Aceton umkristallisiert.

Man erhält 10 g (36,5 % der Theorie) an 4-Isopropylidenimino-3-methyl-1-phenoxycarbonyl-1,2,4-triazolin-5-on vom Schmelzpunkt 162 °C.

Beispiel X-2

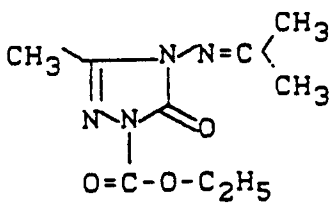

Zu 7,7 g (0.05 Mol) 4-Isopropylidenimino-3-methyl-1,2,4-(1H)-triazolin-5-on in 50 ml absolutem Tetrahydrofuran gibt man bei 20 °C 1,5 g (0.05 Mol) Natriumhydrid, rührt eine Stunde bei Raumtemperatur, gibt dann tropfenweise unter Rühren 5,4 g (0.05 Mol) Chlorameisensäureethylester zu und rührt nach beendeter Zugabe weitere 12 Stunden bei 20 °C.
Zur Aufarbeitung wird mit Eisessig angesäuert, im Vakuum eingeengt, der Rückstand in Dichlormethan aufgenommen, mit Wasser gewaschen, über Natriumsulfat getrocknet, abermals im Vakuum eingeengt und aus Isopropanol umkristallisiert.

Man erhält 5,0 g (44 % der Theorie) an 1-Ethoxycarbonyl-4-isopropylidenimino-3-methyl-1,2,4-triazolin-5-on vom Schmelzpunkt 91 °C.

Anwendungsbeispiele:

In den folgenden Anwendungsbeispielen wurden die nachstehend aufgeführten Verbindungen als Vergleichsubstanzen eingesetzt:

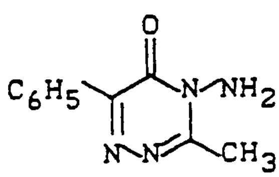
(A)

4-Amino-3-methyl-6-phenyl-1,2,4-triazin-5-on
(bekannt aus DE-OS 23 64 474, Beispiel I-22)

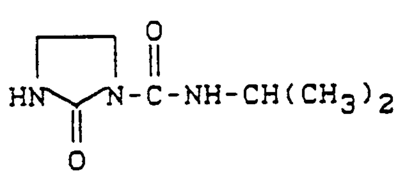
(B)

N-Isobutyl-2-oximidazolidine-1-carboxamid
(bekannt aus R. Wegler "Chemie der Pflanzenschutz- und Schädlingsbekämpfungsmittel" Bd. 5, Seite 219 (1977))

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßgen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die Verbindungen gemäß der Herstellungsbeispiele 2, 14, 23, 32, 41, 45, 48 und 57 eine deutlich bessere herbizide Wirkung gegen Unkräuter als auch eine deutliche bessere Selektivität in Nutzpflanzen wie beispielsweise Zuckerrüben als die Vergleichssubstanz (B).

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen z.B. die Verbindungen gemäß den Beispielen 1 und 48 sowie 1, 2, 3, 14, 23, 32, 41, 45, 48, 57 und 78 bei der Bekämpfung von mono- und dikotylen Unkräutern eine deutlich bessere herbizide Wirkung als die Vergleichssubstanz (A) bzw. (B).

## Patentansprüche

**Patentansprüche für folgende Vertragsstaaten : DE, AU, CH, LI, IT, FR, BE, NL, GB, SE, GR**

**1.** Substituierte Triazolinone der Formel (I),

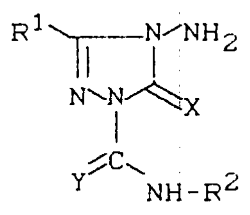

(I)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit 1 bis 6 Kohlenstoffatomen in den einzelnen Alkylteilen, für Cycloalkyl oder Cycloalkylalkyl mit jeweils 3 bis 7 Kohlenstoffatomen im Cycloalkylteil und gegebenenfalls 1 bis 6 Kohlenstoffatomen im geradkettigen oder verzweigten Alkylteil, für einfach oder mehrfach, gleich oder verschieden substituiertes Aryl mit 6 bis 10 Kohlenstoffatomen im Arylteil steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für Wasserstoff, für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteiten, für Cycloalkyl mit 11 Kohlenstoffatomen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im geradkettigen oder verzweigten Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder geradkettiges oder verzweigtes Halogenalkenyl mit bis zu 4 Kohlenstoffatomen und 1 bis 5 gleichen oder verschiedenen Halogenatomen oder jeweils zweifach verknüpftes Alkandiyl bzw. Alkendiyl mit jeweils bis zu 4 Kohlenstoffatomen; $R^2$ außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; $R^2$ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aroyl, Aryl, Aralkyloxy oder Aryloxy, mit jeweils 6 bis 10 Kohlenstoffatomen im Arylteil und gegebenenfalls 1 bis 8 Kohlenstoffatomen im Alkylteil steht, wobei als Alkylsubstituenten gegebenenfalls Halogon und Cyano infrage kommen und wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy,

X für Sauerstoff oder Schwefel steht und

Y für Sauerstoff oder Schwefel steht.

**2.** Substituierte Triazolinone der Formel (I) gemäß Anspruch 1, dadurch gekennzeichnet, daß

$R^1$ für Wasserstoff, Methyl, Ethyl, n- oder i-Propyl, n-, i-, s- oder t-Butyl, n- oder i-Pentyl, n- oder i-Hexyl, für Allyl, Propargyl, geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Methoxymethyl, Ethoxymethyl, Propoxymethyl, Cyclopentyl, Cyclohexyl, Cyclopropyl, Cyclopropylmethyl, Cyclohexylmethyl, Cyclohexylethyl, für für gegebenenfalls ein- bis dreifach gleich oder verschieden substituiertes Phenyl steht, wobei als Substituenten

infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^2$ Wasserstoff, Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl, Dodecyl, für Allyl, jeweils geradkettiges oder verzweigtes Butenyl, Pentenyl oder Hexenyl, Propargyl, jeweils geradkettiges oder verzweigtes Butinyl, Pentinyl oder Hexinyl, für geradkettiges oder verzweigtes Halogenalkyl mit 1 bis 8 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 8 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl steht, wobei als Substituenten jeweils infrage kommen: Fluor, Chlor, Brom, Methyl, Ethyl, n- oder i-Propyl, n-, i-, s- oder t-Butyl, Cyano, Methandiyl, Ethandiyl, Butandiyl oder Butadiendiyl oder Dichlorallyl;

$R^2$ weiterhin für im Heterocyclylteil gegebenenfalls ein- bis dreifach, gleich oder verschieden substituiertes Heterocyclylmethyl, Heterocyclylpropyl oder Heterocyclylethyl steht, wobei als Heterocyclen jeweils infrage kommen:

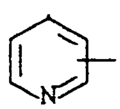 ; 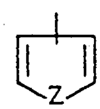 ; 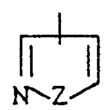 ; 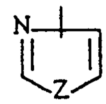 ;

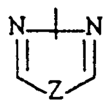 ; 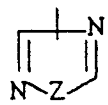 ; 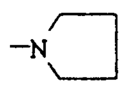 ; 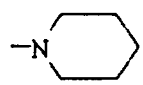 ;

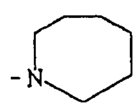 ; 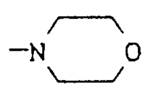 oder 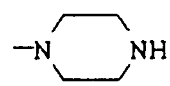

wobei Z jeweils für Sauerstoff oder Schwefel steht und wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio:

$R^2$ außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl steht, wobei als Phenylsubstituenten jeweils infrage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder

Phenoxy,
X für Sauerstoff oder Schwefel steht und
Y für Sauerstoff oder Schwefel steht.

3. Verfahren zur Herstellung von substituierten Triazolinonen der Formel (I),

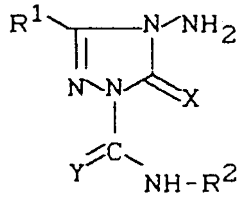

(I)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit 1 bis 6 Kohlenstoffatomen in den einzelnen Alkylteilen, für Cycloalkyl oder Cycloalkylalkyl mit jeweils 3 bis 7 Kohlenstoffatomen im Cycloalkylteil und gegebenenfalls 1 bis 6 Kohlenstoffatomen im geradkettigen oder verzweigten Alkylteil, für einfach oder mehrfach, gleich oder verschieden substituiertes Aryl mit 6 bis 10 Kohlenstoffatomen im Arylteil steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für Wasserstoff, für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für Cycloalkyl mit 11 Kohlenstoffatomen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im geradkettigen oder verzweigten Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder geradkettiges oder verzweigtes Halogenalkenyl mit bis zu 4 Kohlenstoffatomen und 1 bis 5 gleichen oder verschiedenen Halogenatomen oder jeweils zweifach verknüpftes Alkandiyl bzw. Alkendiyl mit jeweils bis zu 4 Kohlenstoffatomen; $R^2$ außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; $R^2$ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich

oder verschieden substituiertes Aralkyl, Aroyl, Aryl, Aralkyloxy oder Aryloxy, mit jeweils 6 bis 10 Kohlenstoffatomen im Arylteil und gegebenenfalls 1 bis 8 Kohlenstoffatomen im Alkylteil steht, wobei als Alkylsubstituenten gegebenenfalls Halogen und Cyano infrage kommen und wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy,

X für Sauerstoff oder Schwefel steht und

Y für Sauerstoff oder Schwefel steht,

dadurch gekennzeichnet, daß man

a) Hydrazone der Formel (II),

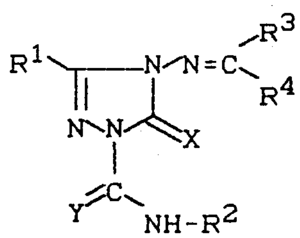

(II)

in welcher

$R^1$, $R^2$, X und Y die oben angegebene Bedeutung haben

und

$R^3$ und $R^4$ unabhängig voneinander jeweils für Wasserstoff, Alkyl, Aralkyl oder Aryl stehen,

mit einer Säure gegebenenfalls in Gegenwart eines Verdünnungsmittels umsetzt,

oder daß man

(b) 1H-Triazolinone der Formel (III),

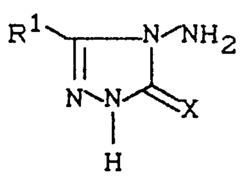

(III)

in welcher

$R^1$ und X die oben angegebene Bedeutung haben,

mit Iso(thio)cyanaten der Formel (IV),

$R^2\text{-}N=C=Y$ (IV)

in welcher

$R^2$ und Y die oben angegebene Bedeutung haben,

gegebenenfalls in Gegenwart eines Verdünnungsmittels und gegebenenfalls in Gegenwart eines Reaktionshilfsmittels umsetzt,

oder daß man

(c) Triazolinone der Formel (V),

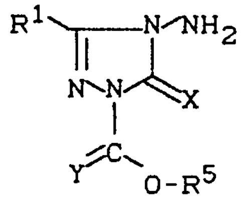

(V)

in welcher
$R^1$, X und Y die oben angegebene Bedeutung haben,
und
$R^5$ für Alkyl, Aryl oder Arylalkyl steht,
mit Aminen der Formel (VI),

$R^2$-$NH_2$ (VI)

in welcher
$R^2$ die oben angegebene Bedeutung hat,
gegebenenfalls in Gegenwart eines Verdünnungsmittels und gegebenenfalls in Gegenwart eines Reaktionshilfsmittels umsetzt.

**4.** Herbizide Mittel, gekennzeichnet durch einen Gehalt an mindestens einem substituierten Triazolinon der Formel (I) gemäß den Ansprüchen 1 und 2.

**5.** Verfahren zur Bekämpfung von Unkräutern, dadurch gekennzeichnet, daß man substituierte Triazolinone der Formel (I) gemäß den Ansprüchen 1 und 2 auf die Unkräuter und/oder ihren Lebensraum einwirken läßt.

**6.** Verwendung von substituierten Triazolinonen der Formel (I) gemäß den Ansprüchen 1 und 2 zur Bekämpfung von Unkräutern.

**7.** Verfahren zur Herstellung von herbiziden Mitteln, dadurch gekennzeichnet, daß man substituierte Triazolinone der Formel (I) gemäß Ansprüchen 1 und 2 mit Streckmitteln und/oder oberflächenaktiven Substanzen vermischt.

**8.** Hydrazone der Formel (II),

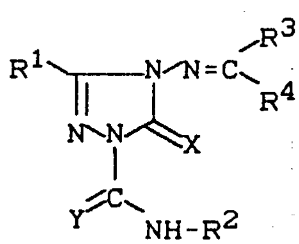

(II)

in welcher
$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit 1 bis 6 Kohlenstoffatomen in den einzelnen Alkylteilen, für Cycloalkyl oder Cycloalkylalkyl mit jeweils 3 bis 7 Kohlenstoffatomen im Cycloalkylteil und gegebenenfalls 1 bis 6 Kohlenstoffatomen im geradkettigen oder verzweigten Alkylteil, für einfach oder mehrfach, gleich oder verschieden substituiertes Aryl mit 6 bis 10 Kohlenstoffatomen im Arylteil

steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für Wasserstoff, für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für Cycloalkyl mit 11 Kohlenstoffatomen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im geradkettigen oder verzweigten Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder geradkettiges oder verzweigtes Halogenalkenyl mit bis zu 4 Kohlenstoffatomen und 1 bis 5 gleichen oder verschiedenen Halogenatomen oder jeweils zweifach verknüpftes Alkandiyl bzw. Alkendiyl mit jeweils bis zu 4 Kohlenstoffatomen; $R^2$ außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; $R^2$ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aroyl, Aryl, Aralkyloxy oder Aryloxy, mit jeweils 6 bis 10 Kohlenstoffatomen im Arylteil und gegebenenfalls 1 bis 8 Kohlenstoffatomen im Alkylteil steht, wobei als Alkylsubstituenten gegebenenfalls Halogen und Cyano infrage kommen und wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy,

X für Sauerstoff oder Schwefel steht,

Y für Sauerstoff oder Schwefel steht,

$R^3$ und $R^4$ stehen jeweils unabhängig voneinander für Wasserstoff, für geradkettiges oder verzweigtes Alkyl mit 1 bis 4 Kohlenstoffatomen oder für Phenyl oder Benzyl,

ausgenommen die Verbindung
1-(N-4-Bromphenylthiocarbamoyl)-4-phenylidenamino-1,2,4-triazolin-5-thion.

**9.** Triazolinone der Formel (Va),

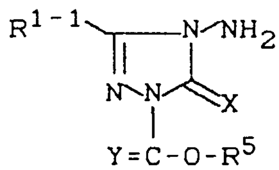

(Va)

$R^{1-1}$ steht für geradkettiges oder verzweigtes Alkyl mit 1 bis 4 Kohlenstoffatomen,
X und Y stehen unabhängig voneinander für Sauerstoff oder Schwefel,
$R^5$ steht für geradkettiges oder verzweigtes Alkyl mit 1 bis 4 Kohlenstoffatomen oder für gegebenenfalls ein- bis dreifach, gleich oder verschieden substituiertes Phenyl oder Benzyl, wobei als Substituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen.

**10.** Triazolinone der Formel (Xa)

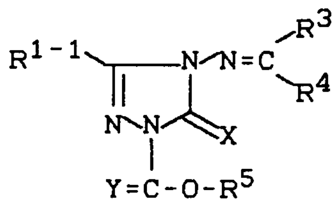

(Xa)

in welcher
$R^{1-1}$ steht für geradkettiges oder verzweigtes Alkyl mit 1 bis 4 Kohlenstoffatomen,
$R^3$ und $R^4$ stehen jeweils unabhängig voneinander für Wasserstoff, für geradkettiges oder verzweigtes Alkyl mit 1 bis 4 Kohlenstoffatomen oder für Phenyl oder Benzyl,
$R^5$ steht vorzugsweise für geradkettiges oder verzweigtes Alkyl mit 1 bis 4 Kohlenstoffatomen oder für gegebenenfalls ein- bis dreifach, gleich oder verschieden substituiertes Phenyl oder Benzyl, wobei als Substituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,
X und Y stehen unabhängig voneinander für Sauerstoff oder Schwefel.

**Patentansprüche für folgenden Vertragsstaat : ES**

**1.** Verfahren zur Herstellung von substituierten Triazolinonen der Formel (I),

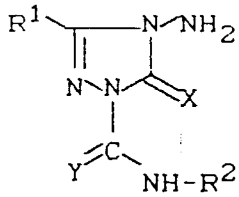

(I)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit 1 bis 6 Kohlenstoffatomen in den einzelnen Alkylteilen, für Cycloalkyl oder Cycloalkylalkyl mit jeweils 3 bis 7 Kohlenstoffatomen im Cycloalkylteil und gegebenenfalls 1 bis 6 Kohlenstoffatomen im geradkettigen oder verzweigten Alkylteil, für
einfach oder mehrfach, gleich oder verschieden substituiertes Aryl mit 6 bis 10 Kohlenstoffatomen im Arylteil
steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für Wasserstoff, für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für Cycloalkyl mit 11 Kohlenstoffatomen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im geradkettigen oder verzweigten Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder geradkettiges oder verzweigtes Halogenalkenyl mit bis zu 4 Kohlenstoffatomen und 1 bis 5 gleichen oder verschiedenen Halogenatomen oder jeweils zweifach verknüpftes Alkandiyl bzw. Alkendiyl mit jeweils bis zu 4 Kohlenstoffatomen; $R^2$ außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; $R^2$ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aroyl, Aryl, Aralkyloxy oder Aryloxy, mit jeweils 6 bis 10 Kohlenstoffatomen im Arylteil und gegebenenfalls 1 bis 8 Kohlenstoffatomen im Alkylteil steht, wobei als Alkylsubstituenten gegebenenfalls Halogen und Cyano infrage kommen und wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy,

X für Sauerstoff oder Schwefel steht und

Y für Sauerstoff oder Schwefel steht,

dadurch gekennzeichnet, daß man

a) Hydrazone der Formel (II),

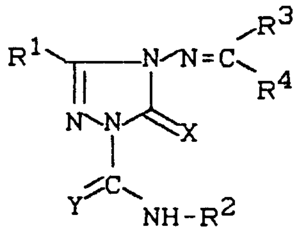

(II)

in welcher
$R^1$, $R^2$, X und Y die oben angegebene Bedeutung haben
und
$R^3$ und $R^4$ unabhängig voneinander jeweils für Wasserstoff, Alkyl, Aralkyl oder Aryl stehen,
mit einer Säure gegebenenfalls in Gegenwart eines Verdünnungsmittels umsetzt,
oder daß man
(b) 1H-Triazolinone der Formel (III),

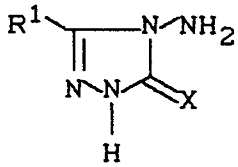

(III)

in welcher
$R^1$ und X die oben angegebene Bedeutung haben,
mit Iso(thio)cyanaten der Formel (IV),

$R^2$-N = C = Y (IV)

in welcher
$R^2$ und Y die oben angegebene Bedeutung haben,
gegebenenfalls in Gegenwart eines Verdünnungsmittels und gegebenenfalls in Gegenwart eines Reaktionshilfsmittels umsetzt,
oder daß man
(c) Triazolinone der Formel (V),

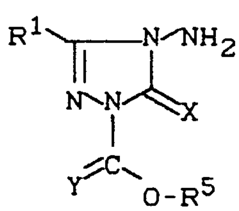

(V)

in welcher
$R^1$, X und Y die oben angegebene Bedeutung haben,
und
$R^5$ für Alkyl, Aryl oder Arylalkyl steht,
mit Aminen der Formel (VI),

$R^2$-$NH_2$ (VI)

in welcher
$R^2$ die oben angegebene Bedeutung hat,
gegebenenfalls in Gegenwart eines Verdünnungsmittels und gegebenenfalls in Gegenwart eines Reaktionshilfsmittels umsetzt`

**2.** Verfahren zur Herstellung von substituierten Triazolinonen der Formel (I) gemäß Anspruch 1, dadurch gekennzeichnet, daß

$R^1$ für Wasserstoff, Methyl, Ethyl, n- oder i-Propyl, n-, i-, s- oder t-Butyl, n- oder i-Pentyl, n- oder i-Hexyl, für Allyl, Propargyl, geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Methoxymethyl, Ethoxymethyl, Propoxymethyl, Cyclopentyl, Cyclohexyl, Cyclopropyl, Cyclopropylmethyl, Cyclohexylmethyl, Cyclohexylethyl, für für gegebenenfalls ein- bis dreifach gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^2$ Wasserstoff, Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl, Dodecyl, für Allyl, jeweils geradkettiges oder verzweigtes Butenyl, Pentenyl oder Hexenyl, Propargyl, jeweils geradkettiges oder verzweigtes Butinyl, Pentinyl oder Hexinyl, für geradkettiges oder verzweigtes Halogenalkyl mit 1 bis 8 Kohlenstoffatomen und 1 bis 9 gleichen oder verschiedenen Halogenatomen, insbesondere Fluor, Chlor oder Brom, für jeweils geradkettiges oder verzweigtes Halogenalyenyl oder Halogenalkinyl mit jeweils 3 bis 8 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl steht, wobei als Substituenten jeweils infrage kommen: Fluor, Chlor, Brom: Methyl, Ethyl, n- oder i-Propyl, n-, i-, s- oder t-Butyl, Cyano, Methandiyl, Ethandiyl, Butandiyl oder Butadiendiyl oder Dichlorallyl;

$R^2$ weiterhin für im Heterocyclylteil gegebenenfalls ein- bis dreifach, gleich oder verschieden substituiertes Heterocyclylmethyl, Heterocyclylpropyl oder Heterocyclylethyl steht, wobei als Heterocyclen jeweils infrage kommen:

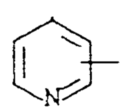 ; 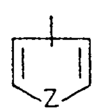 ; 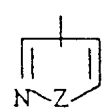 ; 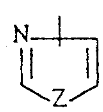 ;

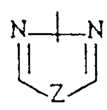 ; 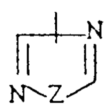 ; 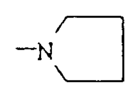 ; 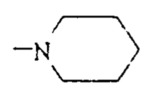 ;

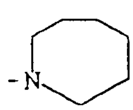 ; 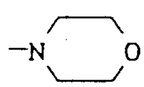 oder 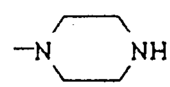

wobei Z jeweils für Sauerstoff oder Schwefel steht und wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;

$R^2$ außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich-oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl steht, wobei als Phenylsubstituenten jeweils infrage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy,

X für Sauerstoff oder Schwefel steht und

Y für Sauerstoff oder Schwefel steht.

3. Herbizide Mittel, gekennzeichnet durch einen Gehalt an mindestens einem substituierten Triazolinon der Formel (I) gemäß den Ansprüchen 1 und 2.

4. Verfahren zur Bekämpfung von Unkräutern, dadurch gekennzeichnet, daß man substituierte Triazolinone der Formel (I) gemäß den Ansprüchen 1 und 2 auf die Unkräuter und/oder ihren Lebensraum einwirken läßt.

5. Verwendung von substituierten Triazolinonen der Formel (I) gemäß den Ansprüchen 1 und 2 zur Bekämpfung von Unkräutern.

6. Verfahren zur Herstellung von herbiziden Mitteln, dadurch gekennzeichnet, daß man substituierte Triazolinone der Formel (I) gemäß Ansprüchen 1 und 2 mit Streckmitteln und/oder oberflächenaktiven Substanzen vermischt.

7. Verfahren zur Herstellung von Hydrazonen der Formel (II),

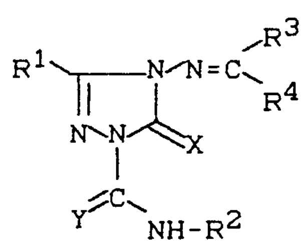

(II)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit 1 bis 6 Kohlenstoffatomen in den einzelnen Alkylteilen, für Cycloalkyl oder Cycloalkylalkyl mit jeweils 3 bis 7 Kohlenstoffatomen im Cycloalkylteil und gegebenenfalls 1 bis 6 Kohlenstoffatomen im geradkettigen oder verzweigten Alkylteil, für einfach oder mehrfach, gleich oder verschieden substituiertes Aryl mit 6 bis 10 Kohlenstoffatomen im Arylteil steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis

4 Kohlenstoffatomen und gegebenenfalls 1 bis 9 gleichen oder verschiedenen Halogenatomen,

$R^2$ für Wasserstoff, für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für Cycloalkyl mit 11 Kohlenstoffatomen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im geradkettigen oder verzweigten Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder geradkettiges oder verzweigtes Halogenalkenyl mit bis zu 4 Kohlenstoffatomen und 1 bis 5 gleichen oder verschiedenen Halogenatomen oder jeweils zweifach verknüpftes Alkandiyl bzw. Alkendiyl mit jeweils bis zu 4 Kohlenstoffatomen; $R^2$ außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; $R^2$ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aroyl, Aryl, Aralkyloxy oder Aryloxy, mit jeweils 6 bis 10 Kohlenstoffatomen im Arylteil und gegebenenfalls 1 bis 8 Kohlenstoffatomen im Alkylteil steht, wobei als Alkylsubstituenten gegebenenfalls Halogen und Cyano infrage kommen und wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy,

X für Sauerstoff oder Schwefel steht,

Y für Sauerstoff oder Schwefel steht,

$R^3$ und $R^4$ stehen jeweils unabhängig voneinander für Wasserstoff, für geradkettiges oder verzweigtes Alkyl mit 1 bis 4 Kohlenstoffatomen oder für Phenyl oder Benzyl,

ausgenommen die Verbindung
1-(N-4-Bromphenylthiocarbamoyl)-4-phenylidenamino-1,2,4-triazolin-5-thion.
dadurch gekennzeichnet, daß man 1-unsubstituierte 4-Amino-triazolinone der Formel (III)

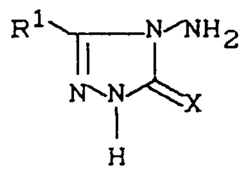

(III)

in welcher
$R^1$ und X die oben angegebene Bedeutung haben,
mit Aldehyden oder Ketonen der Formel (VII),

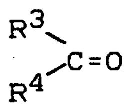

(VII)

in welcher
$R^3$ und $R^4$ die oben angegebene Bedeutung haben,
gegebenenfalls in Gegenwart eines Verdünnungsmittels wie beispielsweise Dichlormethan oder Toluol und gegebenenfalls in einer anschließenden 3. Stufe mit Aminen der Formel (VI),

$R^2$-$NH_2$ (VI)

in welcher
$R^2$ die oben angegebene Bedeutung hat,
gegebenenfalls in Gegenwart eines Verdünnungsmittels wie beispielsweise Tetrahydrofuran sowie gegebenenfalls in Gegenwart einer Base wie beispielsweise Natriumhydroxid oder Kaliumhydroxid bei Temperaturen zwischen 20 °C und 50 °C umsetzt.

**8.** Verfahren zur Herstellung von Triazolinonen der Formel (Va),

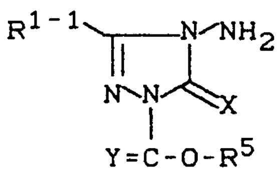

(Va)

$R^{1-1}$ für geradkettiges oder verzweigtes Alkyl mit 1 bis 4 Kohlenstoffatomen steht und
$R^5$ für geradkettiges oder verzweigtes Alkyl mit 1 bis 4 Kohlenstoffatomen oder für gegebenenfalls ein- bis dreifach, gleich oder verschieden substituiertes Phenyl oder Benzyl, wobei als Substituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen steht,
X und Y unabhängig voneinander für Sauerstoff oder Schwefel stehen,
dadurch gekennzeichnet, daß man 1H-Triazolinone der Formel (IIIa)

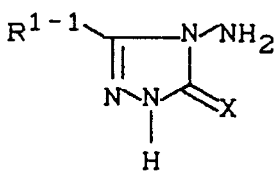

(IIIa)

in welcher
$R^{1-1}$ und X die oben angegebene Bedeutung haben,
mit (Thio)Chlorameisensäureestern der Formel (IX),

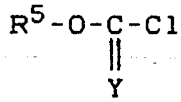

(IX)

in welcher

$R^5$ und Y die oben angegebene Bedeutung haben,

gegebenenfalls in Gegenwart eines Verdünnungsmittels wie beispielsweise Tetrahydrofuran und gegebenenfalls in Gegenwart eines Reaktionshilfsmittels wie beispielsweise Kalium-t-butylat oder Natriumhydrid bei Temperaturen zwischen -20 °C und +40 °C umsetzt.

**9.** Verfahren zur Herstellung von Triazolinonen der Formel (Xa)

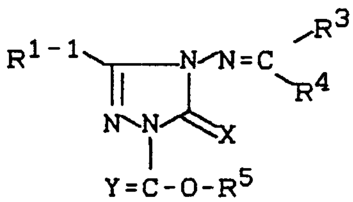

(Xa)

in welcher

$R^{1-1}$ für geradkettiges oder verzweigtes Alkyl mit 1 bis 4 Kohlenstoffatomen steht,

$R^3$ und $R^4$ jeweils unabhängig voneinander für Wasserstoff, für geradkettiges oder verzweigtes Alkyl mit 1 bis 4 Kohlenstoffatomen oder für Phenyl oder Benzyl stehen,

$R^5$ steht für geradkettiges oder verzweigtes Alkyl mit 1 bis 4 Kohlenstoffatomen oder für gegebenenfalls ein- bis dreifach, gleich oder verschieden substituiertes Phenyl oder Benzyl steht, wobei als Substituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

X und Y unabhängig voneinander jeweils für Sauerstoff oder Schwefel stehen,

dadurch gekennzeichnet, daß man 1-unsubstituierte 4-Amino-triazolinone der Formel (III)

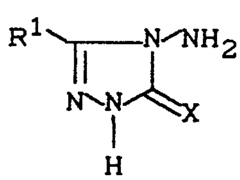

(III)

in welcher

$R^1$ und X die in der Beschreibung genannten Bedeutungen haben mit Aldehyden oder Ketonen der Formel (VII)

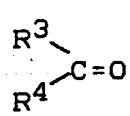

(VII)

in welcher

$R^3$ und $R^4$ die in der Beschreibung genannten Bedeutungen haben

gegebenenfalls in Gegenwart eines Verdünnungsmittels wie beispielsweise Dichlormethan oder Toluol

und gegebenenfalls in Gegenwart eines Katalysators wie beispielsweise p-Toluolsulfonsäure mit Temperaturen zwischen 40 °C und 120 °C umsetzt und die so erhältlichen 1-unsubstituierten Triazolinon-Hydrazone der Formel (VIII)

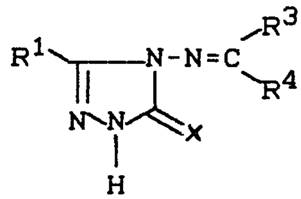

(VIII)

in welcher

$R^1$, $R^3$, $R^4$ und x die in der Beschreibung angegebenen Bedeutungen haben,

in einer anschließenden 2. Stufe mit (Thio)chlorameisensäureestern der Formel (IX)

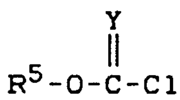

(IX)

in welcher

$R^5$ und Y die in der Beschreibung angegebenen Bedeutungen haben,

gegebenenfalls in Gegenwart eines Verdünnungsmittels wie beispielsweise Tetrahydrofuran und gegebenenfalls in Gegenwart eines Reaktionshilfsmittels wie beispielsweise Natriumhydrid oder Kalium-t-butylat bei Temperaturen zwischen -20 °C und +40 °C umsetzt.

## Claims

### Claims for the following Contracting States : DE, AU, CH, LI, IT, FR, BE, NL, GB, SE, GR

1. Substituted triazolinones of the formula (I)

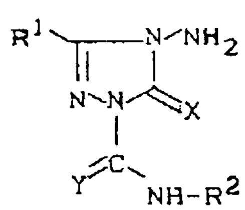

(I)

in which

$R^1$ represents hydrogen, in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl having 1 to 6 carbon atoms in the individual alkyl moieties, cycloalkyl or cycloalkylalkyl in each case having 3 to 7 carbon atoms in the cycloalkyl moiety and optionally 1 to 6 carbon atoms in the straight-chain or branched alkyl moiety, aryl having 6 to 10 carbon atoms in the aryl moiety, which is monosubstituted or polysubstituted by identical or different substituents, suitable aryl substituents in each case being: halogen, cyano, nitro and in each case straight-chain or branched alkyl, alkoxy, alkylthio, halogenoalkyl, halogenoalkoxy or halogenoalkylthio in each case having 1 to 4 carbon atoms and optionally 1 to 9 identical or different halogen atoms,

$R^2$ represents hydrogen, in each case straight-chain or branched alkyl having 1 to 18 carbon atoms, alkenyl having 2 to 8 carbon atoms, alkinyl having 2 to 8 carbon atoms, halogenoalkyl

having 1 to 8 carbon atoms and 1 to 17 identical or different halogen atoms, halogenoalkenyl or halogenoalkinyl in each case having 2 to 8 carbon atoms and 1 to 15 or 13 identical or different halogen atoms, cyanoalkyl having 1 to 8 carbon atoms, hydroxyalkyl having 1 to 8 carbon atoms and 1 to 6 hydroxyl groups, alkoxyalkyl, alkoxycarbonylalkyl or alkoxycarbonylalkenyl in each case having up to 6 carbon atoms in the individual alkyl or alkenyl moieties, alkylaminoalkyl or dialkylaminoalkyl in each case having 1 to 6 carbon atoms in the individual alkyl moieties, cycloalkyl having 11 carbon atoms or cycloalkyl, cycloalkylalkyl, cycloalkenyl or cycloalkenylalkyl in each case having 3 to 8 carbon atoms in the cycloalkyl or cycloalkenyl moiety and optionally 1 to 6 carbon atoms in the straight-chain or branched alkyl moiety, in each case optionally monosubstituted or polysubstituted by identical or different substituents, suitable substituents in each case being: halogen, cyano and in each case straight-chain or branched alkyl or halogenoalkyl in each case having 1 to 4 carbon atoms and optionally 1 to 9 identical or different halogen atoms or straight-chain or branched halogenoalkenyl having up to 4 carbon atoms and 1 to 5 identical or different halogen atoms or in each case doubly linked alkanediyl or alkenediyl in each case having up to 4 carbon atoms; $R^2$ additionally represents heterocyclylalkyl having 1 to 6 carbon atoms in the straight-chain or branched alkyl moiety and 1 to 9 carbon atoms and also 1 to 3 hetero atoms - in particular nitrogen, oxygen and/or sulphur - in the heterocyclyl moiety and which is optionally monosubstituted or polysubstituted in the heterocyclyl moiety by identical or different substituents, suitable substituents being: halogen, cyano, nitro, and in each case straight-chain or branched alkyl, alkoxy, alkylthio, halogenoalkyl, halogenoalkoxy, halogenoalkylthio or alkoxycarbonyl in each case having 1 to 5 carbon atoms and optionally 1 to 9 identical or different halogen atoms; $R^2$ additionally represents in each case straight-chain or branched alkoxy having 1 to 8 carbon atoms, alkenyloxy having 2 to 8 carbon atoms or alkinyloxy having 2 to 8 carbon atoms and finally aralkyl, aroyl, aryl, aralkyloxy or aryloxy, in each case having 6 to 10 carbon atoms in the aryl moiety and optionally 1 to 8 carbon atoms in the alkyl moiety, and in each case optionally monosubstituted or polysubstituted by identical or different substituents, suitable alkyl substituents optionally being halogen and cyano and suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl in each case having 1 to 6 carbon atoms in the alkyl moiety and optionally 1 to 9 identical or different halogen atoms, cycloalkyl having 3 to 6 carbon atoms and phenoxy,

X represents oxygen or sulphur and

Y represents oxygen or sulphur.

**2.** Substituted triazolinones of the formula (I) according to Claim 1, characterized in that

$R^1$ represents hydrogen, methyl, ethyl, n- or i-propyl, n-, i-, s- or t-butyl, n- or i-pentyl, n- or i-hexyl, allyl, propargyl, straight-chain or branched halogenoalkyl having 1 to 4 carbon atoms and 1 to 9 identical or different halogen atoms, in particular fluorine, chlorine or bromine, methoxymethyl, ethoxymethyl, propoxymethyl, cyclopentyl, cyclohexyl, cyclopropyl, cyclopropylmethyl, cyclohexylmethyl, cyclohexylethyl,

phenyl which is optionally monosubstituted to trisubstituted by identical or different substituents, suitable substituents being:

fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio,

$R^2$ represents hydrogen, methyl, ethyl, n- or i-propyl, n-, i-, s- or t-butyl, in each case straight-chain or branched pentyl, hexyl, heptyl, octyl, nonyl, decyl, dodecyl, allyl, in each case straight-chain or branched butenyl, pentenyl or hexenyl, propargyl, in each case straight-chain or branched butinyl, pentinyl or hexinyl, straight-chain or branched halogenoalkyl having 1 to 8 carbon atoms and 1 to 9 identical or different halogen atoms, in particular fluorine, chlorine or bromine, in each case straight-chain or branched halogenoalkenyl or halogenoakynyl in each case having 3 to 8 carbon atoms and 1 to 3 halogen atoms, in particular fluorine or chlorine, in each case straight-chain or branched cyanoalkyl having 1 to 6 carbon atoms in the alkyl moiety, hydroxyalkyl having 1 to 6 carbon atoms and 1 to 3 hydroxyl groups, alkoxyalkyl, alkoxycarbonylalkyl or alkoxycarbonylalkenyl, alkylaminoalkyl or dialkylaminoalkyl in each case having up to 4 carbon atoms in the individual alkyl or alkenyl moieties or cyclopropyl, cyclopropylmethyl, cyclopropylethyl, cyclopentyl, cyclohexyl, cycloheptyl,

cyclooctyl, cyclohexylmethyl, cyclohexylethyl, cyclohexenyl or cyclohexenylmethyl, in each case optionally monosubstituted to trisubstituted by identical or different substituents, suitable substituents in each case being: fluorine, chlorine, bromine, methyl, ethyl, n- or i-propyl, n-, i-, s- or t-butyl, cyano, methanediyl, ethanediyl, butanediyl or butadienediyl or dichloroallyl;

$R^2$ furthermore represents heterocyclylmethyl, heterocyclylpropyl or heterocyclylethyl which are optionally monosubstituted to trisubstituted in the heterocyclyl moiety by identical or different substituents, suitable heterocycles in each case being:

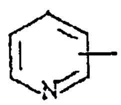 ; 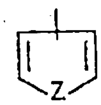 ; 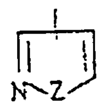 ; 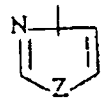 ;

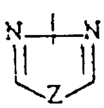 ; 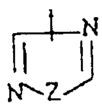 ; 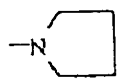 ; 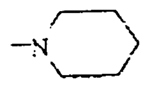 ;

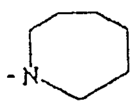 ; 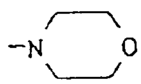 or 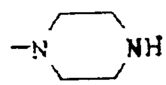 ,

Z in each case representing oxygen or sulphur and suitable substituents in each case being: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio;

$R^2$ additionally represents in each case straight-chain or branched alkoxy having 1 to 6 carbon atoms, alkenyloxy having 3 to 6 carbon atoms or alkinyloxy having 3 to 6 carbon atoms or optionally straight-chain or branched benzyl, phenylethyl, phenylpropyl, phenylbutyl, phenylpentyl, phenylhexyl, phenylheptyl, phenylcyanomethyl, phenylcyanoethyl, phenylcyanopropyl, benzyloxy, phenylethoxy, phenoxy, benzoyl, phenyl or naphthyl, in each case optionally monosubstituted to trisubstituted by identical or different substituents, suitable phenyl substituents in each case being: fluorine, chlorine, bromine, hydroxyl, cyano, nitro, methyl, ethyl, n- or i-propyl, n-, i-, s- or t-butyl, methoxy, ethoxy, methylthio, trifluoromethyl, trifluoromethoxy, trifluoromethylthio, trifluoromethylsulphinyl, trifluoromethylsulphonyl, methylsulphinyl, methylsulphonyl, acetyl, propionyl, methoxycarbonyl, ethoxycarbonyl, cyclohexyl or phenoxy,

X represents oxygen or sulphur and

Y represents oxygen or sulphur.

**3.** Process for the preparation of substituted triazolinones of the formula (I)

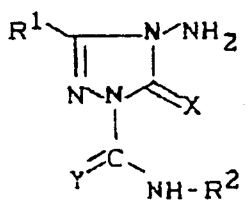

(I)

in which

$R^1$ represents hydrogen, in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl

having 1 to 6 carbon atoms in the individual alkyl moieties, cycloalkyl or cycloalkylalkyl in each case having 3 to 7 carbon atoms in the cycloalkyl moiety and optionally 1 to 6 carbon atoms in the straight-chain or branched alkyl moiety, aryl having 6 to 10 carbon atoms in the aryl moiety, which is monosubstituted or polysubstituted by identical or different substituents, suitable aryl substituents in each case being: halogen, cyano, nitro and in each case straight-chain or branched alkyl, alkoxy, alkylthio, halogenoalkyl, halogenoalkoxy or halogenoalkylthio in each case having 1 to 4 carbon atoms and optionally 1 to 9 identical or different halogen atoms,

$R^2$ represents hydrogen, in each case straight-chain or branched alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl in each case having 2 to 8 carbon atoms and 1 to 15 or 13 identical or different halogen atoms, cyanoalkyl having 1 to 8 carbon atoms, hydroxyalkyl having 1 to 8 carbon atoms and 1 to 6 hydroxyl groups, alkoxyalkyl, alkoxycarbonylalkyl or alkoxycarbonylalkenyl in each case having up to 6 carbon atoms in the individual alkyl or alkenyl moieties, alkylaminoalkyl or dialkylaminoalkyl in each case having 1 to 6 carbon atoms in the individual alkyl moieties, cycloalkyl having 11 carbon atoms or cycloalkyl, cycloalkylalkyl, cycloalkenyl or cycloalkenylalkyl in each case having 3 to 8 carbon atoms in the cycloalkyl or cycloalkenyl moiety and optionally 1 to 6 carbon atoms in the straight-chain or branched alkyl moiety, in each case optionally monosubstituted or polysubstituted by identical or different substituents, suitable substituents in each case being: halogen, cyano and in each case straight-chain or branched alkyl or halogenoalkyl in each case having 1 to 4 carbon atoms and optionally 1 to 9 identical or different halogen atoms or straight-chain or branched halogenoalkenyl having up to 4 carbon atoms and 1 to 5 identical or different halogen atoms or in each case doubly linked alkanediyl or alkenediyl in each case having up to 4 carbon atoms; $R^2$ additionally represents heterocyclylalkyl having 1 to 6 carbon atoms in the straight-chain or branched alkyl moiety and 1 to 9 carbon atoms and also 1 to 3 hetero atoms - in particular nitrogen, oxygen and/or sulphur - in the heterocyclyl moiety and which is optionally monosubstituted or polysubstituted in the heterocyclyl moiety by identical or different substituents, suitable substituents being: halogen, cyano, nitro, and in each case straight-chain or branched alkyl, alkoxy, alkylthio, halogenoalkyl, halogenoalkoxy, halogenoalkylthio or alkoxycarbonyl in each case having 1 to 5 carbon atoms and optionally 1 to 9 identical or different halogen atoms; $R^2$ additionally represents in each case straight-chain or branched alkoxy having 1 to 8 carbon atoms, alkenyloxy having 2 to 8 carbon atoms or alkinyloxy having 2 to 8 carbon atoms and finally aralkyl, aroyl, aryl, aralkyloxy or aryloxy, in each case having 6 to 10 carbon atoms in the aryl moiety and optionally 1 to 8 carbon atoms in the alkyl moiety, and in each case optionally monosubstituted or polysubstituted by identical or different substituents, suitable alkyl substituents optionally being halogen and cyano and suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl, halogenoalkyl sulphinyl, halogenoalkylsulphonyl, alkanoyl or alkoxycarbonyl in each case having 1 to 6 carbon atoms in the alkyl moiety and optionally 1 to 9 identical or different halogen atoms, cycloalkyl having 3 to 6 carbon atoms and phenoxy,

X represents oxygen or sulphur and

Y represents oxygen or sulphur,

characterized in that

a) hydrazones of the formula (II)

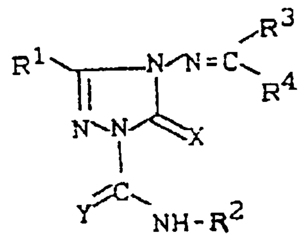

(II)

in which
$R^1$, $R^2$, X and Y have the abovementioned meaning and
$R^3$ and $R^4$ independently of one another in each case represent hydrogen, alkyl, aralkyl or aryl, are reacted with an acid, if appropriate in the presence of a diluent,
or in that
(b) 1H-triazolinones of the formula (III)

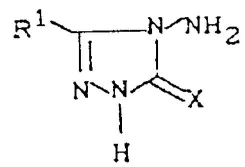

(III)

in which
$R^1$ and X have the abovementioned meaning,
are reacted with iso(thio)cyanates of the formula (IV)

$R^2$-N=C=Y (IV)

in which
$R^2$ and Y have the abovementioned meaning,
if appropriate in the presence of a diluent and if appropriate in the presence of a reaction auxiliary,
or in that
(c) triazolinones of the formula (V)

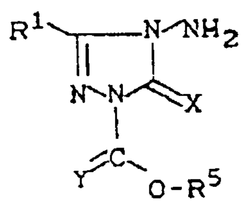

(V)

in which
$R^1$, X and Y have the abovementioned meaning,
and
$R^5$ represents alkyl, aryl or arylalkyl,
are reacted with amines of the formula (VI)

$R^2$-$NH_2$ (VI)

in which
$R^2$ has the abovementioned meaning,
if appropriate in the presence of a diluent and if appropriate in the presence of a reaction auxiliary.

**4.** Herbicidal agents, characterized in that they contain at least one substituted triazolinone of the formula (I) according to Claims 1 and 2.

**5.** Process for the control of weeds, characterized in that substituted triazolinones of the formula (I) according to Claims 1 and 2 are allowed to act on the weeds and/or their environment.

**6.** Use of substituted triazolinones of the formula (I) according to Claims 1 and 2 for the control of weeds.

**7.** Process for the production of herbicidal agents, characterized in that substituted triazolinones of the formula (I) according to Claims 1 and 2 are mixed with extenders and/or surface-active substances.

**8.** Hydrazones of the formula (II)

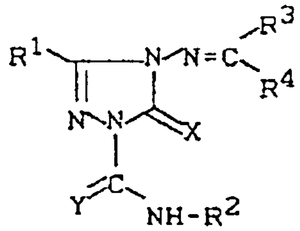

(II)

in which

$R^1$ represents hydrogen, in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl having 1 to 6 carbon atoms in the individual alkyl moieties, cycloalkyl or cycloalkylalkyl in each case having 3 to 7 carbon atoms in the cycloalkyl moiety and optionally 1 to 6 carbon atoms in the straight-chain or branched alkyl moiety, aryl having 6 to 10 carbon atoms in the aryl moiety, which is monosubstituted or polysubstituted by identical or different substituents,
suitable aryl substituents in each case being: halogen, cyano, nitro and in each case straight-chain or branched alkyl, alkoxy, alkylthio, halogenoalkyl, halogenoalkoxy or halogenoalkylthio in each case having 1 to 4 carbon atoms and optionally 1 to 9 identical or different halogen atoms,

$R^2$ represents hydrogen, in each case straight-chain or branched alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl in each case having 2 to 8 carbon atoms and 1 to 15 or 13 identical or different halogen atoms, cyanoalkyl having 1 to 8 carbon atoms, hydroxyalkyl having 1 to 8 carbon atoms and 1 to 6 hydroxyl groups, alkoxyalkyl, alkoxycarbonylalkyl or alkoxycarbonylalkenyl in each case having up to 6 carbon atoms in the individual alkyl or alkenyl moieties, alkylaminoalkyl or dialkylaminoalkyl in each case having 1 to 6 carbon atoms in the individual alkyl moieties, cycloalkyl having 11 carbon atoms or cycloalkyl, cycloalkylalkyl, cycloalkenyl or cycloalkenylalkyl in each case having 3 to 8 carbon atoms in the cycloalkyl or cycloalkenyl moiety and optionally 1 to 6 carbon atoms in the straight-chain or branched alkyl moiety, in each case optionally monosubstituted or polysubstituted by identical or different substituents, suitable substituents in each case being: halogen, cyano and in each case straight-chain or branched alkyl or halogenoalkyl in each case having 1 to 4 carbon atoms and optionally 1 to 9 identical or different halogen atoms or straight-chain or branched halogenoalkenyl having up to 4 carbon atoms and 1 to 5 identical or different halogen atoms or in each case doubly linked alkanediyl or alkenediyl in each case having up to 4 carbon atoms; $R^2$ additionally represents heterocyclylalkyl having 1 to 6 carbon atoms in the straight-chain or branched alkyl moiety and 1 to 9 carbon atoms and also 1 to 3 hetero atoms - in particular nitrogen, oxygen and/or sulphur - in the heterocyclyl moiety and which is optionally monosubstituted or polysubstituted in the heterocyclyl moiety by identical or different substituents, suitable substituents being: halogen, cyano, nitro, and in each case straight-chain or branched alkyl, alkoxy, alkylthio, halogenoalkyl, halogenoalkoxy, halogenoalkylthio or alkoxycarbonyl in each case having 1 to 5 carbon atoms and optionally 1 to 9 identical or different halogen atoms; $R^2$ additionally represents in each case straight-chain or branched alkoxy having 1 to 8 carbon atoms, alkenyloxy having 2 to 8 carbon atoms or alkinyloxy having 2 to 8 carbon atoms and finally aralkyl, aroyl, aryl, aralkyloxy or aryloxy, in each case having 6 to 10 carbon atoms in the aryl moiety and optionally 1 to 8 carbon atoms in the alkyl moiety, and in each case optionally

monosubstituted or polysubstituted by identical or different substituents, suitable alkyl substituents optionally being halogen and cyano and suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl in each case having 1 to 6 carbon atoms in the alkyl moiety and optionally 1 to 9 identical or different halogen atoms, cycloalkyl having 3 to 6 carbon atoms and phenoxy,

X represents oxygen or sulphur,

Y represents oxygen or sulphur and

$R^3$ and $R^4$ independently of one another in each case represent hydrogen, straight-chain or branched alkyl having 1 to 4 carbon atoms or phenyl or benzyl,

apart from the compound
1-(N-4-bromophenylthiocarbamoyl)-4-phenylideneamino-1,2,4-triazoline-5-thione.

**9.** Triazolinones of the formula (Va)

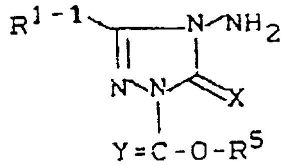

(Va)

in which

$R^{1-1}$ represents straight-chain or branched alkyl having 1 to 4 carbon atoms,

X and Y independently of one another represent oxygen or sulphur,

$R^5$ represents straight-chain or branched alkyl having 1 to 4 carbon atoms or phenyl or benzyl, optionally monosubstituted to trisubstituted by identical or different substituents, suitable substituents in each case being: halogen, cyano, nitro, in each case straight-chain or branched alkyl, alkoxy or alkylthio in each case having 1 to 4 carbon atoms or in each case straight-chain or branched halogenoalkyl, halogenoalkoxy or halogenoalkylthio in each case having 1 to 4 carbon atoms and 1 to 9 identical or different halogen atoms.

**10.** Triazolinones of the formula (Xa)

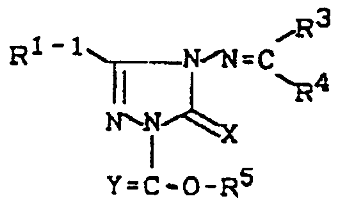

(Xa)

in which

$R^{1-1}$ represents straight-chain or branched alkyl having 1 to 4 carbon atoms,

$R^3$ and $R^4$ independently of one another in each case represent hydrogen, straight-chain or branched alkyl having 1 to 4 carbon atoms or phenyl or benzyl,

$R^5$ represents straight-chain or branched alkyl having 1 to 4 carbon atoms or phenyl or benzyl, optionally monosubstituted to trisubstituted by identical or different substituents, suitable substituents in each case being: halogen, cyano, nitro, in each case straight-chain or branched alkyl, alkoxy or alkylthio in each case having 1 to 4 carbon atoms or in each case straight-chain or branched halogenoalkyl, halogenoalkoxy or halogenoalkylthio in each case having 1 to 4 carbon atoms and 1 to 9 identical or different halogen atoms,

X and Y independently of one another represent oxygen or sulphur.

**Claims for the following Contracting State : ES**

**1.** Process for the preparation of substituted triazolinones of the formula (I)

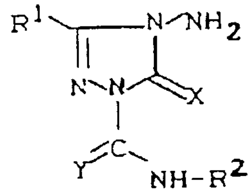

(I)

in which

$R^1$ represents hydrogen, in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl having 1 to 6 carbon atoms in the individual alkyl moieties, cycloalkyl or cycloalkylalkyl in each case having 3 to 7 carbon atoms in the cycloalkyl moiety and optionally 1 to 6 carbon atoms in the straight-chain or branched alkyl moiety, aryl having 6 to 10 carbon atoms in the aryl moiety, which is monosubstituted or polysubstituted by identical or different substituents, suitable aryl substituents in each case being: halogen, cyano, nitro and in each case straight-chain or branched alkyl, alkoxy, alkylthio, halogenoalkyl, halogenoalkoxy or halogenoalkylthio in each case having 1 to 4 carbon atoms and optionally 1 to 9 identical or different halogen atoms,

$R^2$ represents hydrogen, in each case straight-chain or branched alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl in each case having 2 to 8 carbon atoms and 1 to 15 or 13 identical or different halogen atoms, cyanoalkyl having 1 to 8 carbon atoms, hydroxyalkyl having 1 to 8 carbon atoms and 1 to 6 hydroxyl groups, alkoxyalkyl, alkoxycarbonylalkyl or alkoxycarbonylalkenyl in each case having up to 6 carbon atoms in the individual alkyl or alkenyl moieties, alkylaminoalkyl or dialkylaminoalkyl in each case having 1 to 6 carbon atoms in the individual alkyl moieties, cycloalkyl having 11 carbon atoms or cycloalkyl, cycloalkylalkyl, cycloalkenyl or cycloalkenylalkyl in each case having 3 to 8 carbon atoms in the cycloalkyl or cycloalkenyl moiety and optionally 1 to 6 carbon atoms in the straight-chain or branched alkyl moiety, in each case optionally monosubstituted or polysubstituted by identical or different substituents, suitable substituents in each case being: halogen, cyano and in each case straight-chain or branched alkyl or halogenoalkyl in each case having 1 to 4 carbon atoms and optionally 1 to 9 identical or different halogen atoms or straight-chain or branched halogenoalkenyl having up to 4 carbon atoms and 1 to 5 identical or different halogen atoms or in each case doubly linked alkanediyl or alkenediyl in each case having up to 4 carbon atoms; $R^2$ additionally represents heterocyclylalkyl having 1 to 6 carbon atoms in the straight-chain or branched alkyl moiety and 1 to 9 carbon atoms and also 1 to 3 hetero atoms - in particular nitrogen, oxygen and/or sulphur - in the heterocyclyl moiety and which is optionally monosubstituted or polysubstituted in the heterocyclyl moiety by identical or different substituents, suitable substituents being: halogen, cyano, nitro, and in each case straight-chain or branched alkyl, alkoxy, alkylthio, halogenoalkyl, halogenoalkoxy, halogenoalkylthio or alkoxycarbonyl in each case having 1 to 5 carbon atoms and optionally 1 to 9 identical or different halogen atoms; $R^2$ additionally represents in each case straight-chain or branched alkoxy having 1 to 8 carbon atoms, alkenyloxy having 2 to 8 carbon atoms or alkinyloxy having 2 to 8 carbon atoms and finally aralkyl, aroyl, aryl, aralkyloxy or aryloxy, in each case having 6 to 10 carbon atoms in the aryl moiety and optionally 1 to 8 carbon atoms in the alkyl moiety, and in each case optionally monosubstituted or polysubstituted by identical or different

substituents, suitable alkyl substituents optionally being halogen and cyano and suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl in each case having 1 to 6 carbon atoms in the alkyl moiety and optionally 1 to 9 identical or different halogen atoms, cycloalkyl having 3 to 6 carbon atoms and phenoxy,

X represents oxygen or sulphur and

Y represents oxygen or sulphur,

characterized in that

a) hydrazones of the formula (II)

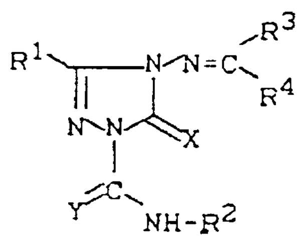

(II)

in which

$R^1$, $R^2$, X and Y have the abovementioned meaning and

$R^3$ and $R^4$ independently of one another in each case represent hydrogen, alkyl, aralkyl or aryl,

are reacted with an acid, if appropriate in the presence of a diluent,

or in that

(b) 1H-triazolinones of the formula (III)

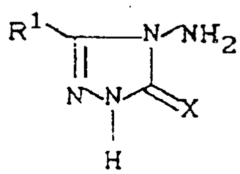

(III)

in which

$R^1$ and X have the abovementioned meaning,

are reacted with iso(thio)cyanates of the formula (IV)

$R^2$-N = C = Y (IV)

in which

$R^2$ and Y have the abovementioned meaning,

if appropriate in the presence of a diluent and if appropriate in the presence of a reaction auxiliary,

or in that

(c) triazolinones of the formula (V)

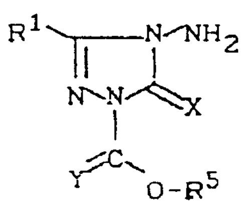

(V)

in which
$R^1$, X and Y have the abovementioned meaning,
and
$R^5$ represents alkyl, aryl or arylalkyl,
are reacted with amines of the formula (VI)

$R^2$-NH2 (VI)

in which
$R^2$ has the abovementioned meaning,
if appropriate in the presence of a diluent and if appropriate in the presence of a reaction auxiliary.

**2.** Process for the preparation of substituted triazolinones of the formula (I) according to Claim 1, characterized in that

$R^1$ represents hydrogen, methyl, ethyl, n- or i-propyl, n-, i-, s- or t-butyl, n- or i-pentyl, n- or i-hexyl, allyl, propargyl, straight-chain or branched halogenoalkyl having 1 to 4 carbon atoms and 1 to 9 identical or different halogen atoms, in particular fluorine, chlorine or bromine, methoxymethyl, ethoxymethyl, propoxymethyl, cyclopentyl, cyclohexyl, cyclopropyl, cyclopropylmethyl, cyclohexylmethyl, cyclohexylethyl,
phenyl which is optionally monosubstituted to trisubstituted by identical or different substituents, suitable substituents being:
fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio,

$R^2$ represents hydrogen, methyl, ethyl, n- or i-propyl, n-, i-, s- or t-butyl, in each case straight-chain or branched pentyl, hexyl, heptyl, octyl, nonyl, decyl, dodecyl, allyl, in each case straight-chain or branched butenyl, pentenyl or hexenyl, propargyl, in each case straight-chain or branched butinyl, pentinyl or hexinyl, straight-chain or branched halogenoalkyl having 1 to 8 carbon atoms and 1 to 9 identical or different halogen atoms, in particular fluorine, chlorine or bromine, in each case straight-chain or branched halogenoalkenyl or halogenoakynyl in each case having 3 to 8 carbon atoms and 1 to 3 halogen atoms, in particular fluorine or chlorine, in each case straight-chain or branched cyanoalkyl having 1 to 6 carbon atoms in the alkyl moiety, hydroxyalkyl having 1 to 6 carbon atoms and 1 to 3 hydroxyl groups, alkoxyalkyl, alkoxycarbonylalkyl or alkoxycarbonylalkenyl, alkylaminoalkyl or dialkylaminoalkyl in each case having up to 4 carbon atoms in the individual alkyl or alkenyl moieties or cyclopropyl, cyclopropylmethyl, cyclopropylethyl, cyclopentyl, cyclohexyl, cycloheptyl, cyclooctyl, cyclohexylmethyl, cyclohexylethyl, cyclohexenyl or cyclohexenylmethyl, in each case optionally monosubstituted to trisubstituted by identical or different substituents, suitable substituents in each case being: fluorine, chlorine, bromine, methyl, ethyl, n- or i-propyl, n-, i-, s- or t-butyl, cyano, methanediyl, ethanediyl, butanediyl or butadienediyl or dichloroallyl;

$R^2$ furthermore represents heterocyclylmethyl, heterocyclylpropyl or heterocyclylethyl which are optionally monosubstituted to trisubstituted in the heterocyclyl moiety by identical or different substituents, suitable heterocycles in each case being:

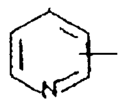 ; 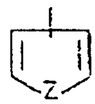 ; 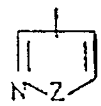 ; 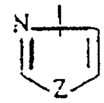 ;

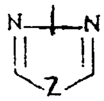 ; 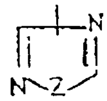 ; 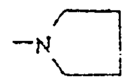 ; 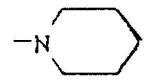 ;

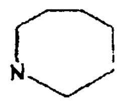 ; 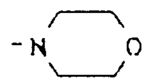 or 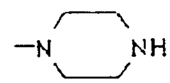 ,

Z in each case representing oxygen or sulphur and suitable substituents in each case being: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio;

$R^2$ additionally represents in each case straight-chain or branched alkoxy having 1 to 6 carbon atoms, alkenyloxy having 3 to 6 carbon atoms or alkinyloxy having 3 to 6 carbon atoms or optionally straight-chain or branched benzyl, phenylethyl, phenylpropyl, phenylbutyl, phenylpentyl, phenylhexyl, phenylheptyl, phenylcyanomethyl, phenylcyanoethyl, phenylcyanopropyl, benzyloxy, phenylethoxy, phenoxy, benzoyl, phenyl or naphthyl, in each case optionally monosubstituted to trisubstituted by identical or different substituents, suitable phenyl substituents in each case being: fluorine, chlorine, bromine, hydroxyl, cyano, nitro, methyl, ethyl, n- or i-propyl, n-, i-, s- or t-butyl, methoxy, ethoxy, methylthio, trifluoromethyl, trifluoromethoxy, trifluoromethylthio, trifluoromethylsulphinyl, trifluoromethylsulphonyl, methylsulphinyl, methylsulphonyl, acetyl, propionyl, methoxycarbonyl, ethoxycarbonyl, cyclohexyl or phenoxy,

X represents oxygen or sulphur and

Y represents oxygen or sulphur.

**3.** Herbicidal agents, characterized in that they contain at least one substituted triazolinone of the formula (I) according to Claims 1 and 2.

**4.** Process for the control of weeds, characterized in that substituted triazolinones of the formula (I) according to Claims 1 and 2 are allowed to act on the weeds and/or their environment.

**5.** Use of substituted triazolinones of the formula (I) according to Claims 1 and 2 for the control of weeds.

**6.** Process for the production of herbicidal agents, characterized in that substituted triazolinones of the formula (I) according to Claims 1 and 2 are mixed with extenders and/or surface-active substances.

**7.** Process for the preparation of hydrazones of the formula (II)

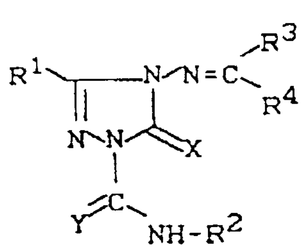

(II)

in which

$R^1$ represents hydrogen, in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl having 1 to 6 carbon atoms in the individual alkyl moieties, cycloalkyl or cycloalkylalkyl in each case having 3 to 7 carbon atoms in the cycloalkyl moiety and optionally 1 to 6 carbon atoms in the straight-chain or branched alkyl moiety, aryl having 6 to 10 carbon atoms in the aryl moiety, which is monosubstituted or polysubstituted by identical or different substituents,
suitable aryl substituents in each case being: halogen, cyano, nitro and in each case straight-chain or branched alkyl, alkoxy, alkylthio, halogenoalkyl, halogenoalkoxy or halogenoalkylthio in each case having 1 to 4 carbon atoms and optionally 1 to 9 identical or different halogen atoms,

$R^2$ represents hydrogen, in each case straight-chain or branched alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl in each case having 2 to 8 carbon atoms and 1 to 15 or 13 identical or different halogen atoms, cyanoalkyl having 1 to 8 carbon atoms, hydroxyalkyl having 1 to 8 carbon atoms and 1 to 6 hydroxyl groups, alkoxyalkyl, alkoxycarbonylalkyl or alkoxycarbonylalkenyl in each case having up to 6 carbon atoms in the individual alkyl or alkenyl moieties, alkylaminoalkyl or dialkylaminoalkyl in each case having 1 to 6 carbon atoms in the individual alkyl moieties, cycloalkyl having 11 carbon atoms or cycloalkyl, cycloalkylalkyl, cycloalkenyl or cycloalkenylalkyl in each case having 3 to 8 carbon atoms in the cycloalkyl or cycloalkenyl moiety and optionally 1 to 6 carbon atoms in the straight-chain or branched alkyl moiety, in each case optionally monosubstituted or polysubstituted by identical or different substituents, suitable substituents in each case being: halogen, cyano and in each case straight-chain or branched alkyl or halogenoalkyl in each case having 1 to 4 carbon atoms and optionally 1 to 9 identical or different halogen atoms or straight-chain or branched halogenoalkenyl having up to 4 carbon atoms and 1 to 5 identical or different halogen atoms or in each case doubly linked alkanediyl or alkenediyl in each case having up to 4 carbon atoms; $R^2$ additionally represents heterocyclylalkyl having 1 to 6 carbon atoms in the straight-chain or branched alkyl moiety and 1 to 9 carbon atoms and also 1 to 3 hetero atoms - in particular nitrogen, oxygen and/or sulphur - in the heterocyclyl moiety and which is optionally monosubstituted or polysubstituted in the heterocyclyl moiety by identical or different substituents, suitable substituents being: halogen, cyano, nitro, and in each case straight-chain or branched alkyl, alkoxy, alkylthio, halogenoalkyl, halogenoalkoxy, halogenoalkylthio or alkoxycarbonyl in each case having 1 to 5 carbon atoms and optionally 1 to 9 identical or different halogen atoms; $R^2$ additionally represents in each case straight-chain or branched alkoxy having 1 to 8 carbon atoms, alkenyloxy having 2 to 8 carbon atoms or alkinyloxy having 2 to 8 carbon atoms and finally aralkyl, aroyl, aryl, aralkyloxy or aryloxy, in each case having 6 to 10 carbon atoms in the aryl moiety and optionally 1 to 8 carbon atoms in the alkyl moiety, and in each case optionally monosubstituted or polysubstituted by identical or different substituents, suitable alkyl substituents optionally being halogen and cyano and suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl in each case having 1 to 6 carbon atoms in the alkyl moiety and optionally 1 to 9 identical or different halogen atoms, cycloalkyl having 3 to 6 carbon atoms and phenoxy,

X represents oxygen or sulphur,

Y represents oxygen or sulphur and

$R^3$ and $R^4$ independently of one another in each case represent hydrogen, straight-chain or branched alkyl having 1 to 4 carbon atoms or phenyl or benzyl,

apart from the compound

1-(N-4-bromophenylthiocarbamoyl)-4-phenylideneamino-1,2,4-triazoline-5-thione, characterized in that 1-unsubstituted 4-amino-triazolinones of the formula (III)

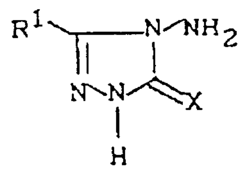

(III)

in which
$R^1$ and X have the abovementioned meaning,
are reacted with aldehydes or ketones of the formula (VII)

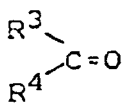

(VII)

in which
$R^3$ and $R^4$ have the abovementioned meaning,
if appropriate in the presence of a diluent such as, for example, dichloromethane or toluene and if appropriate in a subsequent 3rd step reacted with amines of the formula (VI)

$R^2-NH_2$ (VI)

in which
$R^2$ has the abovementioned meaning,
if appropriate in the presence of a diluent such as, for example, tetrahydrofuran and if appropriate in the presence of a base such as, for example, sodium hydroxide or potassium hydroxide, at temperatures between 20 °C and 50 °C.

**8.** Process for the preparation of triazolinones of the formula (Va)

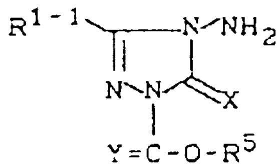

(Va)

in which

| | |
|---|---|
| $R^{1-1}$ | represents straight-chain or branched alkyl having 1 to 4 carbon atoms and |
| $R^5$ | represents straight-chain or branched alkyl having 1 to 4 carbon atoms or phenyl or benzyl, optionally monosubstituted to trisubstituted by identical or different substituents, suitable substituents in each case being: halogen, cyano, nitro, in each case straight-chain or branched alkyl, alkoxy or alkylthio in each case having 1 to 4 carbon atoms or in each case straight-chain or branched halogenoalkyl, halogenoalkoxy or halogenoalkylthio in each case having 1 to 4 carbon atoms and 1 to 9 identical or different halogen atoms, |
| X and Y | independently of one another represent oxygen or sulphur, |

characterized in that 1H-triazolinones of the formula (IIIa)

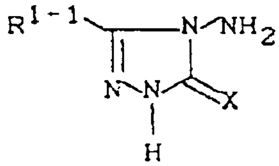

(IIIa)

in which
$R^{1-1}$ and X have the abovementioned meaning,
are reacted with (thio)chloroformic acid esters of the formula (IX)

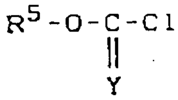

(IX)

in which
$R^5$ and Y have the abovementioned meaning,
if appropriate in the presence of a diluent such as, for example, tetrahydrofuran and if appropriate in the presence of a reaction auxiliary such as, for example, potassium t-butoxide or sodium hydride, at temperatures between -20 °C and +40 °C.

**9.** Process for the preparation of triazolinones of the formula (Xa)

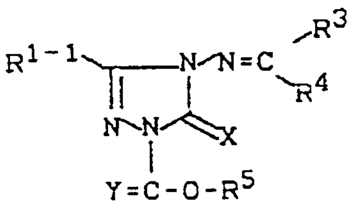

(Xa)

in which

| | |
|---|---|
| $R^{1-1}$ | represents straight-chain or branched alkyl having 1 to 4 carbon atoms, |
| $R^3$ and $R^4$ | independently of one another in each case represent hydrogen, straight-chain or branched alkyl having 1 to 4 carbon atoms or phenyl or benzyl, |
| $R^5$ | represents straight-chain or branched alkyl having 1 to 4 carbon atoms or phenyl or benzyl, optionally monosubstituted to trisubstituted by identical or different substituents, suitable substituents in each case being: halogen, cyano, nitro, in each case straight-chain or branched alkyl, alkoxy or alkylthio in each case having 1 to 4 carbon atoms or in each case straight-chain or branched halogenoalkyl, halogenoalkoxy or halogenoalkylthio in each case having 1 to 4 carbon atoms and 1 to 9 identical or different halogen atoms, |
| X and Y | independently of one another represent oxygen or sulphur, |

characterized in that 1-unsubstituted 4-amino-triazolinones of the formula (III)

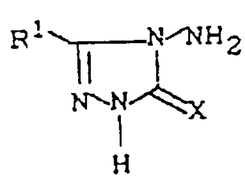

(III)

in which
$R^1$ and X have the meanings mentioned in the description are reacted with aldehydes or ketones

of the formula (VII)

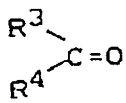

(VII)

in which
$R^3$ and $R^4$ have the meanings mentioned in the description
if appropriate in the presence of a diluent such as, for example, dichloromethane or toluene and if appropriate in the presence of a catalyst such as, for example, p-toluenesulphonic acid, at temperatures between 40 °C and 120 °C and the 1-unsubstituted triazolinone hydrazones obtainable in this way, of the formula (VIII)
in which

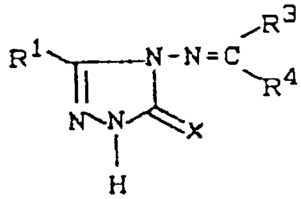

(VIII)

$R^1$, $R^3$, $R^4$ and X have the meanings given in the description,
are reacted in a following 2nd step with (thio)chloroformic acid esters of the formula (IX)

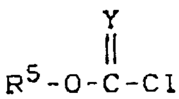

(IX)

in which
$R^5$ and Y have the meanings given in the description,
if appropriate in the presence of a diluent such as, for example, tetrahydrofuran and if appropriate in the presence of a reaction auxiliary such as, for example, sodium hydride or potassium t-butoxide, at temperatures between -20 °C and +40 °C.

**Revendications**

**Revendications pour les Etats contractants suivants : DE, AU, CH, LI, IT, FR, BE, NL, GB, SE, GR**

**1.** Triazolinones substituées répondant à la formule (I)

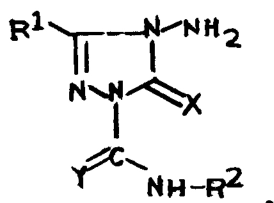

(I)

dans laquelle
$R^1$ représente un atome d'hydrogène; un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle contenant

de 2 à 8 atomes de carbone et de 1 à 15 atomes d'halogène identiques ou différents, un groupe halogénalcynyle contenant de 2 à 8 atomes de carbone et de 1 à 13 atomes d'halogène identiques ou différents, un groupe alcoxyalkyle contenant de 1 à 6 atomes de carbone dans les fractions alkyle individuelles, chacun de ces groupes étant à chaîne droite ou ramifiée; un groupe cycloalkyle ou un groupe cycloalkylalkyle contenant chacun de 3 à 7 atomes de carbone dans la fraction cycloalkyle et éventuellement de 1 à 6 atomes de carbone dans la fraction alkyle à chaîne droite ou ramifiée; un groupe aryle contenant de 6 à 10 atomes de carbone dans la fraction aryle, substitué une ou plusieurs fois de manière identique ou différente, dans lequel, comme substituants du groupe aryle, entrent chaque fois en ligne de compte : un atome d'halogène, un groupe cyano, un groupe nitro; ainsi qu'un groupe alkyle, un groupe alcoxy, un groupe alkylthio, un groupe halogénalkyle, un groupe halogénalcoxy ou un groupe halogénalkylthio contenant chacun de 1 à 4 atomes de carbone et éventuellement de 1 à 9 atomes d'halogène identiques ou différents, chacun de ces groupes étant à chaîne droite ou ramifiée,

$R^2$ représente un atome d'hydrogène; un groupe alkyle contenant de 1 à 18 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou halogénalcynyle contenant chacun de 2 à 8 atomes de carbone et de 1 à 15 ou 13 atomes d'halogène identiques ou différents, un groupe cyanalkyle contenant de 1 à 8 atomes de carbone, un groupe hydroxyalkyle contenant de 1 à 8 atomes de carbone et de 1 à 6 groupes hydroxyle, un groupe alcoxyalkyle, un groupe alcoxycarbonylalkyle ou un groupe alcoxycarbonylalcényle contenant chacun jusqu'à 6 atomes de carbone dans les fractions alkyle ou alcényle individuelles, un groupe alkylaminoalkyle ou dialkylaminoalkyle contenant chacun de 1 à 6 atomes de carbone dans les fractions alkyle individuelles, chacun de ces groupes étant à chaîne droite ou ramifiée; un groupe cycloalkyle contenant 11 atomes de carbone; ou encore un groupe cycloalkyle, un groupe cycloalkylalkyle, un groupe cycloalcényle ou un groupe cycloalcénylalkyle contenant chacun de 3 à 8 atomes de carbone dans la fraction cycloalkyle ou cycloalcényle et éventuellement de 1 à 6 atomes de carbone dans la fraction alkyle à chaîne droite ou ramifiée, chacun ces groupes étant éventuellement substitué une ou plusieurs fois de manière identique ou différente, dans lesquels, comme substituants, entrent chaque fois en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée; ou bien un groupe halogénalcényle à chaîne droite ou ramifiée contenant jusqu'à 4 atomes de carbone et de 1 à 5 atomes d'halogène identiques ou différents ou encore un groupe alcanediyle ou alcènediyle contenant chacun jusqu'à 4 atomes de carbone et étant chacun deux fois lié;

$R^2$ représente, en outre, un groupe hétérocyclylalkyle contenant de 1 à 6 atomes de carbone dans la fraction alkyle à chaîne droite ou ramifiée et de 1 à 9 atomes de carbone, ainsi que de 1 à 3 hétéro-atomes - en particulier un atome d'azote, un atome d'oxygène et/ou un atome de soufre - dans la fraction hétérocyclyle, éventuellement substitué une ou plusieurs fois de manière identique ou différente dans la fraction hétérocyclyle, dans lequel, comme substituants, entrent en ligne de compte : un atome d'halogène, un groupe cyano, un groupe nitro; ainsi qu'un groupe alkyle, un groupe alcoxy, un groupe alkylthio, un groupe halogénalkyle, un groupe halogénalcoxy, un groupe halogénalkylthio ou un groupe alcoxycarbonyle contenant chacun de 1 à 5 atomes de carbone et éventuellement de 1 à 9 atomes d'halogène identiques ou différents, chacun de ces groupes étant à chaîne droite ou ramifiée; $R^2$ représente, en outre, un groupe alcoxy contenant de 1 à 8 atomes de carbone, un groupe alcényloxy contenant de 2 à 8 atomes de carbone ou un groupe alcynyloxy contenant de 2 à 8 atomes de carbone, chacun de ces groupes étant à chaîne droite ou ramifiée; et enfin un groupe aralkyle, un groupe aroyle, un groupe aryle, un groupe aralkyloxy ou un groupe aryloxy contenant chacun de 6 à 10 atomes de carbone dans la fraction aryle et éventuellement de 1 à 8 atomes de carbone dans la fraction alkyle, chacun de ces groupes étant éventuellement substitué une ou plusieurs fois de manière identique ou différente, dans lesquels, comme substituants du groupe alkyle, on envisage éventuellement un atome d'halogène et un groupe cyano, et dans lesquels, comme substituants du groupe aryle, entrent chaque fois en ligne de compte : un atome d'halogène, un groupe cyano, un groupe

nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halogénalkylsulfonyle, un groupe alcanoyle ou un groupe alcoxycarbonyle contenant chacun de 1 à 6 atomes de carbone dans la fraction alkyle et éventuellement de 1 à 9 atomes d'halogène identiques ou différents, chacun de ces groupes étant à chaîne droite ou ramifiée; un groupe cycloalkyle contenant de 3 à 6 atomes de carbone et un groupe phénoxy;

X représente un atome d'oxygène ou un atome de soufre; et

Y représente un atome d'oxygène ou un atome de soufre.

**2.** Triazolinones substituées de formule (I) selon la revendication 1, caractérisées en ce que

$R^1$ représente un atome d'hydrogène, un groupe méthyle, un groupe éthyle, un groupe n- ou i-propyle, un groupe n-, i-, s- ou t-butyle, un groupe n- ou i-pentyle, un groupe n- ou i-hexyle; un groupe allyle, un groupe propargyle; un groupe halogénalkyle à chaîne droite ou ramifiée contenant de 1 à 4 atomes de carbone et de 1 à 9 atomes d'halogène identiques ou différents, en particulier un atome de fluor, un atome de chlore ou un atome de brome; un groupe méthoxyméthyle, un groupe éthoxyméthyle, un groupe propoxyméthyle, un groupe cyclopentyle, un groupe cyclohexyle, un groupe cyclopropyle, un groupe cyclopropylméthyle, un groupe cyclohexylméthyle, un groupe cyclohexyléthyle; un groupe phényle éventuellement substitué de 1 à 3 fois de manière identique ou différente, dans lequel, comme substituants on envisage : un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy ou un groupe trifluorométhylthio,

$R^2$ représente un atome d'hydrogène; un groupe méthyle, un groupe éthyle, un groupe n- ou i-propyle, un groupe n-, i-, s- ou t-butyle; un groupe pentyle, un groupe hexyle, un groupe heptyle, un groupe octyle, un groupe nonyle, un groupe décyle, un groupe dodécyle, chacun étant à chaîne droite ou ramifiée; un groupe allyle; un groupe butényle, un groupe pentényle ou un groupe hexényle, chacun étant à chaîne droite ou ramifiée; un groupe propargyle; un groupe butynyle, un groupe pentynyle ou un groupe hexynyle, chacun étant à chaîne droite ou ramifiée; un groupe halogénalkyle à chaîne droite ou ramifiée contenant de 1 à 8 atomes de carbone et de 1 à 9 atomes d'halogène identiques ou différents, en particulier un atome de fluor, un atome de chlore ou un atome de brome; un groupe halogénalcényle ou un groupe halogénalcynyle contenant chacun de 3 à 8 atomes de carbone et de 1 à 3 atomes d'halogène, en particulier un atome de fluor ou un atome de chlore, chacun de ces groupes étant à chaîne droite ou ramifiée; un groupe cyanalkyle contenant de 1 à 6 atomes de carbone dans la fraction alkyle, un groupe hydroxyalkyle contenant de 1 à 6 atomes de carbone et de 1 à 3 groupes hydroxyle, un groupe alcoxyalkyle, un groupe alcoxycarbonylalkyle ou un groupe alcoxycarbonylalcényle, un groupe alkylaminoalkyle ou un groupe dialkylaminoalkyle contenant chacun jusqu'à 4 atomes de carbone dans les fractions alkyle ou alcényle individuelles, chacun de ces groupes étant à chaîne droite ou ramifiée; ou encore représente un groupe cyclopropyle, un groupe cyclopropylméthyle, un groupe cyclopropyléthyle, un groupe cyclopentyle, un groupe cyclohexyle, un groupe cycloheptyle, un groupe cyclooctyle, un groupe cyclohexylméthyle, un groupe cyclohexyléthyle, un groupe cyclohexényle ou un groupe cyclohexénylméthyle, chacun de ces groupes étant éventuellement substitué de 1 à 3 fois de manière identique ou différente, dans lesquels, comme substituants, entrent chaque fois en ligne de compte : un atome de fluor, un atome de chlore, un atome de brome, un groupe méthyle, un groupe éthyle, un groupe n- ou i-propyle, un groupe n-, i-, s- ou t-butyle, un groupe cyano, un groupe méthanediyle, un groupe éthanediyle, un groupe butanediyle ou un groupe butadiènediyle ou encore un groupe dichloroallyle;

$R^2$ représente, en outre, un groupe hétérocyclylméthyle, un groupe hétérocyclylpropyle ou un groupe hétérocyclyléthyle éventuellement substitués de 1 à 3 fois de manière identique ou différente dans la fraction hétérocyclyle, dans lesquels, comme composés hétérocycliques, entrent chaque fois en ligne de compte :

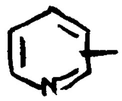 ; 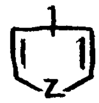 ; 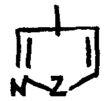 ; 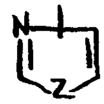 ;

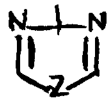 ; 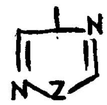 ; 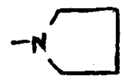 ; 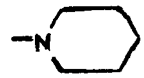 ;

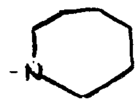 ; 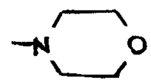 ou 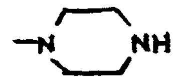

dans lesquels Z représente respectivement un atome d'oxygène ou un atome de soufre, et dans lesquels, comme substituants, entrent chaque fois en ligne de compte : un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy ou un groupe trifluorométhylthio;

$R^2$ représente, en outre, un groupe alcoxy contenant de 1 à 6 atomes de carbone, un groupe alcényloxy contenant de 3 à 6 atomes de carbone ou un groupe alcynyloxy contenant de 3 à 6 atomes de carbone, chacun de ces groupes étant à chaîne droite ou ramifiée; ou représente un groupe benzyle, un groupe phényléthyle, un groupe phénylpropyle, un groupe phénylbutyle, un groupe phénylpentyle, un groupe phénylhexyle, un groupe phénylheptyle, un groupe phénylcyanométhyle, un groupe phénylcyanoéthyle, un groupe phénylcyanopropyle, un groupe benzyloxy, un groupe phényléthyloxy, un groupe phénoxy, un groupe benzoyle, un groupe phényle ou un groupe naphtyle, chacun de ces groupes étant éventuellement substitué de 1 à 3 fois de manière identique ou différente, dans lesquels, comme substituants du groupe phényle, entrent chaque fois en ligne de compte : un atome de fluor, un atome de chlore, un atome de brome, un groupe hydroxyle, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy, un groupe trifluorométhylthio, un groupe trifluorométhylsulfinyle, un groupe trifluorométhylsulfonyle, un groupe méthylsulfinyle, un groupe méthylsulfonyle, un groupe acétyle, un groupe propionyle, un groupe méthoxycarbonyle, un groupe éthoxycarbonyle, un groupe cyclohexyle ou un groupe phénoxy,

X représente un atome d'oxygène ou un atome de soufre; et

Y représente un atome d'oxygène ou un atome de soufre.

**3.** Procédé pour la préparation de triazolinones substituées de formule (I)

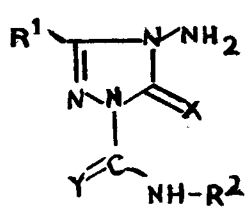

(I)

dans laquelle

$R^1$ représente un atome d'hydrogène; un groupe alkyle contenant de 1 à 8 atomes de carbone, un groupe alcényle contenant de 2 à 8 atomes de carbone, un groupe alcynyle contenant de

2 à 8 atomes de carbone, un groupe halogénalkyle contenant de 1 à 8 atomes de carbone et de 1 à 17 atomes d'halogène identiques ou différents, un groupe halogénalcényle contenant de 2 à 8 atomes de carbone et de 1 à 15 atomes d'halogène identiques ou différents, un groupe halogénalcynyle contenant de 2 à 8 atomes de carbone et de 1 à 13 atomes d'halogène identiques ou différents, un groupe alcoxyalkyle contenant de 1 à 6 atomes de carbone dans les fractions alkyle individuelles, chacun de ces groupes étant à chaîne droite ou ramifiée; un groupe cycloalkyle ou un groupe cycloalkylalkyle contenant chacun de 3 à 7 atomes de carbone dans la fraction cycloalkyle et éventuellement de 1 à 6 atomes de carbone dans la fraction alkyle à chaîne droite ou ramifiée; un groupe aryle contenant de 6 à 10 atomes de carbone dans la fraction aryle, substitué une ou plusieurs fois de manière identique ou différente, dans lequel, comme substituants du groupe aryle, entrent chaque fois en ligne de compte : un atome d'halogène, un groupe cyano, un groupe nitro; ainsi qu'un groupe alkyle, un groupe alcoxy, un groupe alkylthio, un groupe halogénalkyle, un groupe halogénalcoxy ou un groupe halogénalkylthio contenant chacun de 1 à 4 atomes de carbone et éventuellement de 1 à 9 atomes d'halogène identiques ou différents, chacun de ces groupes étant à chaîne droite ou ramifiée,

$R^2$ représente un atome d'hydrogène; un groupe alkyle contenant de 1 à 18 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou halogénalcynyle contenant chacun de 2 à 8 atomes de carbone et de 1 à 15 ou 13 atomes d'halogène identiques ou différents, un groupe cyanalkyle contenant de 1 à 8 atomes de carbone, un groupe hydroxyalkyle contenant de 1 à 8 atomes de carbone et de 1 à 6 groupes hydroxyle, un groupe alcoxyalkyle, un groupe alcoxycarbonylalkyle ou un groupe alcoxycarbonylalcényle contenant chacun jusqu'à 6 atomes de carbone dans les fractions alkyle ou alcényle individuelles, un groupe alkylaminoalkyle ou dialkylaminoalkyle contenant chacun de 1 à 6 atomes de carbone dans les fractions alkyle individuelles; un groupe cycloalkyle contenant 11 atomes de carbone; ou encore un groupe cycloalkyle, un groupe cycloalkylalkyle, un groupe cycloalcényle ou un groupe cycloalcénylalkyle contenant chacun de 3 à 8 atomes de carbone dans la fraction cycloalkyle ou cycloalcényle et éventuellement de 1 à 6 atomes de carbone dans la fraction alkyle à chaîne droite ou ramifiée, chacun de ces groupes étant éventuellement substitué une ou plusieurs fois de manière identique ou différente, dans lesquels, comme substituants, entrent chaque fois en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée; ou bien un groupe halogénalcényle à chaîne droite ou ramifiée contenant jusqu'à 4 atomes de carbone et de 1 à 5 atomes d'halogène identiques ou différents ou encore un groupe alcanediyle ou alcènediyle contenant chacun jusqu'à 4 atomes de carbone et étant chacun deux fois lié;
$R^2$ représente, en outre, un groupe hétérocyclylalkyle contenant de 1 à 6 atomes de carbone dans la fraction alkyle à chaîne droite ou ramifiée et de 1 à 9 atomes de carbone, ainsi que de 1 à 3 hétéro-atomes - en particulier un atome d'azote, un atome d'oxygène et/ou un atome de soufre - dans la fraction hétérocyclyle, éventuellement substitué une ou plusieurs fois de manière identique ou différente dans la fraction hétérocyclyle, dans lequel, comme substituants, entrent en ligne de compte : un atome d'halogène, un groupe cyano, un groupe nitro; ainsi qu'un groupe alkyle, un groupe alcoxy, un groupe alkylthio, un groupe halogénalkyle, un groupe halogénalcoxy, un groupe halogénalkylthio ou un groupe alcoxycarbonyle contenant chacun de 1 à 5 atomes de carbone et éventuellement de 1 à 9 atomes d'halogène identiques ou différents, chacun de ces groupes étant à chaîne droite ou ramifiée; $R^2$ représente, en outre, un groupe alcoxy contenant de 1 à 8 atomes de carbone, un groupe alcényloxy contenant de 2 à 8 atomes de carbone ou un groupe alcynyloxy contenant de 2 à 8 atomes de carbone, chacun de ces groupes étant à chaîne droite ou ramifiée; et enfin un groupe aralkyle, un groupe aroyle, un groupe aryle, un groupe aralkyloxy ou un groupe aryloxy contenant chacun de 6 à 10 atomes de carbone dans la fraction aryle et éventuellement de 1 à 8 atomes de carbone dans la fraction alkyle, chacun de ces groupes étant éventuellement substitué une ou plusieurs fois de manière identique ou différente, dans, lesquels, comme substituants du groupe alkyle, on envisage éventuellement un atome d'halogène et un groupe cyano, et dans lesquels, comme substituants du groupe aryle,

entrent chaque fois en ligne de compte : un atome d'halogène, un groupe cyano, un groupe nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halogénalkylsulfonyle, un groupe alcanoyle ou un groupe alcoxycarbonyle contenant chacun de 1 à 6 atomes de carbone dans la fraction alkyle et éventuellement de 1 à 9 atomes d'halogène identiques ou différents, chacun de ces groupes étant à chaîne droite ou ramifiée; un groupe cycloalkyle contenant de 3 à 6 atomes de carbone et un groupe phénoxy;

X représente un atome d'oxygène ou un atome de soufre; et

Y représente un atome d'oxygène ou un atome de soufre,

caractérisé en ce qu'on fait réagir

a) des hydrazones de formule (II)

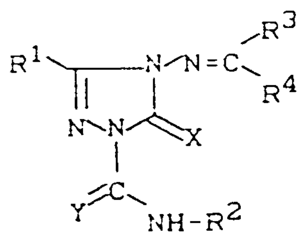

(II)

dans laquelle $R^1$, $R^2$, X et Y ont la signification indiquée ci-dessus, et

$R^3$ et $R^4$ représentent chacun, indépendamment l'un de l'autre, un atome d'hydrogène, un groupe alkyle, un groupe aralkyle ou un groupe aryle,

avec un acide, éventuellement en présence d'un diluant;

ou bien en ce qu'on fait réagir

b) des 1H-triazolinones de formule (III)

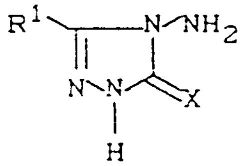

(III)

dans laquelle $R^1$ et X ont la signification indiquée ci-dessus,

avec des iso(thio)cyanates de formule (IV)

$R^2\text{-}N=C=Y$ (IV)

dans laquelle $R^2$ et Y ont la signification indiquée ci-dessus,

éventuellement en présence d'un diluant et éventuellement en présence d'un adjuvant réactionnel;

ou bien en ce qu'on fait réagir

c) des triazolinones de formule (V)

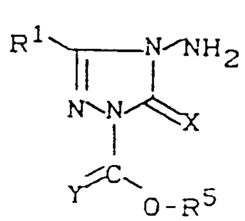

(V)

dans laquelle $R^1$, X et Y ont la signification indiquée ci-dessus, et $R^5$ représente un groupe alkyle, un groupe aryle ou un groupe arylalkyle,

avec des amines de formule (VI)

$R^2-NH_2$ (VI)

dans laquelle $R^2$ a la signification indiquée ci-dessus,
éventuellement en présence d'un diluant et éventuellement en présence d'un adjuvant réactionnel.

**4.** Agents herbicides caractérisés par une teneur en au moins une triazolinone substituée de formule (I) selon les revendications 1 et 2.

**5.** Procédé pour lutter contre les mauvaises herbes, caractérisé en ce qu'on laisse agir des triazolinones substituées de formule (I) selon les revendications 1 et 2, sur les mauvaises herbes et/ou sur leur biotope.

**6.** Utilisation de triazolinones substituées de formule (I) selon les revendications 1 et 2, pour lutter contre les mauvaises herbes.

**7.** Procédé pour la préparation d'agents herbicides, caractérisé en ce qu'on mélange des triazolinones substituées de formule (I) selon les revendications 1 et 2, avec des diluants et/ou des substances tensioactives.

**8.** Hydrazones de formule (II)

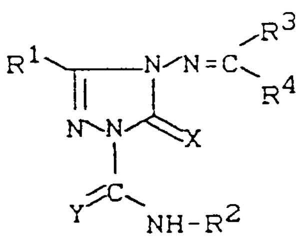

(II)

dans laquelle

$R^1$ représente un atome d'hydrogène; un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle contenant de 2 à 8 atomes de carbone et de 1 à 15 atomes d'halogène identiques ou différents, un groupe halogénalcynyle contenant de 2 à 8 atomes de carbone et de 1 à 13 atomes d'halogène identiques ou différents, un groupe alcoxyalkyle contenant de 1 à 6 atomes de carbone dans les fractions alkyle individuelles, chacun de ces groupes étant à chaîne droite ou ramifiée; un groupe cycloalkyle ou un groupe cycloalkylalkyle contenant chacun de 3 à 7 atomes de carbone dans la fraction cycloalkyle et éventuellement de 1 à 6 atomes de carbone dans la fraction alkyle à chaîne droite ou ramifiée; un groupe aryle contenant de 6 à 10 atomes de carbone dans la fraction aryle, substitué une ou plusieurs fois de manière identique ou différente, dans lequel, comme substituants du groupe aryle, entrent chaque fois en ligne de compte : un atome d'halogène, un groupe cyano, un groupe nitro; ainsi qu'un groupe alkyle, un groupe alcoxy, un groupe alkylthio, un groupe halogénalkyle, un groupe halogénalcoxy ou un groupe halogénalkylthio contenant chacun de 1 à 4 atomes de carbone et éventuellement de 1 à 9 atomes d'halogène identiques ou différents, chacun de ces groupes étant à chaîne droite ou ramifiée,

$R^2$ représente un atome d'hydrogène; un groupe alkyle contenant de 1 à 18 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou halogénalcynyle contenant chacun de 2 à 8 atomes de carbone et de 1 à 15 ou 13 atomes d'halogène identiques ou différents, un groupe cyanalkyle contenant de 1 à 8 atomes de carbone, un groupe hydroxyalkyle contenant

de 1 à 8 atomes de carbone et de 1 à 6 groupes hydroxyle, un groupe alcoxyalkyle, un groupe alcoxycarbonyl-alkyle ou un groupe alcoxycarbonylalcényle contenant chacun jusqu'à 6 atomes de carbone dans les fractions alkyle ou alcényle individuelles, un groupe alkylaminoalkyle ou dialkylaminoalkyle contenant chacun de 1 à 6 atomes de carbone dans les fractions alkyle individuelles, chacun de ces groupes étant à chaîne droite ou ramifiée, un groupe cycloalkyle contenant 11 atomes de carbone; ou encore un groupe cycloalkyle, un groupe cycloalkylalkyle, un groupe cycloalcényle ou un groupe cycloalcénylalkyle contenant chacun de 3 à 8 atomes de carbone dans la fraction cycloalkyle ou cycloalcényle et éventuellement de 1 à 6 atomes de carbone dans la fraction alkyle à chaîne droite ou ramifiée, chacun de ces groupes étant éventuellement substitué une ou plusieurs fois de manière identique ou différente, dans lesquels, comme substituants, entrent chaque fois en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée; ou encore un groupe halogénalcényle à chaîne droite ou ramifiée contenant jusqu'à 4 atomes de carbone et de 1 à 5 atomes d'halogène identiques ou différents ou encore un groupe alcanediyle ou alcènediyle contenant chacun jusqu'à 4 atomes de carbone et étant chacun deux fois lié;

$R^2$ représente, en outre, un groupe hétérocyclylalkyle contenant de 1 à 6 atomes de carbone dans la fraction alkyle à chaîne droite ou ramifiée et de 1 à 9 atomes de carbone, ainsi que de 1 à 3 hétéro-atomes - en particulier un atome d'azote, un atome d'oxygène et/ou un atome de soufre - dans la fraction hétérocyclyle, éventuellement substitué une ou plusieurs fois de manière identique ou différente dans la fraction hétérocyclyle, dans lequel, comme substituants, entrent en ligne de compte : un atome d'halogène, un groupe cyano, un groupe nitro; ainsi qu'un groupe alkyle, un groupe alcoxy, un groupe alkylthio, un groupe halogénalkyle, un groupe halogénalcoxy, un groupe halogénalkylthio ou un groupe alcoxycarbonyle contenant chacun de 1 à 5 atomes de carbone et éventuellement de 1 à 9 atomes d'halogène identiques ou différents, chacun de ces groupes étant à chaîne droite ou ramifiée;

$R^2$ représente, en outre, un groupe alcoxy contenant de 1 à 8 atomes de carbone, un groupe alcényloxy contenant de 2 à 8 atomes de carbone ou un groupe alcynyloxy contenant de 2 à 8 atomes de carbone, chacun de ces groupes étant à chaîne droite ou ramifiée; et enfin représente un groupe aralkyle, un groupe aroyle, un groupe aryle, un groupe aralkyloxy ou un groupe aryloxy contenant chacun de 6 à 10 atomes de carbone dans la fraction aryle et éventuellement de 1 à 8 atomes de carbone dans la fraction alkyle, chacun de ces groupes étant éventuellement substitué une ou plusieurs fois de manière identique ou différente, dans lesquels, comme substituants du groupe alkyle, on envisage éventuellement un atome d'halogène et un groupe cyano, et dans lesquels, comme substituants du groupe aryle, entrent chaque fois en ligne de compte : un atome d'halogène, un groupe cyano, un groupe nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halogénalkylsulfonyle, un groupe alcanoyle ou un groupe alcoxycarbonyle contenant chacun de 1 à 6 atomes de carbone dans la fraction alkyle et éventuellement de 1 à 9 atomes d'halogène identiques ou différents, chacun de ces groupes étant à chaîne droite ou ramifiée; un groupe cycloalkyle contenant de 3 à 6 atomes de carbone et un groupe phénoxy;

X représente un atome d'oxygène ou un atome de soufre,

Y représente un atome d'oxygène ou un atome de soufre,

$R^3$ et $R^4$ représentent chacun, indépendamment l'un de l'autre, un atome d'hydrogène; un groupe alkyle à chaîne droite ou ramifiée contenant de 1 à 4 atomes de carbone, ou encore un groupe phényle ou un groupe benzyle,

à l'exception du composé

1-(N-4-bromophénylthiocarbamoyl)-4-phénylidènamino-1,2,4-triazolin-5-thione.

**9.** Triazolinones de formule (Va)

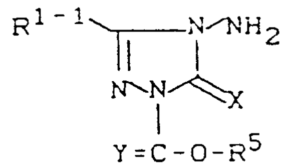

(Va)

dans laquelle

$R^{1-1}$ représente un groupe alkyle à chaîne droite ou ramifiée contenant de 1 à 4 atomes de carbone,

X et Y représentent, indépendamment l'un de l'autre, un atome d'oxygène ou un atome de soufre,

$R^5$ représente un groupe alkyle à chaîne droite ou ramifiée contenant de 1 à 4 atomes de carbone, ou encore un groupe phényle ou un groupe benzyle éventuellement substitués de 1 à 3 fois de manière identique ou différente, dans lesquels, comme substituants, entrent chaque fois en ligne de compte : un atome d'halogène, un groupe cyano, un groupe nitro; un groupe alkyle, un groupe alcoxy ou un groupe alkylthio contenant chacun de 1 à 4 atomes de carbone, chacun de ces groupes étant à chaîne droite ou ramifiée; ou encore un groupe halogénalkyle, un groupe halogénalcoxy ou un groupe halogénalkylthio contenant chacun de 1 à 4 atomes de carbone et de 1 à 9 atomes d'halogène identiques ou différents, chacun de ces groupes étant à chaîne droite ou ramifiée.

**10.** Triazolinones de formule (Xa)

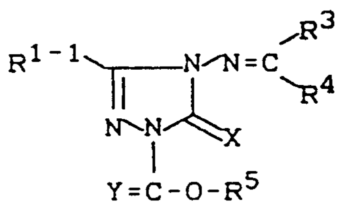

(Xa)

dans laquelle

$R^{1-1}$ représente un groupe alkyle à chaîne droite ou ramifiée contenant de 1 à 4 atomes de carbone,

$R^3$ et $R^4$ représentent chacun, indépendamment l'un de l'autre, un atome d'hydrogène; un groupe alkyle à chaîne droite ou ramifiée contenant de 1 à 4 atomes de carbone, ou encore un groupe phényle ou un groupe benzyle

$R^5$ représente de préférence un groupe alkyle à chaîne droite ou ramifiée contenant de 1 à 4 atomes de carbone, ou encore un groupe phényle ou un groupe benzyle éventuellement substitués de 1 à 3 fois de manière identique ou différente, dans lesquels, comme substituants, entrent chaque fois en ligne de compte : un atome d'halogène, un groupe cyano, un groupe nitro; un groupe alkyle, un groupe alcoxy ou un groupe alkylthio contenant chacun de 1 à 4 atomes de carbone, chacun de ces groupes étant à chaîne droite ou ramifiée; ou encore un groupe halogénalkyle, un groupe halogénalcoxy ou un groupe halogénalkylthio contenant chacun de 1 à 4 atomes de carbone et de 1 à 9 atomes d'halogène identiques ou différents, chacun de ces groupes étant à chaîne droite ou ramifiée,

X et Y représentent, indépendamment l'un de l'autre, un atome d'oxygène ou un atome de soufre.

**Revendications pour l'Etat contractant suivant : ES**

**1.** Procédé pour la préparation de triazolinones substituées de formule (I)

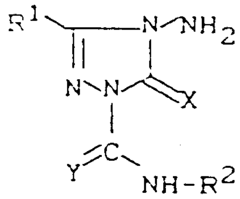

(I)

dans laquelle

$R^1$ représente un atome d'hydrogène; un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle contenant de 2 à 8 atomes de carbone et de 1 à 15 atomes d'halogène identiques ou différents, un groupe halogénalcynyle contenant de 2 à 8 atomes de carbone et de 1 à 13 atomes d'halogène identiques ou différents, un groupe alcoxyalkyle contenant de 1 à 6 atomes de carbone dans les fractions alkyle individuelles, chacun de ces groupes étant à chaîne droite ou ramifiée; un groupe cycloalkyle ou un groupe cycloalkylalkyle contenant chacun de 3 à 7 atomes de carbone dans la fraction cycloalkyle et éventuellement de 1 à 6 atomes de carbone dans la fraction alkyle à chaîne droite ou ramifiée; un groupe aryle contenant de 6 à 10 atomes de carbone dans la fraction aryle, substitué une ou plusieurs fois de manière identique ou différente, dans lequel, comme substituants du groupe aryle, entrent chaque fois en ligne de compte : un atome d'halogène, un groupe cyano, un groupe nitro; ainsi qu'un groupe alkyle, un groupe alcoxy, un groupe alkylthio, un groupe halogénalkyle, un groupe halogénalcoxy ou un groupe halogénalkylthio contenant chacun de 1 à 4 atomes de carbone et éventuellement de 1 à 9 atomes d'halogène identiques ou différents, chacun de ces groupes étant à chaîne droite ou ramifiée,

$R^2$ représente un atome d'hydrogène; un groupe alkyle contenant de 1 à 18 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou halogénalcynyle contenant chacun de 2 à 8 atomes de carbone et de 1 à 15 ou 13 atomes d'halogène identiques ou différents, un groupe cyanalkyle contenant de 1 à 8 atomes de carbone, un groupe hydroxyalkyle contenant de 1 à 8 atomes de carbone et de 1 à 6 groupes hydroxyle, un groupe alcoxyalkyle, un groupe alcoxycarbonylalkyle ou un groupe alcoxycarbonylalcényle contenant chacun jusqu'à 6 atomes de carbone dans les fractions alkyle ou alcényle individuelles, un groupe alkylaminoalkyle ou dialkylaminoalkyle contenant chacun de 1 à 6 atomes de carbone dans les fractions alkyle individuelles, chacun de ces groupes étant à chaîne droite ou ramifiée, un groupe cycloalkyle contenant 11 atomes de carbone; ou encore un groupe cycloalkyle, un groupe cycloalkylalkyle, un groupe cycloalcényle ou un groupe cycloalcénylalkyle contenant chacun de 3 à 8 atomes de carbone dans la fraction cycloalkyle ou cycloalcényle et éventuellement de 1 à 6 atomes de carbone dans la fraction alkyle à chaîne droite ou ramifiée, chacun de ces groupes étant éventuellement substitué une ou plusieurs fois de manière identique ou différente, dans lesquels, comme substituants, entrent chaque fois en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée; ou bien un groupe halogénalcényle à chaîne droite ou ramifiée contenant jusqu'à 4 atomes de carbone et de 1 à 5 atomes d'halogène identiques ou différents ou encore un groupe alcanediyle ou alcènediyle contenant chacun jusqu'à 4 atomes de carbone et étant chacun deux fois lié;

$R^2$ représente, en outre, un groupe hétérocyclylalkyle contenant de 1 à 6 atomes de carbone

dans la fraction alkyle à chaîne droite ou ramifiée et de 1 à 9 atomes de carbone, ainsi que de 1 à 3 hétéro-atomes - en particulier un atome d'azote, un atome d'oxygène et/ou un atome de soufre - dans la fraction hétérocyclyle, éventuellement substitué une ou plusieurs fois de manière identique ou différente dans la fraction hétérocyclyle, dans lequel, comme substituants, entrent en ligne de compte : un atome d'halogène, un groupe cyano, un groupe nitro; ainsi qu'un groupe alkyle, un groupe alcoxy, un groupe alkylthio, un groupe halogénalkyle, un groupe halogénalcoxy, un groupe halogénalkylthio ou un groupe alcoxycarbonyle contenant chacun de 1 à 5 atomes de carbone et éventuellement de 1 à 9 atomes d'halogène identiques ou différents, chacun de ces groupes étant à chaîne droite ou ramifiée; $R^2$ représente, en outre, un groupe alcoxy contenant de 1 à 8 atomes de carbone, un groupe alcényloxy contenant de 2 à 8 atomes de carbone ou un groupe alcynyloxy contenant de 2 à 8 atomes de carbone, chacun de ces groupes étant à chaîne droite ou ramifiée; et enfin un groupe aralkyle, un groupe aroyle, un groupe aryle, un groupe aralkyloxy ou un groupe aryloxy contenant chacun de 6 à 10 atomes de carbone dans la fraction aryle et éventuellement de 1 à 8 atomes de carbone dans la fraction alkyle, chacun de ces groupes étant éventuellement substitué une ou plusieurs fois de manière identique ou différente, dans lesquels, comme substituants du groupe alkyle, on envisage éventuellement un atome d'halogène et un groupe cyano, et dans lesquels, comme substituants du groupe aryle, entrent chaque fois en ligne de compte : un atome d'halogène, un groupe cyano, un groupe nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halogénalkylsulfonyle, un groupe alcanoyle ou un groupe alcoxycarbonyle contenant chacun de 1 à 6 atomes de carbone dans la fraction alkyle et éventuellement de 1 à 9 atomes d'halogène identiques ou différents, chacun de ces groupes étant à chaîne droite ou ramifiée; un groupe cycloalkyle contenant de 3 à 6 atomes de carbone et un groupe phénoxy;

X représente un atome d'oxygène ou un atome de soufre; et

Y représente un atome d'oxygène ou un atome de soufre,

caractérisé en ce qu'on fait réagir

a) des hydrazones de formule (II)

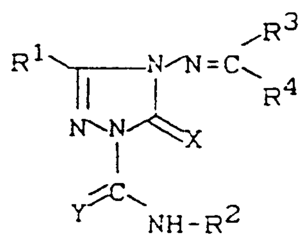

(II)

dans laquelle $R^1$, $R^2$, X et Y ont la signification indiquée ci-dessus, et

$R^3$ et $R^4$ représentent chacun, indépendamment l'un de l'autre, un atome d'hydrogène, un groupe alkyle, un groupe aralkyle ou un groupe aryle,

avec un acide, éventuellement en présence d'un diluant;

ou bien en ce qu'on fait réagir

b) des 1H-triazolinones de formule (III)

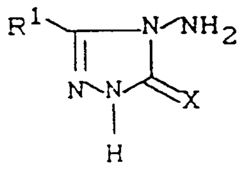

(III)

dans laquelle $R^1$ et X ont la signification indiquée ci-dessus,

avec des iso(thio)cyanates de formule (IV)

$R^2$-N = C = Y (IV)

dans laquelle $R^2$ et Y ont la signification indiquée ci-dessus,
éventuellement en présence d'un diluant et éventuellement en présence d'un adjuvant réactionnel;
ou bien en ce qu'on fait réagir
c) des triazolinones de formule (V)

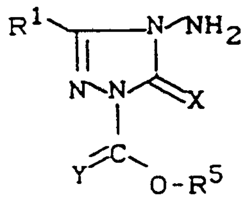

(V)

dans laquelle $R^1$, X et Y ont la signification indiquée ci-dessus, et $R^5$ représente un groupe alkyle, un groupe aryle ou un groupe arylalkyle,
avec des amines de formule (VI)

$R^2$-$NH_2$ (VI)

dans laquelle $R^2$ a la signification indiquée ci-dessus,
éventuellement en présence d'un diluant et éventuellement en présence d'un adjuvant réactionnel.

**2.** Procédé pour la préparation de triazolinones substituées de formule (I) selon la revendication 1, caractérisé en ce que

$R^1$ représente un atome d'hydrogène; un groupe méthyle, un groupe éthyle, un groupe n- ou i-propyle, un groupe n-, i-, s- ou t-butyle, un groupe n- ou i-pentyle, un groupe n- ou i-hexyle; un groupe allyle, un groupe propargyle; un groupe halogénalkyle à chaîne droite ou ramifiée contenant de 1 à 4 atomes de carbone et de 1 à 9 atomes d'halogène identiques ou différents, en particulier un atome de fluor, un atome de chlore ou un atome de brome; un groupe méthoxyméthyle, un groupe éthoxyméthyle, un groupe propoxyméthyle, un groupe cyclopentyle, un groupe cyclohexyle, un groupe cyclopropyle, un groupe cyclopropylméthyle, un groupe cyclohexylméthyle, un groupe cyclohexyléthyle; un groupe phényle éventuellement substitué de 1 à 3 fois de manière identique ou différente, dans lequel, comme substituants on envisage : un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy ou un groupe trifluorométhylthio,

$R^2$ représente un atome d'hydrogène; un groupe méthyle, un groupe éthyle, un groupe n- ou i-propyle, un groupe n-, i-, s- ou t-butyle; un groupe pentyle, un groupe hexyle, un groupe heptyle, un groupe octyle, un groupe nonyle, un groupe décyle, un groupe dodécyle, chacun étant à chaîne droite ou ramifiée; un groupe allyle; un groupe butényle, un groupe pentényle ou un groupe hexényle, chacun étant à chaîne droite ou ramifiée; un groupe propargyle; un groupe butynyle, un groupe pentynyle ou un groupe hexynyle, chacun étant à chaîne droite ou ramifiée; un groupe halogénalkyle à chaîne droite ou ramifiée contenant de 1 à 8 atomes de carbone et de 1 à 9 atomes d'halogène identiques ou différents, en particulier un atome de fluor, un atome de chlore ou un atome de brome; un groupe halogénalcényle ou un groupe halogénalcynyle contenant chacun de 3 à 8 atomes de carbone et de 1 à 3 atomes d'halogène, en particulier un atome de fluor ou un atome de chlore, chacun de ces groupes étant à chaîne droite ou ramifiée; un groupe cyanalkyle contenant de 1 à 6 atomes de carbone dans la fraction alkyle, un groupe hydroxyalkyle contenant de 1 à 6 atomes de carbone et de 1 à 3 groupes hydroxyle, un groupe alcoxyalkyle, un groupe alcoxycarbonylalkyle ou un groupe alcoxycarbonylalcényle, un groupe alkylaminoalkyle ou un groupe dialkylaminoalkyle contenant chacun jusqu'à 4 atomes de carbone dans les fractions alkyle ou alcényle individuelles, chacun de ces groupes étant à chaîne droite ou ramifiée; ou encore représente un groupe cyclopropyle, un groupe cyclopropylméthyle, un groupe cyclopropyléthyle, un groupe cyclopentyle, un groupe cyclohexyle, un groupe cycloheptyle, un groupe cyclooctyle, un groupe cyclohexylméthyle, un groupe cyclohexyléthyle, un groupe cyclohexényle ou un groupe cyclohexénylméthyle, chacun de ces groupes étant éventuellement substitué de 1 à 3 fois de manière identique ou différente, dans lesquels, comme substituants, entrent chaque fois en ligne de compte : un atome de fluor, un atome de chlore, un atome de brome, un groupe méthyle, un groupe éthyle, un groupe n- ou i-propyle, un groupe n-, i-, s- ou t-butyle, un groupe cyano, un groupe méthanediyle, un groupe éthanediyle, un groupe butanediyle ou un groupe butadiènediyle ou encore un groupe dichloroallyle;

$R^2$ représente, en outre, un groupe hétérocyclylméthyle, un groupe hétérocyclylpropyle ou un groupe hétérocyclyléthyle éventuellement substitués de 1 à 3 fois de manière identique ou différente dans la fraction hétérocyclyle, dans lesquels, comme composés hétérocycliques, entrent chaque fois en ligne de compte :

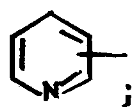 ; 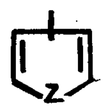 ; 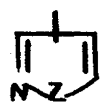 ; 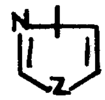 ;

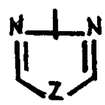 ; 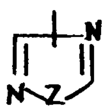 ; 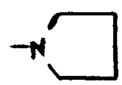 ; 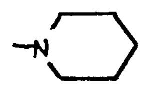 ;

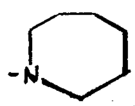 ; 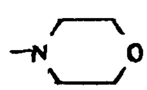 ou 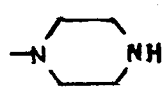

dans lesquels Z représente respectivement un atome d'oxygène ou un atome de soufre, et dans lesquels, comme substituants, entrent chaque fois en ligne de compte : un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy ou un groupe trifluorométhylthio;

$R^2$ représente, en outre, un groupe alcoxy contenant de 1 à 6 atomes de carbone, un groupe alcényloxy contenant de 3 à 6 atomes de carbone ou un groupe alcynyloxy contenant de 3 à 6 atomes de carbone, chacun de ces groupes étant à chaîne droite ou ramifiée; ou représente un groupe benzyle, un groupe phényléthyle, un groupe phénylpropyle, un groupe phénylbutyle, un groupe phénylpentyle, un groupe phénylhexyle, un groupe phénylheptyle, un groupe phénylcyanométhyle, un groupe phénylcyanoéthyle, un groupe phénylcyanopropyle, un groupe benzyloxy, un groupe phényléthyloxy, un groupe phénoxy, un groupe benzoyle, un groupe phényle ou un groupe naphtyle, chacun de ces groupes étant éventuellement substitué de 1 à 3 fois de manière identique ou différente, dans lesquels, comme substituants du groupe phényle, entrent chaque fois en ligne de compte : un atome de fluor, un atome de chlore, un atome de brome, un groupe hydroxyle, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy, un groupe trifluorométhylthio, un groupe trifluorométhylsulfinyle, un groupe trifluorométhylsulfonyle, un groupe méthylsulfinyle, un groupe méthylsulfonyle, un groupe acétyle, un groupe propionyle, un groupe méthoxycarbonyle, un groupe éthoxycarbonyle, un groupe cyclohexyle ou un groupe phénoxy,

X représente un atome d'oxygène ou un atome de soufre; et

Y représente un atome d'oxygène ou un atome de soufre.

**3.** Agents herbicides caractérisés par une teneur en au moins une triazolinone substituée de formule (I) selon les revendications 1 et 2.

4. Procédé pour lutter contre les mauvaises herbes, caractérisé en ce qu'on laisse agir des triazolinones substituées de formule (I) selon les revendications 1 et 2, sur les mauvaises herbes et/ou sur leur biotope.

5. Utilisation de triazolinones substituées de formule (I) selon les revendications 1 et 2, pour lutter contre les mauvaises herbes.

6. Procédé pour la préparation d'agents herbicides, caractérisé en ce qu'on mélange des triazolinones substituées de formule (I) selon les revendications 1 et 2, avec des diluants et/ou des substances tensioactives.

7. Procédé pour la préparation des hydrazones de formule (II)

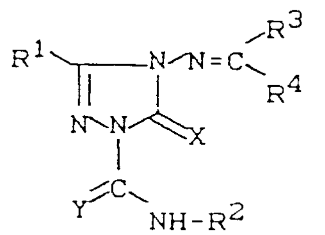

(II)

dans laquelle

$R^1$ représente un atome d'hydrogène; un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle contenant de 2 à 8 atomes de carbone et de 1 à 15 atomes d'halogène identiques ou différents, un groupe halogénalcynyle contenant de 2 à 8 atomes de carbone et de 1 à 13 atomes d'halogène identiques ou différents, un groupe alcoxyalkyle contenant de 1 à 6 atomes de carbone dans les fractions alkyle individuelles, chacun de ces groupes étant à chaîne droite ou ramifiée; un groupe cycloalkyle ou un groupe cycloalkylalkyle contenant chacun de 3 à 7 atomes de carbone dans la fraction cycloalkyle et éventuellement de 1 à 6 atomes de carbone dans la fraction alkyle à chaîne droite ou ramifiée; un groupe aryle contenant de 6 à 10 atomes de carbone dans la fraction aryle, substitué une ou plusieurs fois de manière identique ou différente, dans lequel, comme substituants du groupe aryle, entrent chaque fois en ligne de compte : un atome d'halogène, un groupe cyano, un groupe nitro; ainsi qu'un groupe alkyle, un groupe alcoxy, un groupe alkylthio, un groupe halogénalkyle, un groupe halogénalcoxy ou un groupe halogénalkylthio contenant chacun de 1 à 4 atomes de carbone et éventuellement de 1 à 9 atomes d'halogène identiques ou différents, chacun de ces groupes étant à chaîne droite ou ramifiée,

$R^2$ représente un atome d'hydrogène; un groupe alkyle contenant de 1 à 18 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou halogénalcynyle contenant chacun de 2 à 8 atomes de carbone et de 1 à 15 ou 13 atomes d'halogène identiques ou différents, un groupe cyanalkyle contenant de 1 à 8 atomes de carbone, un groupe hydroxyalkyle contenant de 1 à 8 atomes de carbone et de 1 à 6 groupes hydroxyle, un groupe alcoxyalkyle, un groupe alcoxycarbonylalkyle ou un groupe alcoxycarbonylalcényle contenant chacun jusqu'à 6 atomes de carbone dans les fractions alkyle ou alcényle individuelles, un groupe alkylaminoalkyle ou dialkylaminoalkyle contenant chacun de 1 à 6 atomes de carbone dans les fractions alkyle individuelles; un groupe cycloalkyle contenant 11 atomes de carbone; ou encore un groupe cycloalkyle, un groupe cycloalkylalkyle, un groupe cycloalcényle ou un groupe cycloalcénylalkyle contenant chacun de 3 à 8 atomes de carbone dans la fraction cycloalkyle ou cycloalcényle et éventuellement de 1 à 6 atomes de carbone dans la fraction alkyle à chaîne droite ou ramifiée, chacun de

ces groupes étant éventuellement substitué une ou plusieurs fois de manière identique ou différente, dans lesquels, comme substituants, entrent chaque fois en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée; et un groupe halogénalcényle à chaîne droite ou ramifiée contenant jusqu'à 4 atomes de carbone et de 1 à 5 atomes d'halogène identiques ou différents ou encore un groupe alcanediyle ou alcènediyle contenant chacun jusqu'à 4 atomes de carbone et étant chacun deux fois lié;

$R^2$ représente, en outre, un groupe hétérocyclylalkyle contenant de 1 à 6 atomes de carbone dans la fraction alkyle à chaîne droite ou ramifiée et de 1 à 9 atomes de carbone, ainsi que de 1 à 3 hétéro-atomes - en particulier un atome d'azote, un atome d'oxygène et/ou un atome de soufre - dans la fraction hétérocyclyle, éventuellement substitué une ou plusieurs fois de manière identique ou différente dans la fraction hétérocyclyle, dans lequel, comme substituants, entrent en ligne de compte : un atome d'halogène, un groupe cyano, un groupe nitro, ainsi qu'un groupe alkyle, un groupe alcoxy, un groupe alkylthio, un groupe halogénalkyle, un groupe halogénalcoxy, un groupe halogénalkylthio ou un groupe alcoxycarbonyle contenant chacun de 1 à 5 atomes de carbone et éventuellement de 1 à 9 atomes d'halogène identiques ou différents, chacun de ces groupes étant à chaîne droite ou ramifiée;

$R^2$ représente, en outre, un groupe alcoxy contenant de 1 à 8 atomes de carbone, un groupe alcényloxy contenant de 2 à 8 atomes de carbone ou un groupe alcynyloxy contenant de 2 à 8 atomes de carbone, chacun étant à chaîne droite ou ramifiée; et enfin un groupe aralkyle, un groupe aroyle, un groupe aryle, un groupe aralkyloxy ou un groupe aryloxy contenant chacun de 6 à 10 atomes de carbone dans la fraction aryle et éventuellement de 1 à 8 atomes de carbone dans la fraction alkyle, chacun de ces groupes étant éventuellement substitué une ou plusieurs fois de manière identique ou différente, dans lesquels, comme substituants du groupe alkyle, on envisage éventuellement un atome d'halogène et un groupe cyano, et dans lesquels, comme substituants du groupe aryle, entrent chaque fois en ligne de compte : un atome d'halogène, un groupe cyano, un groupe nitro; un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halogénalkylsulfonyle, un groupe alcanoyle ou un groupe alcoxycarbonyle contenant chacun de 1 à 6 atomes de carbone dans la fraction alkyle et éventuellement de 1 à 9 atomes d'halogène identiques ou différents, chacun de ces groupes étant à chaîne droite ou ramifiée, un groupe cycloalkyle contenant de 3 à 6 atomes de carbone et un groupe phénoxy;

X représente un atome d'oxygène ou un atome de soufre,

Y représente un atome d'oxygène ou un atome de soufre,

$R^3$ et $R^4$ représentent chacun, indépendamment l'un de l'autre, un atome d'hydrogène, un groupe alkyle à chaîne droite ou ramifiée contenant de 1 à 4 atomes de carbone, ou encore un groupe phényle ou un groupe benzyle,

à l'exception du compose

1-(N-4-bromophénylthiocarbamoyl)-4-phénylidènamino-1,2,4-triazolin-5-thione,

caractérisé en ce qu'on fait réagir des 4-aminotriazolinones 1-non substituées de formule (III)

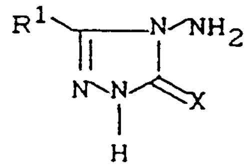

(III)

dans laquelle

$R^1$ et X ont la signification indiquée ci-dessus,

avec des aldéhydes ou des cétones de formule (VII),

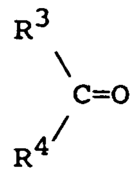

dans laquelle
$R^3$ et $R^4$ ont la signification indiquée ci-dessus,
éventuellement en présence d'un diluant tel que par exemple le dichlorométhane ou le toluène, et éventuellement dans une troisième étape ultérieure, avec des amines de formule (VI)

$R^2-NH_2$ (VI)

dans laquelle
$R^2$ a la signification indiquée ci-dessus,
éventuellement en présence d'un diluant tel que par exemple le tétrahydrofuranne, ainsi qu'éventuellement en présence d'une base telle que par exemple l'hydroxyde de sodium ou l'hydroxyde de potassium à des températures entre 20 °C et 50 °C.

**8.** Procédé pour la préparation de triazolinones de formule (Va)

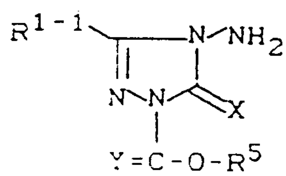

(Va)

dans laquelle

$R^{1-1}$ représente un groupe alkyle à chaîne droite ou ramifiée contenant de 1 à 4 atomes de carbone,

$R^5$ représente un groupe alkyle à chaîne droite ou ramifiée contenant de 1 à 4 atomes de carbone, ou encore un groupe phényle ou un groupe benzyle éventuellement substitués de 1 à 3 fois de manière identique ou différente, dans lesquels, comme substituants, entrent chaque fois en ligne de compte : un atome d'halogène, un groupe cyano, un groupe nitro; un groupe alkyle, un groupe alcoxy ou un groupe alkylthio contenant chacun de 1 à 4 atomes de carbone, chacun de ces groupes étant à chaîne droite ou ramifiée; ou encore un groupe halogénalkyle, un groupe halogénalcoxy ou un groupe halogénalkylthio contenant chacun de 1 à 4 atomes de carbone et de 1 à 9 atomes d'halogène identiques ou différents, chacun de ces groupes étant à chaîne droite ou ramifiée.

X et Y représentent, indépendamment l'un de l'autre, un atome d'oxygène ou un atome de soufre,

caractérisé en ce qu'on fait réagir des 1H-triazolinones de formule (IIIa)

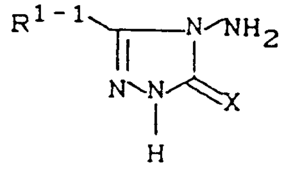

(IIIa)

dans laquelle
$R^{1-1}$ et X ont la signification indiquée ci-dessus,

avec des esters (thio)chloroformiques de formule (IX),

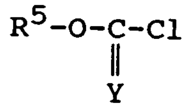

(IX)

dans laquelle

$R^5$ et Y ont la signification indiquée ci-dessus,

éventuellement en présence d'un diluant tel que par exemple le tétrahydrofuranne et éventuellement en présence d'un adjuvant réactionnel tel que par exemple le t-butylate de potassium ou l'hydrure de sodium à des températures entre -20 °C et +40 °C.

**9.** Procédé pour la préparation de triazolinones de formule (Xa)

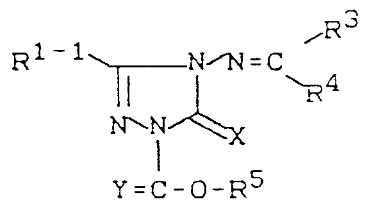

(Xa)

dans laquelle

- $R^{1-1}$ représente un groupe alkyle à chaîne droite ou ramifiée contenant de 1 à 4 atomes de carbone,
- $R^3$ et $R^4$ représentent chacun, indépendamment l'un de l'autre, un atome d'hydrogène, un groupe alkyle à chaîne droite ou ramifiée contenant de 1 à 4 atomes de carbone, ou encore un groupe phényle ou un groupe benzyle
- $R^5$ représente un groupe alkyle à chaîne droite ou ramifiée contenant de 1 à 4 atomes de carbone, ou encore un groupe phényle ou un groupe benzyle éventuellement substitués de 1 à 3 fois de manière identique ou différente, dans lesquels, comme substituants, entrent chaque fois en ligne de compte : un atome d'halogène, un groupe cyano, un groupe nitro; un groupe alkyle, un groupe alcoxy ou un groupe alkylthio contenant chacun de 1 à 4 atomes de carbone, chacun de ces groupes étant à chaîne droite ou ramifiée; ou encore un groupe halogénalkyle, un groupe halogénalcoxy ou un groupe halogénalkylthio contenant chacun de 1 à 4 atomes de carbone et de 1 à 9 atomes d'halogène identiques ou différents, chacun de ces groupes étant à chaîne droite ou ramifiée,
- X et Y représentent, indépendamment l'un de l'autre, un atome d'oxygène ou un atome de soufre.

caractérisé en ce qu'on fait réagir des 4-aminotriazolinones 1-non substituées de formule (III)

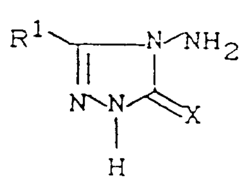

(III)

dans laquelle

- $R^1$ et X ont les significations mentionnées dans la description, avec des aldéhydes ou des cétones de formule (VII),

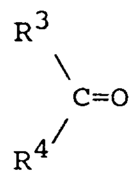

dans laquelle
$R^3$ et $R^4$ ont les significations mentionnées dans la description,
éventuellement en présence d'un diluant tel que par exemple le dichlorométhane ou le toluène, et éventuellement en présence d'un catalyseur tel que par exemple l'acide p-toluènesulfonique à des températures entre 40 °C et 120 °C, et on fait réagir les triazolinone-hydrazones 1-non substituées de formule (VIII) que l'on obtient ainsi

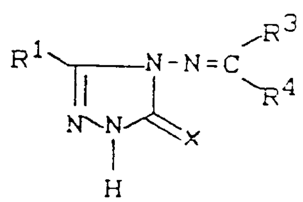

(VIII)

dans laquelle
$R^1$, $R^3$, $R^4$ et X ont les significations mentionnées dans la description,
dans une deuxième étape ultérieure avec des esters (thio)chloroformiques de formule (IX)

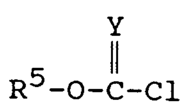

(IX)

dans laquelle
$R^5$ et Y ont les significations mentionnées dans la description,
éventuellement en présence d'un diluant tel que par exemple le tétrahydrofuranne et éventuellement en présence d'un adjuvant réactionnel tel que par exemple l'hydrure de sodium ou le t-butylate de potassium à des températures entre -20 °C et +40 °C.